# EUROPÄISCHE PATENTANMELDUNG

(11) **EP 4 559 999 A1**
(43) Veröffentlichungstag der Anmeldung: **28.05.2025**
(21) Anmeldenummer: 24209145.2
(22) Anmeldetag: 28.10.2024
(51) Int. Cl.: C11D 3/48, C11D 1/83, C11D 3/20, C11D 3/22, C11D 3/33, C11D 3/36

(54) **WASCH- UND REINIGUNGSMITTEL ENTHALTEND ANTIMIKROBIELLES PEPTID**

(30) Priorität: 24.11.2023 DE 102023211746
(71) Anmelder: Henkel AG & Co. KGaA, 40589 Düsseldorf (DE)
(72) Erfinder: Evers, Stefan, 42781 Haan (DE); Islam, Shohana, 64665 Alsbach-Hähnlein (DE); Schages, Jan, 40476 Düsseldorf (DE); Weide, Mirko, 40223 Düsseldorf (DE); Degering, Christian, 40699 Erkrath (DE); Wieland, Susanne, 41541 Zons/Dormagen (DE); Anic, Iva, 40225 Düsseldorf (DE); Schmidt, Irmgard, 42699 Solingen (DE); Boes, Alexander, 50769 Köln (DE); Jakob, Felix, 41812 Erkelenz (DE); Schwaneberg, Ulrich, 4728 Kelmis-Hergenrath (BE); Heine, Elisabeth, 52385 Nideggen (DE)

(57) **Zusammenfassung**

Die Erfindung liegt auf dem Gebiet der Wasch- und Reinigungsmittel, insbesondere peptidhaltige Wasch- und Reinigungsmittel, und betrifft die Verwendung eines Peptids, insbesondere eines antimikrobiell wirksamen Peptids, in einem Wasch- und Reinigungsmittels zur Reinigung von Textilien und/oder harten Oberflächen, insbesondere Geschirr, und/oder Haushaltsoberflächen. Das erfindungsgemäße Wasch- und Reinigungsmittel umfasst vorzugsweise ein antimikrobiell wirksames Peptid, mindestens ein Tensid, ein Buildersystem, und optional mindestens ein Enzym. Des Weiteren betrifft die Erfindung entsprechende Mittel und Verfahren.

## Beschreibung

Die Erfindung liegt auf dem Gebiet der Wasch- und Reinigungsmittel, insbesondere peptidhaltige Wasch- und Reinigungsmittel, und betrifft die Verwendung eines Peptids, insbesondere eines antimikrobiell wirksamen Peptids, in einem Wasch- und Reinigungsmittels zur Reinigung von Textilien und/oder harten Oberflächen, insbesondere Geschirr, und/oder Haushaltsoberflächen. Des Weiteren betrifft die Erfindung entsprechende Mittel und Verfahren.

Das wichtigste Kriterium beim Reinigen von Textilien und/oder harten Oberflächen ist die Reinigungsleistung an verschiedensten Anschmutzungen. Auch wenn die Reinigungsleistung der heutzutage eingesetzten Wasch- und Reinigungsmittel generell hoch ist, stellt sich, auch aufgrund des generellen Trends vermehrt Niedrigtemperatur-Programme zu verwenden sowie durch den Trend zur Kompaktierung der Rezepturen (insbesondere bei vorportionierten Verwendungsformen), allerdings das Problem, dass viele der üblichen Wasch- und Reinigungsmittelrezepturen eine unzureichende Reinigungsleistung, insbesondere an hartnäckigen Anschmutzungen, aufweisen. Eine solche unzureichende Reinigungsleistung führt beim Verbraucher zu Unzufriedenheit und dazu, dass solche Anschmutzungen vom Verbraucher vorbehandelt werden, was wiederum den Wasser- und Energieverbrauch erhöht.

Durch den Trend zu Niedrigtemperatur-Programmen werden Mikroorganismen, wie z.B. Bakterien, Pilze und Viren, die auf Textilien und/oder harten Oberflächen, insbesondere Geschirr, und/oder Haushaltsoberflächen, bevorzugt in Wasch- und Geschirrspülmaschinen, vorhanden sind, nicht in ausreichendem Maße abgetötet und/oder entfernt, so dass dies zu einer Verbreitung von Keimen von einer Oberfläche auf eine andere Oberfläche, z.B. von einem Textil zu einem anderen Textil während des Wasch- oder Reinigungsvorganges, kommen kann. Dies kann unter Umständen ein hygienisches Problem darstellen. Dieser Umstand wird noch verstärkt, wenn bleichefreie Waschmittel, z.B. Color-Waschmittel oder flüssige Waschmittel oder Feinwaschmittel, verwendet werden. Dies betrifft auch flüssige Maschinen- und Handgeschirrspülmittel, die ebenfalls in der Regel frei von Bleiche sind und bei eher niedrigen Temperaturen Anwendung finden. Das Wachstum von Mikroorganismen bedingt nicht nur ein hygienisches Problem, sondern kann auch zu Schlechtgeruch und/oder mikrobiell bedingten Ablagerungen auf Oberfläche, wie Textilien, Geschirr und anderen Haushaltsoberflächen, führen.

Der Begriff "antimikrobiell wirksam" oder antimikrobielle Wirksamkeit", wie hierin verwendet, bedeutet, dass lebende Mikroorganismen, wie z.B. Bakterien, Pilze oder Viren, in einen Zustand versetzt werden, indem sie in ihrem Wachstum gehemmt sind bzw. abgetötet werden, oder zumindest nicht mehr infektiös oder virulent sind. "Im Wachstum gehemmt" bedeutet, dass trotz optimaler Wachstumsbedingungen in einem Medium, die Anzahl an vermehrungsfähigen und/oder lebensfähigen individuellen Mikroorganismen im Wesentlichen konstant bleibt oder sinkt. "Abgetötet" bedeutet, dass die Anzahl an vermehrungsfähigen und/oder lebensfähigen individuellen Mikroorganismen in einem Medium im Vergleich zu einem Referenzwert reduziert ist. Vorzugsweise wird die antimikrobielle Wirksamkeit, d.h. die Reduzierung der lebensfähigen und/oder vermehrungsfähigen Zellen, wie in Beispiel 1 hierin beschrieben bestimmt. Erfindungsgemäß können antimikrobiell wirksame Substanzen sowohl antibakterielle als auch antifungale oder antivirale Substanzen umfassen, sowie auch bakterizide, bakteriostatische, viruzide, virustatische, fungizide oder fungistatische Substanzen.

Mit dem Begriff "Schlechtgeruch", wie hierin verwendet, ist ein Geruch gemeint, der auf Textilien und/oder harten Oberflächen, insbesondere Geschirr, und/oder Haushaltsoberflächen, nicht erwünscht ist. Verschiedenste Ursachen können auf Oberflächen, insbesondere Haushaltsoberflächen, wie z.B. Oberflächen in Wasch- oder Geschirrspülmaschinen, Pumpensümpfen, Vorratsbehältern, Wasserreservoir, Waschtrommel, Spülkammern, Wasch- oder Spülbecken, Toilettenbecken, Spülkasten, WC-Rand, Siphons, Wasserrohren und/oder -leitungen, Wasserzu- und/oder -abläufen, sowie auf gewaschenen Textilien und/oder gereinigten harten Oberflächen, insbesondere Geschirr, einen unangenehmen Geruch, sogenannten Schlechtgeruch, verursachen. Dieses Problem ist insbesondere bei Textilien und Waschmaschinen bekannt. Aber auch in der Geschirrspülmaschine findet sich Schlechtgeruch. Betroffen sind alle Bauteile, die mit verschmutzten Gegenständen und/oder Wasser, insbesondere gebrauchtem Wasser, und/oder Wasch-/Reinigungslauge in Berührung kommen, wie z.B. Oberflächen in Wasch- oder Geschirrspülmaschinen, Pumpensümpfen, Vorratsbehältern, Wasserreservoir, Waschtrommel, Spülkammern, Wasch- oder Spülbecken, Toilettenbecken, Spülkasten, WC-Rand, Siphons, Wasserrohren und/oder -leitungen, Wasserzu- und/oder -abläufen. Eine, wenn nicht die häufigste, Ursache hierfür sind organischer Natur, wie z.B. Sebum, Körperschmutz (body soils; z.B. Körperöle, abgestorbene Hautschuppen und Schweiß), Zellrückstände, Nahrungsmittelreste, Körperausscheidungen, Biofilme und extrazelluläre polymere Substanzen (EPS; z.B. extrazelluläre polymere Saccharide). Hierbei hervorzuheben sind insbesondere auch die mikrobiell bedingten Biofilme und Biobeläge, d.h. mikrobiell bedingter Schlechtgeruch in Folge von mikrobiell bedingten Biofilmen und Biobelägen. Ein weiteres Beispiel für unangenehme Gerüche ist Schweiß oder Körpergeruch, der an einem Gegenstand anhaftet, der mit Menschen und/oder Tieren in Berührung gekommen ist. Schlechtgeruch findet sich nicht nur in der Waschmaschine, sondern auch auf der frisch gewaschenen, noch feuchten Wäsche, sowie auch auf der trockenen Wäsche, sowie auf zahlreichen weiteren Oberflächen, insbesondere Haushaltsoberflächen, die mit verschmutzten Gegenständen und/oder Wasser, insbesondere gebrauchtem Wasser, und/oder Wasch-/Reinigungslauge in Berührung kommen, wie z.B. Oberflächen in Wasch- oder Geschirrspülmaschinen, Pumpensümpfen, Vorratsbehältern, Wasserreservoir, Waschtrommel, Spülkammern, Wasch- oder Spülbecken, Toilettenbecken, Spülkasten, WC-Rand, Siphons, Wasserrohren und/oder -leitungen, Wasserzu- und/oder -abläufen. Biofilme, Ablagerungen, Biobeläge, Body soils und dergleichen führen jedoch nicht nur zu Schlechtgeruch, sondern können auch als Anschmutzungen auf Textilien und/oder Oberflächen, insbesondere Haushaltsoberflächen, wie z.B. Oberflächen in Wasch- oder Geschirrspülmaschinen, Pumpensümpfen, Vorratsbehältern, Wasserreservoir, Waschtrommel, Spülkammern, Wasch- oder Spülbecken, Toilettenbecken, Spülkasten, WC-Rand, Siphons, Wasserrohren und/oder -leitungen, Wasserzu- und/oder -abläufen, sichtbar werden, z.B. in Form von Belägen, Schmier, Ankrustungen, Vergilbungen, Achselflecken, Vergrauungen oder dergleichen. Biofilme, Biobeläge und dergleichen ermöglichen durch ihre typischerweise adhäsive Textur auch das Anhaften weiterer Schlechtgeruchs- und/oder Schmutzpartikel.

Der Begriff "Biofilm" oder "Biobelag", wie hierin verwendet, ist gleichbedeutend mit dem Begriff "mikrobiell bedingte Ablagerung" und meint jegliche mikrobiell bedingten Ablagerungen, Biofilme, Biobeläge oder dergleichen, die von jeglichen Mikroorganismen gebildet werden können, deren Zellen aneinander oder an einer Oberfläche anhaften, z.B. an Textilien, Geschirr oder einer harten Oberfläche oder einer anderen Art von Oberfläche. Diese anhaftenden Zellen sind häufig in eine selbstproduzierte Matrix aus extrazellulärer polymerer Substanz (EPS) eingebettet. Biofilm-EPS ist ein polymeres Konglomerat, das im Allgemeinen aus extrazellulärer DNA, Proteinen und Polysacchariden besteht. Biofilme können sich auf lebenden oder nicht lebenden Oberflächen bilden. Die mikrobiellen Zellen, die in einem Biofilm wachsen, unterscheiden sich physiologisch von den planktonischen Zellen desselben Organismus, bei denen es sich im Gegensatz dazu um Einzelzellen handelt, die in einem flüssigen Medium schwimmen oder schwimmen können. Bakterien, die in einem Biofilm leben, weisen in der Regel deutlich andere Eigenschaften als planktonische Bakterien derselben Art auf, da die dichte und geschützte Umgebung des Films es ihnen ermöglicht, auf verschiedene Weise zu kooperieren und zu interagieren. Ein Vorteil dieses Milieus für die Mikroorganismen ist die erhöhte Resistenz gegenüber Wasch- und Reinigungsmitteln und Antibiotika, da die dichte extrazelluläre Matrix und die äußere Zellschicht das Innere der Gemeinschaft schützen. Zu den Biofilm produzierenden Bakterien auf Textilien gehören z.B. die folgenden Arten: *Acinetobacter* sp., *Aeromicrobium* sp., *Brevundimonas* sp., *Microbacterium* sp., *Micrococcus luteus, Pseudomonas* sp., *Staphylococcus epidermidis* und *Stenotrophomonas* sp. Auf harten Oberflächen finden sich biofilmbildende Bakterien z.B. unter den folgenden Arten: *Acinetobacter* sp., *Aeromicrobium* sp., *Brevundimonas* sp., *Microbacterium* sp., *Micrococcus luteus, Pseudomonas* sp, *Staphylococcus epidermidis, Staphylococcus aureus* und *Stenotrophomonas* sp.

Angesammelte organische Verschmutzungen sind oft schwer zu entfernen, und handelsübliche Wasch- und Reinigungsmittel reduzieren den Schlechtgeruch nicht ausreichend. Stattdessen wird der Schlechtgeruch durch die Verwendung verschiedener Duftstoffe überdeckt, was von den Verbrauchern oft als Reinigungseffekt empfunden wird, obwohl die Ursachen der Schlechtgerüche nicht wirklich beseitigt werden. Dies hat zur Folge, dass sich im Laufe der Zeit immer mehr organisches Material und damit immer mehr Schlechtgeruch ansammelt und immer mehr Duftstoffe zugesetzt werden müssen, damit die Textilien nicht immer schlechter riechen. Viele Duftstoffe haben jedoch Nebenwirkungen wie z.B. allergene Eigenschaften, die sich in Form von Hautausschlägen äußern können. Außerdem sind viele dieser Stoffe als Naturchemikalien umweltschädlich, wenn sie ins Abwasser gelangen. In den letzten Jahren werden Duftstoffe zunehmend in Form von Duftstoffmikrokapseln verwendet, obwohl bekannt ist, dass Mikrokapseln als Mikroplastik gelten und daher potenziell umweltschädlich sind. Außerdem sind Duftstoffe teuer und tragen zu den Gesamtkosten der Wasch- und Reinigungsmittel bei.

Es besteht daher Bedarf, die Reinigungsleistung von Wasch- und Reinigungsmitteln, insbesondere im Hinblick auf Beseitigung des Schlechtgeruchs in Wasch- und Geschirrspülmaschinen und/oder auf den gewaschenen Textilien bzw. gereinigten Oberflächen, insbesondere Geschirr, sowie Oberflächen, insbesondere Haushaltsoberflächen, wie z.B. Oberflächen in Wasch- oder Geschirrspülmaschinen, Pumpensümpfen, Vorratsbehältern, Wasserreservoir, Waschtrommel, Spülkammern, Wasch- oder Spülbecken, Toilettenbecken, Spülkasten, WC-Rand, Siphons, Wasserrohren und/oder -leitungen, Wasserzu- und/oder -abläufen, zu verbessern. Weiter besteht auch Bedarf, die Hygiene in Wasch- und Geschirrspülmaschinen zu verbessern, sowie mikrobiell bedingte Biofilme/-beläge zuverlässig von Oberflächen, insbesondere Haushaltsoberflächen, wie z.B. Oberflächen in Wasch- oder Geschirrspülmaschinen, Pumpensümpfen, Vorratsbehältern, Wasserreservoir, Waschtrommel, Spülkammern, Wasch- oder Spülbecken, Toilettenbecken, Spülkasten, WC-Rand, Siphons, Wasserrohren und/oder -leitungen, Wasserzu- und/oder -abläufen, zu entfernen. Mit umfasst sind dabei auch (halb-)automatisierte Wasch- oder Reinigungssystem wie z.B. Wischrobotor oder Nassstaubsauger.

Überraschenderweise wurde nun festgestellt, dass erfindungsgemäße Peptide antimikrobiell wirksam sind und das Wachstum von Mikroorganismen verhindern und/oder reduzieren. Somit können sie nicht nur dazu beitragen, die Wasch- und Reinigungsleistung hygienisch zu verbessern, sondern auch dazu, mikrobiell bedingten Schlechtgeruch sowie mikrobiell bedingte Ablagerungen (Biofilme, Biobeläge und dergleichen) zu verhindern.

Erfindungsgemäße Peptide können zudem in Wasch- und Pflegeprodukten zur Haarpflege sowie in industriellen und institutionellen Reinigern wie Desinfektionsprodukten, insbesondere Desinfektionsmitteln, antimikrobiell wirksam sein.

Gegenstand der Erfindung ist daher in einem ersten Aspekt ein Mittel, vorzugsweise Wasch- und Reinigungsmittel, bevorzugt flüssiges Wasch- und Reinigungsmittel, besonders bevorzugt flüssiges Textilwaschmittel, umfassend
(A) mindestens ein Peptid, wobei das Peptid
   eine Länge von 10 bis 50, vorzugsweise 10 bis 30, bevorzugt 12 bis 25, Aminosäuren aufweist, die aus der aus Alanin, Arginin, Asparagin, Asparaginsäure, Cystein, Glutamin, Glutaminsäure, Glycin, Histidin, Isoleucin, Leucin, Lysin, Methionin, Phenylalanin, Prolin, Serin, Threonin, Tryptophan, Tyrosin und Valin bestehenden Gruppe ausgewählt sind, vorzugsweise ausgewählt aus Alanin, Arginin, Glutaminsäure, Glycin, Isoleucin, Leucin, Lysin, Phenylalanin, Prolin, Serin, Threonin, Tryptophan, Tyrosin und Valin,
   eine Nettoladung von +2 bis +8, vorzugsweise +3 bis +7, bevorzugt +4 bis +6, aufweist,
   antimikrobiell wirksam ist, wobei die antimikrobielle Wirksamkeit wie in Beispiel 1 beschrieben bestimmt wird,
(B) mindestens ein Tensid, wobei das Tensid aus der aus anionischen Tensiden, nicht-ionischen Tensiden, kationischen Tensiden, zwitterionischen Tensiden, amphoteren Tensiden und Mischungen davon bestehenden Gruppe ausgewählt ist;
(C) ein Buildersystem, umfassend mindestens einen Builder, wobei der Builder aus der aus Polycarbonsäuren wie Zitronensäure, Adipinsäure, Bernsteinsäure, Glutarsäure, Äpfelsäure, Weinsäure, Maleinsäure Fumarsäure, Zuckersäuren und Carboxymethylinuline oder deren Salzen, monomeren und polymeren Aminopolycarbonsäuren wie Glycindiessigsäure, Methylglycindiessigsäure (MGDA), Glutaminsäurediessigsäure (GLDA), Nitriltriessigsäure, Iminodisuccinat wie Ethylendiamin-N,N'-Disuccinsäure und Hydroxyiminodisuccinate, Ethylendiamintetraessigsäure und Polyasparaginsäure oder deren Salze, Polyphosphonsäuren wie Aminotris(methylenphosphonsäure), Ethylendiamintetrakis(methylenphosphonsäure), Lysintetra(methylenphosphonsäure), Diethylentriaminpenta(methylenphosphonsäure) (DTPMP) und 1-Hydroxyethan-1,1-diphosphonsäure (HEDP) oder deren Salze, polymeren Hydroxyverbindungen wie Dextrin, und Mischungen davon bestehenden Gruppe ausgewählt ist; und
(D) optional mindestens ein Enzym, wobei das Enzym aus der aus Proteasen, Amylasen, Lipasen, Mannanasen, Cellulasen oder Mischungen davon bestehenden Gruppe ausgewählt ist.

In einem weiteren Aspekt betrifft die Erfindung ein Mittel, vorzugsweise Wasch- und Reinigungsmittel, wobei das Mittel umfasst
(A) mindestens ein Peptid, wobei das Peptid
   eine Länge von 10 bis 50, vorzugsweise 10 bis 30, bevorzugt 12 bis 25, Aminosäuren aufweist, die aus der aus Alanin, Arginin, Asparagin, Asparaginsäure, Cystein, Glutamin, Glutaminsäure, Glycin, Histidin, Isoleucin, Leucin, Lysin, Methionin, Phenylalanin, Prolin, Serin, Threonin, Tryptophan, Tyrosin und Valin bestehenden Gruppe ausgewählt sind, vorzugsweise ausgewählt aus Alanin, Arginin, Glutaminsäure, Glycin, Isoleucin, Leucin, Lysin, Phenylalanin, Prolin, Serin, Threonin, Tryptophan, Tyrosin und Valin,
   eine Nettoladung von +2 bis +8, vorzugsweise +3 bis +7, bevorzugt +4 bis +6, aufweist, antimikrobiell wirksam ist, wobei die antimikrobielle Wirksamkeit wie in Beispiel 1 beschrieben bestimmt wird,
   in einer Menge von aktivem Peptid von 1 × 10⁻⁸ bis 5 Gew.-%, vorzugsweise 0,0001 bis 1 Gew.-%, bevorzugt 0,0005 bis 0,5 Gew.-%, besonders bevorzugt 0,001 bis 0,1 Gew.-%, bezogen auf das Gesamtgewicht des Mittels;
(B) 2 bis 20 Gew.-%, vorzugsweise 3 bis 17 Gew.-% anionische Tenside,
(C) 1 bis 10 Gew.-%, vorzugsweise 3 bis 8 Gew.-%, nichtionische Tenside,
(D) 0 bis 1 Gew.-%, vorzugsweise 0 bis 0,5 Gew.-%, Seife, und
(E) 0 bis 5 Gew.-%, vorzugsweise 0 bis 3 Gew.-% Fettsäuren,
   jeweils bezogen auf das Gesamtgewicht des Mittels.

In noch einem weiteren Aspekt betrifft die Erfindung ein Mittel, vorzugsweise Wasch- und Reinigungsmittel, wobei das Mittel umfasst
(A) mindestens ein Peptid, wobei das Peptid
   eine Länge von 10 bis 50, vorzugsweise 10 bis 30, bevorzugt 12 bis 25, Aminosäuren aufweist, die aus der aus Alanin, Arginin, Asparagin, Asparaginsäure, Cystein, Glutamin, Glutaminsäure, Glycin, Histidin, Isoleucin, Leucin, Lysin, Methionin, Phenylalanin, Prolin, Serin, Threonin, Tryptophan, Tyrosin und Valin bestehenden Gruppe ausgewählt sind, vorzugsweise ausgewählt aus Alanin, Arginin, Glutaminsäure, Glycin, Isoleucin, Leucin, Lysin, Phenylalanin, Prolin, Serin, Threonin, Tryptophan, Tyrosin und Valin,
   eine Nettoladung von +2 bis +8, vorzugsweise +3 bis +7, bevorzugt +4 bis +6, aufweist, antimikrobiell wirksam ist, wobei die antimikrobielle Wirksamkeit wie in Beispiel 1 beschrieben bestimmt wird,
   in einer Menge von aktivem Peptid von 1 × 10⁻⁸ bis 5 Gew.-%, vorzugsweise 0,0001 bis 1 Gew.-%, bevorzugt 0,0005 bis 0,5 Gew.-%, besonders bevorzugt 0,001 bis 0,1 Gew.-%, bezogen auf das Gesamtgewicht des Mittels;
(B) 0 bis 10 Gew.-%, vorzugsweise 1 bis 4 Gew.-% Zitronensäure und/oder Citrat, vorzugsweise Alkalicitrat,
(C) 0 bis 40 Gew.-%, vorzugsweise 0 bis 15 Gew.-%, stärker bevorzugt 1 bis 3 Gew.-%, Alkalicarbonat, vorzugsweise Natriumcarbonat,
(D) 0 bis 20 Gew.-%, vorzugsweise 3 bis 10 Gew.-% Alkalisilikat,
(E) 0 bis 10 Gew.-%, vorzugsweise 0,5 bis 2 Gew.-%, Phosphonsäure und/oder Alkaliphosphonat, besonders bevorzugt HEDP und/oder DTPMP, und/oder
(F) 0 bis 10 Gew.-%, vorzugsweise 0,5 bis 3 Gew.-%, Aminopolycarbonsäuren, vorzugsweise MGDA und/oder GLDA,
   jeweils bezogen auf das Gesamtgewicht des Mittels.

In bevorzugten Ausführungsformen umfasst das Peptid mindestens 1 bis 5 Tryptophan-Reste und/oder 1 bis 5 Arginin-Reste und/oder 1 bis 5 Lysin-Reste, vorzugsweise mindestens 1 bis 4 Tryptophan-Reste und/oder 1 bis 3 Arginin-Reste und/oder 1 bis 5 Lysin-Reste.

In weiter bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% oder 100% identisch ist.

In bevorzugten Ausführungsformen ist das erfindungsgemäße Peptid in einem Mittel, vorzugsweise Wasch- und Reinigungsmittel, enthalten, wobei
das Mittel im Wesentlichen frei von borhaltigen Verbindungen ist, vorzugsweise frei von borhaltigen Verbindungen ist; und/oder
das Mittel im Wesentlichen frei von Phosphonaten ist, vorzugsweise frei von Phosphonaten, insbesondere frei von HEDP und/oder DTPMP, ist; und/oder
das Mittel in 1 Gew.-%-iger Lösung in entionisiertem Wasser bei 20°C einen pH-Wert in einem Bereich von etwa 6 bis etwa 11, insbesondere von etwa 6,5 bis etwa 10,5, weiter bevorzugt von etwa 7 bis etwa 10, besonders bevorzugt von etwa 8 bis etwa 9 aufweist; und/oder
das Mittel, bezogen auf das Gesamtgewicht des Mittels, weniger als 10 Gew.-% LAS, vorzugsweise weniger als 6 Gew.-%, noch bevorzugter weniger als 3 Gew.-% enthält, noch bevorzugter im Wesentlichen frei von LAS ist, besonders bevorzugt frei von LAS ist; und/oder
das Mittel im Wesentlichen frei von Bleiche ist, vorzugsweise frei von Bleiche ist.

Weitere Gegenstände der Erfindung betreffen
- Verfahren zur Reinigung von Textilien und/oder harten Oberflächen, insbesondere Geschirr, dadurch gekennzeichnet, dass in mindestens einem Verfahrensschritt ein hierin beschriebenes Wasch- und Reinigungsmittel angewendet wird, wobei das Verfahren vorzugsweise in einem Temperaturbereich von etwa 20°C bis etwa 60°C, bevorzugt etwa 40°C, besonders bevorzugt etwa 30°C, durchgeführt wird;
- Verfahren zur Reinigung von Oberflächen, insbesondere Haushaltsoberflächen, bevorzugt in Wasch- oder Geschirrspülmaschinen, Pumpensümpfen, Vorratsbehältern, Wasserreservoir, Waschtrommel, Spülkammern, Wasch- oder Spülbecken, Toilettenbecken, Spülkasten, WC-Rand, Siphons, Wasserrohren und/oder -leitungen, Wasserzu- und/oder -abläufen, und/oder Textilien, insbesondere zur Verhinderung und/oder Reduzierung von Schlechtgeruch und/oder zur Verbesserung der Hygiene, dadurch gekennzeichnet, dass in mindestens einem Verfahrensschritt ein hierin beschriebenes Wasch- und Reinigungsmittel angewendet wird, wobei das Verfahren vorzugsweise in einem Temperaturbereich von etwa 20°C bis etwa 60°C, bevorzugt etwa 40°C, besonders bevorzugt etwa 30°C, durchgeführt wird;
- Verwendung eines Peptids in einem Wasch- und Reinigungsmittels zur Reinigung von Textilien und/oder harten Oberflächen, insbesondere Geschirr, und/oder Haushaltsoberflächen, bevorzugt in Wasch- oder Geschirrspülmaschinen, Pumpensümpfen, Vorratsbehältern, Wasserreservoir, Waschtrommel, Spülkammern, Wasch- oder Spülbecken, Toilettenbecken, Spülkasten, WC-Rand, Siphons, Wasserrohren und/oder -leitungen, Wasserzu- und/oder -abläufen, vorzugsweise in einem Temperaturbereich von etwa 20°C bis etwa 60°C, bevorzugt etwa 40°C, besonders bevorzugt etwa 30°C, wobei das Peptid wie hierin definiert ist;
- Verwendung eines Peptids in einem Wasch- und Reinigungsmittels zur Reduzierung von Schlechtgeruch von Textilien und/oder harten Oberflächen, insbesondere Geschirr, und/oder Haushaltsoberflächen, bevorzugt in Wasch- oder Geschirrspülmaschinen, Pumpensümpfen, Vorratsbehältern, Wasserreservoir, Waschtrommel, Spülkammern, Wasch- oder Spülbecken, Toilettenbecken, Spülkasten, WC-Rand, Siphons, Wasserrohren und/oder -leitungen, Wasserzu- und/oder -abläufen, die mit dem Mittel behandelt wurden, vorzugsweise in einem Temperaturbereich von etwa 20°C bis etwa 60°C, bevorzugt etwa 40°C, besonders bevorzugt etwa 30°C, wobei das Peptid wie hierin definiert ist;
- Verwendung eines Peptids in einem Wasch- und Reinigungsmittels zur Desinfektion von Textilien und/oder harten Oberflächen, insbesondere Geschirr, und/oder Haushaltsoberflächen, bevorzugt in Wasch- oder Geschirrspülmaschinen, Pumpensümpfen, Vorratsbehältern, Wasserreservoir, Waschtrommel, Spülkammern, Wasch- oder Spülbecken, Toilettenbecken, Spülkasten, WC-Rand, Siphons, Wasserrohren und/oder -leitungen, Wasserzu- und/oder -abläufen, die mit dem Mittel behandelt wurden, vorzugsweise in einem Temperaturbereich von etwa 20°C bis etwa 60°C, bevorzugt etwa 40°C, besonders bevorzugt etwa 30°C, wobei das Peptid wie hierin definiert ist;
- Verwendung eines Peptids in einem Wasch- und Reinigungsmittels zur Verhinderung und/oder Reduzierung mikrobiellen Wachstums auf Textilien und/oder harten Oberflächen, insbesondere Geschirr, und/oder Haushaltsoberflächen, bevorzugt in Wasch- oder Geschirrspülmaschinen, Pumpensümpfen, Vorratsbehältern, Wasserreservoir, Waschtrommel, Spülkammern, Wasch- oder Spülbecken, Toilettenbecken, Spülkasten, WC-Rand, Siphons, Wasserrohren und/oder -leitungen, Wasserzu- und/oder -abläufen, die mit dem Mittel behandelt wurden, vorzugsweise in einem Temperaturbereich von etwa 20°C bis etwa 60°C, bevorzugt etwa 40°C, besonders bevorzugt etwa 30°C, wobei das Peptid wie hierin definiert ist.

In einem weiteren Aspekt betrifft die Erfindung auch die Verwendung eines hierin beschriebenen Wasch- und Reinigungsmittels zur Reinigung von Textilien und/oder harten Oberflächen, insbesondere Geschirr, und/oder Haushaltsoberflächen, bevorzugt in Wasch- oder Geschirrspülmaschinen, Pumpensümpfen, Vorratsbehältern, Wasserreservoir, Waschtrommel, Spülkammern, Wasch- oder Spülbecken, Toilettenbecken, Spülkasten, WC-Rand, Siphons, Wasserrohren und/oder -leitungen, Wasserzu- und/oder -abläufen, und/oder zur Reduzierung von Schlechtgeruch von Textilien und/oder harten Oberflächen, insbesondere Geschirr, und/oder Haushaltsoberflächen, bevorzugt in Wasch- oder Geschirrspülmaschinen, Pumpensümpfen, Vorratsbehältern, Wasserreservoir, Waschtrommel, Spülkammern, Wasch- oder Spülbecken, Toilettenbecken, Spülkasten, WC-Rand, Siphons, Wasserrohren und/oder -leitungen, Wasserzu- und/oder -abläufen, die mit dem Mittel behandelt wurden, und/oder zur Desinfektion von Textilien und/oder harten Oberflächen, insbesondere Geschirr, und/oder Haushaltsoberflächen, bevorzugt in Wasch- oder Geschirrspülmaschinen, Pumpensümpfen, Vorratsbehältern, Wasserreservoir, Waschtrommel, Spülkammern, Wasch- oder Spülbecken, Toilettenbecken, Spülkasten, WC-Rand, Siphons, Wasserrohren und/oder -leitungen, Wasserzu- und/oder -abläufen, die mit dem Mittel behandelt wurden, und/oder zur Verhinderung und/oder Reduzierung mikrobiellen Wachstums auf Textilien und/oder harten Oberflächen, insbesondere Geschirr, und/oder Haushaltsoberflächen, bevorzugt in Wasch- oder Geschirrspülmaschinen, Pumpensümpfen, Vorratsbehältern, Wasserreservoir, Waschtrommel, Spülkammern, Wasch- oder Spülbecken, Toilettenbecken, Spülkasten, WC-Rand, Siphons, Wasserrohren und/oder -leitungen, Wasserzu- und/oder -abläufen, die mit dem Mittel behandelt wurden, vorzugsweise in einem Temperaturbereich von etwa 20°C bis etwa 60°C, bevorzugt etwa 40°C, besonders bevorzugt etwa 30°C.

Diese und weitere Aspekte, Merkmale und Vorteile der Erfindung werden für den Fachmann aus dem Studium der folgenden detaillierten Beschreibung und Ansprüche ersichtlich. Dabei kann jedes Merkmal aus einem Aspekt der Erfindung in jedem anderen Aspekt der Erfindung eingesetzt werden. Ferner ist es selbstverständlich, dass die hierin enthaltenen Beispiele die Erfindung beschreiben und veranschaulichen sollen, diese aber nicht einschränken und insbesondere die Erfindung nicht auf diese Beispiele beschränkt ist.

Alle Prozentangaben sind, sofern nicht anders angegeben, Gewichtsprozent (Gew.-%).

Numerische Bereiche, die in dem Format "von x bis y" angegeben sind, schließen die genannten Werte ein. Wenn mehrere bevorzugte numerische Bereiche in diesem Format angegeben sind, ist es selbstverständlich, dass alle Bereiche, die durch die Kombination der verschiedenen Endpunkte entstehen, ebenfalls erfasst werden.

"Mindestens eine", wie hierin verwendet, bedeutet eine oder mehrere, d.h. 1, 2, 3, 4, 5, 6, 7, 8, 9, 10, 11, 12, 13, 14 oder mehr.

Der Begriff "Wasch- und Reinigungsmittel" bzw. "Wasch- oder Reinigungsmittel", wie hierin verwendet, ist gleichbedeutend mit dem Begriff "Mittel" und bezeichnet eine Zusammensetzung zum Reinigen von Textilien und/oder harten Oberflächen, insbesondere Geschirr, wie in der Beschreibung erläutert. Ebenfalls umfasst sind Reinigungsmittel zum Reinigen von Oberflächen, insbesondere Haushaltsoberflächen, bevorzugt in Wasch- oder Geschirrspülmaschinen, Pumpensümpfen, Vorratsbehältern, Wasserreservoir, Waschtrommel, Spülkammern, Wasch- oder Spülbecken, Toilettenbecken, Spülkasten, WC-Rand, Siphons, Wasserrohren und/oder -leitungen, Wasserzu- und/oder -abläufen. Mit umfasst sind dabei auch Mittel für (halb-)automatisierte Wasch- oder Reinigungssysteme wie z.B. Wischrobotor oder Nassstaubsauger.

"Etwa", "ca." oder "ungefähr", wie hierin in Bezug auf einen Zahlenwert verwendet, beziehen sich auf den entsprechenden Zahlenwert ±10%, vorzugsweise ±5%.

"Flüssig", wie hierin verwendet, schließt Flüssigkeiten und Gele sowie auch pastöse Zusammensetzungen ein. Es ist bevorzugt, dass die flüssigen Zusammensetzungen bei Raumtemperatur fließfähig und gießbar sind, es ist aber auch möglich, dass sie eine Fließgrenze aufweisen.

Ein Stoff, z.B. eine Zusammensetzung oder ein Mittel ist gemäß Definition der Erfindung festförmig, wenn sie bei 25°C und 1013 mbar im festen Aggregatzustand vorliegt.

Ein Stoff, z.B. eine Zusammensetzung oder ein Mittel ist gemäß Definition der Erfindung flüssig, wenn sie bei 25°C und 1013 mbar im flüssigen Aggregatzustand vorliegt. Dabei umfasst flüssig auch gelförmig.

"Variante", wie hierin verwendet, bezieht sich auf natürliche oder artifiziell erzeugte Variationen eines nativen Enzyms oder Peptids, das eine gegenüber der Referenzform abgewandelte Aminosäuresequenz aufweist.

Der Begriff "Textil", wie hierin verwendet, bezeichnet jedes textile Material, einschließlich Garne, Garnvorprodukte, Fasern, Vliesstoffe, natürliche Materialien, synthetische Materialien und alle anderen textilen Materialien, Gewebe aus diesen Materialien und aus Geweben hergestellte Produkte (z.B. Kleidungsstücke und andere Artikel). Das Textil oder Gewebe kann in Form von Gewirken, Geweben, Denims, Vliesstoffen, Filzen, Garnen und Frottee vorliegen. Das Textil kann auf Cellulose basieren, wie z.B. natürliche Cellulosefasern wie Baumwolle, Flachs/Leinen, Jute, Ramie, Sisal oder Kokosfasern, oder künstlich hergestellte Zellulosefasern (z.B. aus Zellstoff) wie Viskose/Rayon, Celluloseacetatfasern (Tricell), Lyocell oder Mischungen davon. Das Textil oder Gewebe kann auch aus Nicht-Cellulosefasern bestehen, z.B. aus natürlichen Polyamiden wie Wolle, Kamel, Kaschmir, Mohair, Kaninchen und Seide oder aus synthetischen Polymeren wie Nylon, Aramid, Polyester, Acryl, Polypropylen und Spandex/Elasthan oder Mischungen daraus sowie aus Mischungen von Cellulosefasern und Nicht-Cellulosefasern. Beispiele für Mischungen sind Mischungen aus Baumwolle und/oder Rayon/Viskose mit einem oder mehreren Begleitmaterialien wie Wolle, synthetischen Fasern (z.B. Polyamidfasern, Acrylfasern, Polyesterfasern, Polyvinylchloridfasern, Polyurethanfasern, Polyharnstofffasern, Aramidfasern) und/oder cellulosehaltigen Fasern (z.B. Rayon/Viskose, Ramie, Flachs/Leinen, Jute, Celluloseacetatfasern, Lyocell). Bei dem Gewebe kann es sich um herkömmliche waschbare Wäsche handeln, z.B. um verschmutzte Haushaltswäsche. Wenn der Begriff "Gewebe" oder "Kleidungsstück" verwendet wird, soll er auch den weiter gefassten Begriff "Textilien" umfassen.

Die vorliegende Erfindung basiert auf der überraschenden Erkenntnis der Erfinder, dass erfindungsgemäße Peptide das Wachstum von Mikroorganismen, wie z.B. Bakterien, Pilzen oder Viren, verhindern und/oder verringern, und/oder die Anzahl lebensfähiger und/oder vermehrungsfähiger individueller Mikroorganismen reduzieren.

Erfindungsgemäß wird ein Peptid, das eine Länge von 10 bis 50, vorzugsweise 10 bis 30, bevorzugt 12 bis 25, Aminosäuren aufweist, die aus der aus Alanin, Arginin, Asparagin, Asparaginsäure, Cystein, Glutamin, Glutaminsäure, Glycin, Histidin, Isoleucin, Leucin, Lysin, Methionin, Phenylalanin, Prolin, Serin, Threonin, Tryptophan, Tyrosin und Valin bestehenden Gruppe ausgewählt sind, vorzugsweise ausgewählt aus Alanin, Arginin, Glutaminsäure, Glycin, Isoleucin, Leucin, Lysin, Phenylalanin, Prolin, Serin, Threonin, Tryptophan, Tyrosin und Valin, eine Nettoladung von +2 bis +8, vorzugsweise +3 bis +7, bevorzugt +4 bis +6, aufweist, und antimikrobiell wirksam ist, wobei die antimikrobielle Wirksamkeit wie in Beispiel 1 beschrieben bestimmt wird, in einem Wasch- und Reinigungsmittel angewendet.

Die Anwendung eines erfindungsgemäßen Peptids in Wasch- und Reinigungsmittel bzw. die Anwendung eines erfindungsgemäßen Wasch- und Reinigungsmittels führt zu einer verbesserten Reinigung von Textilien und/oder harten Oberflächen, insbesondere Geschirr, und/oder Haushaltsoberflächen, bevorzugt in Wasch- oder Geschirrspülmaschinen, Pumpensümpfen, Vorratsbehältern, Wasserreservoir, Waschtrommel, Spülkammern, Wasch- oder Spülbecken, Toilettenbecken, Spülkasten, WC-Rand, Siphons, Wasserrohren und/oder -leitungen, Wasserzu- und/oder -abläufen, und/oder zur Reduzierung von Schlechtgeruch von Textilien und/oder harten Oberflächen, insbesondere Geschirr, und/oder Haushaltsoberflächen, bevorzugt in Wasch- oder Geschirrspülmaschinen, Pumpensümpfen, Vorratsbehältern, Wasserreservoir, Waschtrommel, Spülkammern, Wasch- oder Spülbecken, Toilettenbecken, Spülkasten, WC-Rand, Siphons, Wasserrohren und/oder -leitungen, Wasserzu- und/oder -abläufen, und/oder zur Verhinderung und/oder Reduzierung mikrobiellen Wachstums auf Textilien und/oder harten Oberflächen, insbesondere Geschirr, und/oder Haushaltsoberflächen, bevorzugt in Wasch- oder Geschirrspülmaschinen, Pumpensümpfen, Vorratsbehältern, Wasserreservoir, Waschtrommel, Spülkammern, Wasch- oder Spülbecken, Toilettenbecken, Spülkasten, WC-Rand, Siphons, Wasserrohren und/oder -leitungen, Wasserzu- und/oder -abläufen.

Das ist insbesondere insoweit überraschend, als bisher die Anwendung solcher Peptide in Wasch- und Reinigungsmittel nicht beschrieben wurde. Zudem wurden auch die hierin beschriebenen Effekte, die solche Peptide auf Textilien und/oder harten Oberflächen, insbesondere Geschirr, und/oder Haushaltsoberflächen verwirklichen, nicht beschrieben, insbesondere dass solche Peptide das Wachstum von Mikroorganismen verhindern und/oder reduzieren und/oder dazu beitragen, die Wasch- und Reinigungsleistung hygienisch zu verbessern, und/oder mikrobiell bedingten Schlechtgeruch sowie mikrobiell bedingte Ablagerungen (Biofilme, Biobeläge und dergleichen) zu verhindern.

Erfindungsgemäß wird ein Peptid verwendet, das eine Länge von 10 bis 50, vorzugsweise 10 bis 30, bevorzugt 12 bis 25, Aminosäuren aufweist, die aus der aus Alanin, Arginin, Asparagin, Asparaginsäure, Cystein, Glutamin, Glutaminsäure, Glycin, Histidin, Isoleucin, Leucin, Lysin, Methionin, Phenylalanin, Prolin, Serin, Threonin, Tryptophan, Tyrosin und Valin bestehenden Gruppe ausgewählt sind, vorzugsweise ausgewählt aus Alanin, Arginin, Glutaminsäure, Glycin, Isoleucin, Leucin, Lysin, Phenylalanin, Prolin, Serin, Threonin, Tryptophan, Tyrosin und Valin, eine Nettoladung von +2 bis +8, vorzugsweise +3 bis +7, bevorzugt +4 bis +6, aufweist, und antimikrobiell wirksam ist, wobei die antimikrobielle Wirksamkeit wie in Beispiel 1 beschrieben bestimmt wird.

In bevorzugten Ausführungsformen umfasst das Peptid mindestens 1 bis 5 Tryptophan-Reste und/oder 1 bis 5 Arginin-Reste und/oder 1 bis 5 Lysin-Reste, vorzugsweise mindestens 1 bis 4 Tryptophan-Reste und/oder 1 bis 3 Arginin-Reste und/oder 1 bis 5 Lysin-Reste.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% oder 100% identisch ist.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 10 bis 50, vorzugsweise 10 bis 30, bevorzugt 12 bis 25, Aminosäuren aufweist, die aus der aus Alanin, Arginin, Asparagin, Asparaginsäure, Cystein, Glutamin, Glutaminsäure, Glycin, Histidin, Isoleucin, Leucin, Lysin, Methionin, Phenylalanin, Prolin, Serin, Threonin, Tryptophan, Tyrosin und Valin bestehenden Gruppe ausgewählt sind, vorzugsweise ausgewählt aus Alanin, Arginin, Glutaminsäure, Glycin, Isoleucin, Leucin, Lysin, Phenylalanin, Prolin, Serin, Threonin, Tryptophan, Tyrosin und Valin, eine Nettoladung von +2 bis +8, vorzugsweise +3 bis +7, bevorzugt +4 bis +6, aufweist, und antimikrobiell wirksam ist, wobei die antimikrobielle Wirksamkeit wie in Beispiel 1 beschrieben bestimmt wird.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 10 bis 50 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +2 bis +8 aufweist, und antimikrobiell wirksam ist, wobei die antimikrobielle Wirksamkeit wie in Beispiel 1 beschrieben bestimmt wird.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 10 bis 50 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +3 bis +7 aufweist, und antimikrobiell wirksam ist, wobei die antimikrobielle Wirksamkeit wie in Beispiel 1 beschrieben bestimmt wird.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 10 bis 50 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +4 bis +6 aufweist, und antimikrobiell wirksam ist, wobei die antimikrobielle Wirksamkeit wie in Beispiel 1 beschrieben bestimmt wird.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 10 bis 30 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +2 bis +8 aufweist, und antimikrobiell wirksam ist, wobei die antimikrobielle Wirksamkeit wie in Beispiel 1 beschrieben bestimmt wird.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 10 bis 30 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +3 bis +7 aufweist, und antimikrobiell wirksam ist, wobei die antimikrobielle Wirksamkeit wie in Beispiel 1 beschrieben bestimmt wird.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 10 bis 30 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +4 bis +6 aufweist, und antimikrobiell wirksam ist, wobei die antimikrobielle Wirksamkeit wie in Beispiel 1 beschrieben bestimmt wird.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 12 bis 25 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +2 bis +8 aufweist, und antimikrobiell wirksam ist, wobei die antimikrobielle Wirksamkeit wie in Beispiel 1 beschrieben bestimmt wird.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 12 bis 25 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +3 bis +7 aufweist, und antimikrobiell wirksam ist, wobei die antimikrobielle Wirksamkeit wie in Beispiel 1 beschrieben bestimmt wird.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 12 bis 25 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +4 bis +6 aufweist, und antimikrobiell wirksam ist, wobei die antimikrobielle Wirksamkeit wie in Beispiel 1 beschrieben bestimmt wird.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 10 bis 50, vorzugsweise 10 bis 30, bevorzugt 12 bis 25, Aminosäuren aufweist, die aus der aus Alanin, Arginin, Asparagin, Asparaginsäure, Cystein, Glutamin, Glutaminsäure, Glycin, Histidin, Isoleucin, Leucin, Lysin, Methionin, Phenylalanin, Prolin, Serin, Threonin, Tryptophan, Tyrosin und Valin bestehenden Gruppe ausgewählt sind, vorzugsweise ausgewählt aus Alanin, Arginin, Glutaminsäure, Glycin, Isoleucin, Leucin, Lysin, Phenylalanin, Prolin, Serin, Threonin, Tryptophan, Tyrosin und Valin, eine Nettoladung von +2 bis +8, vorzugsweise +3 bis +7, bevorzugt +4 bis +6, aufweist, und antimikrobiell wirksam ist, wobei die antimikrobielle Wirksamkeit wie in Beispiel 1 beschrieben bestimmt wird, wobei das Peptid mindestens 1 bis 5 Tryptophan-Reste und/oder 1 bis 5 Arginin-Reste und/oder 1 bis 5 Lysin-Reste, vorzugsweise mindestens 1 bis 4 Tryptophan-Reste und/oder 1 bis 3 Arginin-Reste und/oder 1 bis 5 Lysin-Reste, umfasst.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 10 bis 50 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +2 bis +8 aufweist, und antimikrobiell wirksam ist, wobei die antimikrobielle Wirksamkeit wie in Beispiel 1 beschrieben bestimmt wird, wobei das Peptid mindestens 1 bis 4 Tryptophan-Reste und/oder 1 bis 3 Arginin-Reste und/oder 1 bis 5 Lysin-Reste umfasst.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 10 bis 50 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +3 bis +7 aufweist, und antimikrobiell wirksam ist, wobei die antimikrobielle Wirksamkeit wie in Beispiel 1 beschrieben bestimmt wird, wobei das Peptid mindestens 1 bis 4 Tryptophan-Reste und/oder 1 bis 3 Arginin-Reste und/oder 1 bis 5 Lysin-Reste umfasst.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 10 bis 50 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +4 bis +6 aufweist, und antimikrobiell wirksam ist, wobei die antimikrobielle Wirksamkeit wie in Beispiel 1 beschrieben bestimmt wird, wobei das Peptid mindestens 1 bis 4 Tryptophan-Reste und/oder 1 bis 3 Arginin-Reste und/oder 1 bis 5 Lysin-Reste umfasst.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 10 bis 30 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +2 bis +8 aufweist, und antimikrobiell wirksam ist, wobei die antimikrobielle Wirksamkeit wie in Beispiel 1 beschrieben bestimmt wird, wobei das Peptid mindestens 1 bis 4 Tryptophan-Reste und/oder 1 bis 3 Arginin-Reste und/oder 1 bis 5 Lysin-Reste umfasst.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 10 bis 30 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +3 bis +7 aufweist, und antimikrobiell wirksam ist, wobei die antimikrobielle Wirksamkeit wie in Beispiel 1 beschrieben bestimmt wird, wobei das Peptid mindestens 1 bis 4 Tryptophan-Reste und/oder 1 bis 3 Arginin-Reste und/oder 1 bis 5 Lysin-Reste umfasst.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 10 bis 30 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +4 bis +6 aufweist, und antimikrobiell wirksam ist, wobei die antimikrobielle Wirksamkeit wie in Beispiel 1 beschrieben bestimmt wird, wobei das Peptid mindestens 1 bis 4 Tryptophan-Reste und/oder 1 bis 3 Arginin-Reste und/oder 1 bis 5 Lysin-Reste umfasst.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 12 bis 25 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +2 bis +8 aufweist, und antimikrobiell wirksam ist, wobei die antimikrobielle Wirksamkeit wie in Beispiel 1 beschrieben bestimmt wird, wobei das Peptid mindestens 1 bis 4 Tryptophan-Reste und/oder 1 bis 3 Arginin-Reste und/oder 1 bis 5 Lysin-Reste umfasst.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 12 bis 25 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +3 bis +7 aufweist, und antimikrobiell wirksam ist, wobei die antimikrobielle Wirksamkeit wie in Beispiel 1 beschrieben bestimmt wird, wobei das Peptid mindestens 1 bis 4 Tryptophan-Reste und/oder 1 bis 3 Arginin-Reste und/oder 1 bis 5 Lysin-Reste umfasst.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 12 bis 25 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +4 bis +6 aufweist, und antimikrobiell wirksam ist, wobei die antimikrobielle Wirksamkeit wie in Beispiel 1 beschrieben bestimmt wird, wobei das Peptid mindestens 1 bis 4 Tryptophan-Reste und/oder 1 bis 3 Arginin-Reste und/oder 1 bis 5 Lysin-Reste umfasst.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 80% und zunehmend bevorzugt zu mindestens 81%, 82%, 83%, 84%, 85%, 86%, 87%, 88%, 89%, 90%, 91%, 92%, 93%, 94%, 95%, 96%, 97%, 98%, 99% oder 100% identisch ist.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 80% und zunehmend bevorzugt zu mindestens 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 10 bis 50, vorzugsweise 10 bis 30, bevorzugt 12 bis 25, Aminosäuren aufweist, die aus der aus Alanin, Arginin, Asparagin, Asparaginsäure, Cystein, Glutamin, Glutaminsäure, Glycin, Histidin, Isoleucin, Leucin, Lysin, Methionin, Phenylalanin, Prolin, Serin, Threonin, Tryptophan, Tyrosin und Valin bestehenden Gruppe ausgewählt sind, vorzugsweise ausgewählt aus Alanin, Arginin, Glutaminsäure, Glycin, Isoleucin, Leucin, Lysin, Phenylalanin, Prolin, Serin, Threonin, Tryptophan, Tyrosin und Valin, eine Nettoladung von +2 bis +8, vorzugsweise +3 bis +7, bevorzugt +4 bis +6, aufweist, und antimikrobiell wirksam ist, wobei die antimikrobielle Wirksamkeit wie in Beispiel 1 beschrieben bestimmt wird.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 80% und zunehmend bevorzugt zu mindestens 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 10 bis 50 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +2 bis +8 aufweist, und antimikrobiell wirksam ist, wobei die antimikrobielle Wirksamkeit wie in Beispiel 1 beschrieben bestimmt wird.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 80% und zunehmend bevorzugt zu mindestens 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 10 bis 50 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +3 bis +7 aufweist, und antimikrobiell wirksam ist, wobei die antimikrobielle Wirksamkeit wie in Beispiel 1 beschrieben bestimmt wird.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens zu mindestens 80% und zunehmend bevorzugt zu mindestens 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 10 bis 50 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +4 bis +6 aufweist, und antimikrobiell wirksam ist, wobei die antimikrobielle Wirksamkeit wie in Beispiel 1 beschrieben bestimmt wird.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 80% und zunehmend bevorzugt zu mindestens 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 10 bis 30 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +2 bis +8 aufweist, und antimikrobiell wirksam ist, wobei die antimikrobielle Wirksamkeit wie in Beispiel 1 beschrieben bestimmt wird.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens zu mindestens 80% und zunehmend bevorzugt zu mindestens 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 10 bis 30 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +3 bis +7 aufweist, und antimikrobiell wirksam ist, wobei die antimikrobielle Wirksamkeit wie in Beispiel 1 beschrieben bestimmt wird.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 80% und zunehmend bevorzugt zu mindestens 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 10 bis 30 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +4 bis +6 aufweist, und antimikrobiell wirksam ist, wobei die antimikrobielle Wirksamkeit wie in Beispiel 1 beschrieben bestimmt wird.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 80% und zunehmend bevorzugt zu mindestens 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 12 bis 25 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +2 bis +8 aufweist, und antimikrobiell wirksam ist, wobei die antimikrobielle Wirksamkeit wie in Beispiel 1 beschrieben bestimmt wird.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 80% und zunehmend bevorzugt zu mindestens 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 12 bis 25 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +3 bis +7 aufweist, und antimikrobiell wirksam ist, wobei die antimikrobielle Wirksamkeit wie in Beispiel 1 beschrieben bestimmt wird.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 80% und zunehmend bevorzugt zu mindestens 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 12 bis 25 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +4 bis +6 aufweist, und antimikrobiell wirksam ist, wobei die antimikrobielle Wirksamkeit wie in Beispiel 1 beschrieben bestimmt wird.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 80% und zunehmend bevorzugt zu mindestens 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 10 bis 50, vorzugsweise 10 bis 30, bevorzugt 12 bis 25, Aminosäuren aufweist, die aus der aus Alanin, Arginin, Asparagin, Asparaginsäure, Cystein, Glutamin, Glutaminsäure, Glycin, Histidin, Isoleucin, Leucin, Lysin, Methionin, Phenylalanin, Prolin, Serin, Threonin, Tryptophan, Tyrosin und Valin bestehenden Gruppe ausgewählt sind, vorzugsweise ausgewählt aus Alanin, Arginin, Glutaminsäure, Glycin, Isoleucin, Leucin, Lysin, Phenylalanin, Prolin, Serin, Threonin, Tryptophan, Tyrosin und Valin, eine Nettoladung von +2 bis +8, vorzugsweise +3 bis +7, bevorzugt +4 bis +6, aufweist, und antimikrobiell wirksam ist, wobei die antimikrobielle Wirksamkeit wie in Beispiel 1 beschrieben bestimmt wird, wobei das Peptid mindestens 1 bis 5 Tryptophan-Reste und/oder 1 bis 5 Arginin-Reste und/oder 1 bis 5 Lysin-Reste, vorzugsweise mindestens 1 bis 4 Tryptophan-Reste und/oder 1 bis 3 Arginin-Reste und/oder 1 bis 5 Lysin-Reste, umfasst.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 80% und zunehmend bevorzugt zu mindestens 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 10 bis 50 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +2 bis +8 aufweist, und antimikrobiell wirksam ist, wobei die antimikrobielle Wirksamkeit wie in Beispiel 1 beschrieben bestimmt wird, wobei das Peptid mindestens 1 bis 4 Tryptophan-Reste und/oder 1 bis 3 Arginin-Reste und/oder 1 bis 5 Lysin-Reste umfasst.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 80% und zunehmend bevorzugt zu mindestens 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 10 bis 50 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +3 bis +7 aufweist, und antimikrobiell wirksam ist, wobei die antimikrobielle Wirksamkeit wie in Beispiel 1 beschrieben bestimmt wird, wobei das Peptid mindestens 1 bis 4 Tryptophan-Reste und/oder 1 bis 3 Arginin-Reste und/oder 1 bis 5 Lysin-Reste umfasst.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 80% und zunehmend bevorzugt zu mindestens 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 10 bis 50 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +4 bis +6 aufweist, und antimikrobiell wirksam ist, wobei die antimikrobielle Wirksamkeit wie in Beispiel 1 beschrieben bestimmt wird, wobei das Peptid mindestens 1 bis 4 Tryptophan-Reste und/oder 1 bis 3 Arginin-Reste und/oder 1 bis 5 Lysin-Reste umfasst.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 80% und zunehmend bevorzugt zu mindestens 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 10 bis 30 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +2 bis +8 aufweist, und antimikrobiell wirksam ist, wobei die antimikrobielle Wirksamkeit wie in Beispiel 1 beschrieben bestimmt wird, wobei das Peptid mindestens 1 bis 4 Tryptophan-Reste und/oder 1 bis 3 Arginin-Reste und/oder 1 bis 5 Lysin-Reste umfasst.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 80% und zunehmend bevorzugt zu mindestens 80%, 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 10 bis 30 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +3 bis +7 aufweist, und antimikrobiell wirksam ist, wobei die antimikrobielle Wirksamkeit wie in Beispiel 1 beschrieben bestimmt wird, wobei das Peptid mindestens 1 bis 4 Tryptophan-Reste und/oder 1 bis 3 Arginin-Reste und/oder 1 bis 5 Lysin-Reste umfasst.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 80% und zunehmend bevorzugt zu mindestens 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 10 bis 30 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +4 bis +6 aufweist, und antimikrobiell wirksam ist, wobei die antimikrobielle Wirksamkeit wie in Beispiel 1 beschrieben bestimmt wird, wobei das Peptid mindestens 1 bis 4 Tryptophan-Reste und/oder 1 bis 3 Arginin-Reste und/oder 1 bis 5 Lysin-Reste umfasst.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 80% und zunehmend bevorzugt zu mindestens 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 12 bis 25 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +2 bis +8 aufweist, und antimikrobiell wirksam ist, wobei die antimikrobielle Wirksamkeit wie in Beispiel 1 beschrieben bestimmt wird, wobei das Peptid mindestens 1 bis 4 Tryptophan-Reste und/oder 1 bis 3 Arginin-Reste und/oder 1 bis 5 Lysin-Reste umfasst.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 80% und zunehmend bevorzugt zu mindestens 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 12 bis 25 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +3 bis +7 aufweist, und antimikrobiell wirksam ist, wobei die antimikrobielle Wirksamkeit wie in Beispiel 1 beschrieben bestimmt wird, wobei das Peptid mindestens 1 bis 4 Tryptophan-Reste und/oder 1 bis 3 Arginin-Reste und/oder 1 bis 5 Lysin-Reste umfasst.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 80% und zunehmend bevorzugt zu mindestens 81%, 82%, 83%, 84%, 85%, 86%, 87%, 88%, 89%, 90%, 91%, 92%, 93%, 94%, 95%, 96%, 97%, 98%, 99% oder 100% identisch ist, wobei das Peptid eine Länge von 12 bis 25 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +4 bis +6 aufweist, und antimikrobiell wirksam ist, wobei die antimikrobielle Wirksamkeit wie in Beispiel 1 beschrieben bestimmt wird, wobei das Peptid mindestens 1 bis 4 Tryptophan-Reste und/oder 1 bis 3 Arginin-Reste und/oder 1 bis 5 Lysin-Reste umfasst.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 90% und zunehmend bevorzugt zu mindestens 91%, 92%, 93%, 94%, 95%, 96%, 97%, 98%, 99% oder 100% identisch ist.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 90% und zunehmend bevorzugt zu mindestens 91%, 92%, 93%, 94%, 95%, 96%, 97%, 98%, 99% oder 100% identisch ist, wobei das Peptid eine Länge von 10 bis 50, vorzugsweise 10 bis 30, bevorzugt 12 bis 25, Aminosäuren aufweist, die aus der aus Alanin, Arginin, Asparagin, Asparaginsäure, Cystein, Glutamin, Glutaminsäure, Glycin, Histidin, Isoleucin, Leucin, Lysin, Methionin, Phenylalanin, Prolin, Serin, Threonin, Tryptophan, Tyrosin und Valin bestehenden Gruppe ausgewählt sind, vorzugsweise ausgewählt aus Alanin, Arginin, Glutaminsäure, Glycin, Isoleucin, Leucin, Lysin, Phenylalanin, Prolin, Serin, Threonin, Tryptophan, Tyrosin und Valin, eine Nettoladung von +2 bis +8, vorzugsweise +3 bis +7, bevorzugt +4 bis +6, aufweist, und antimikrobiell wirksam ist, wobei die antimikrobielle Wirksamkeit wie in Beispiel 1 beschrieben bestimmt wird.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 90% und zunehmend bevorzugt zu mindestens 91%, 92%, 93%, 94%, 95%, 96%, 97%, 98%, 99% oder 100% identisch ist, wobei das Peptid eine Länge von 10 bis 50 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +2 bis +8 aufweist, und antimikrobiell wirksam ist, wobei die antimikrobielle Wirksamkeit wie in Beispiel 1 beschrieben bestimmt wird.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 90% und zunehmend bevorzugt zu mindestens 91%, 92%, 93%, 94%, 95%, 96%, 97%, 98%, 99% oder 100% identisch ist, wobei das Peptid eine Länge von 10 bis 50 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +3 bis +7 aufweist, und antimikrobiell wirksam ist, wobei die antimikrobielle Wirksamkeit wie in Beispiel 1 beschrieben bestimmt wird.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 90% und zunehmend bevorzugt zu mindestens 91%, 92%, 93%, 94%, 95%, 96%, 97%, 98%, 99% oder 100% identisch ist, wobei das Peptid eine Länge von 10 bis 50 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +4 bis +6 aufweist, und antimikrobiell wirksam ist, wobei die antimikrobielle Wirksamkeit wie in Beispiel 1 beschrieben bestimmt wird.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 90% und zunehmend bevorzugt zu mindestens 91%, 92%, 93%, 94%, 95%, 96%, 97%, 98%, 99% oder 100% identisch ist, wobei das Peptid eine Länge von 10 bis 30 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +2 bis +8 aufweist, und antimikrobiell wirksam ist, wobei die antimikrobielle Wirksamkeit wie in Beispiel 1 beschrieben bestimmt wird.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 90% und zunehmend bevorzugt zu mindestens 91%, 92%, 93%, 94%, 95%, 96%, 97%, 98%, 99% oder 100% identisch ist, wobei das Peptid eine Länge von 10 bis 30 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +3 bis +7 aufweist, und antimikrobiell wirksam ist, wobei die antimikrobielle Wirksamkeit wie in Beispiel 1 beschrieben bestimmt wird.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 90% und zunehmend bevorzugt zu mindestens 91%, 92%, 93%, 94%, 95%, 96%, 97%, 98%, 99% oder 100% identisch ist, wobei das Peptid eine Länge von 10 bis 30 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +4 bis +6 aufweist, und antimikrobiell wirksam ist, wobei die antimikrobielle Wirksamkeit wie in Beispiel 1 beschrieben bestimmt wird.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 90% und zunehmend bevorzugt zu mindestens 91%, 92%, 93%, 94%, 95%, 96%, 97%, 98%, 99% oder 100% identisch ist, wobei das Peptid eine Länge von 12 bis 25 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +2 bis +8 aufweist, und antimikrobiell wirksam ist, wobei die antimikrobielle Wirksamkeit wie in Beispiel 1 beschrieben bestimmt wird.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 90% und zunehmend bevorzugt zu mindestens 91%, 92%, 93%, 94%, 95%, 96%, 97%, 98%, 99% oder 100% identisch ist, wobei das Peptid eine Länge von 12 bis 25 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +3 bis +7 aufweist, und antimikrobiell wirksam ist, wobei die antimikrobielle Wirksamkeit wie in Beispiel 1 beschrieben bestimmt wird.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 90% und zunehmend bevorzugt zu mindestens 91%, 92%, 93%, 94%, 95%, 96%, 97%, 98%, 99% oder 100% identisch ist, wobei das Peptid eine Länge von 12 bis 25 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +4 bis +6 aufweist, und antimikrobiell wirksam ist, wobei die antimikrobielle Wirksamkeit wie in Beispiel 1 beschrieben bestimmt wird.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 90% und zunehmend bevorzugt zu mindestens 91%, 92%, 93%, 94%, 95%, 96%, 97%, 98%, 99% oder 100% identisch ist, wobei das Peptid eine Länge von 10 bis 50, vorzugsweise 10 bis 30, bevorzugt 12 bis 25, Aminosäuren aufweist, die aus der aus Alanin, Arginin, Asparagin, Asparaginsäure, Cystein, Glutamin, Glutaminsäure, Glycin, Histidin, Isoleucin, Leucin, Lysin, Methionin, Phenylalanin, Prolin, Serin, Threonin, Tryptophan, Tyrosin und Valin bestehenden Gruppe ausgewählt sind, vorzugsweise ausgewählt aus Alanin, Arginin, Glutaminsäure, Glycin, Isoleucin, Leucin, Lysin, Phenylalanin, Prolin, Serin, Threonin, Tryptophan, Tyrosin und Valin, eine Nettoladung von +2 bis +8, vorzugsweise +3 bis +7, bevorzugt +4 bis +6, aufweist, und antimikrobiell wirksam ist, wobei die antimikrobielle Wirksamkeit wie in Beispiel 1 beschrieben bestimmt wird, wobei das Peptid mindestens 1 bis 5 Tryptophan-Reste und/oder 1 bis 5 Arginin-Reste und/oder 1 bis 5 Lysin-Reste, vorzugsweise mindestens 1 bis 4 Tryptophan-Reste und/oder 1 bis 3 Arginin-Reste und/oder 1 bis 5 Lysin-Reste, umfasst.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 90% und zunehmend bevorzugt zu mindestens 91%, 92%, 93%, 94%, 95%, 96%, 97%, 98%, 99% oder 100% identisch ist, wobei das Peptid eine Länge von 10 bis 50 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +2 bis +8 aufweist, und antimikrobiell wirksam ist, wobei die antimikrobielle Wirksamkeit wie in Beispiel 1 beschrieben bestimmt wird, wobei das Peptid mindestens 1 bis 4 Tryptophan-Reste und/oder 1 bis 3 Arginin-Reste und/oder 1 bis 5 Lysin-Reste umfasst.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 90% und zunehmend bevorzugt zu mindestens 91%, 92%, 93%, 94%, 95%, 96%, 97%, 98%, 99% oder 100% identisch ist, wobei das Peptid eine Länge von 10 bis 50 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +3 bis +7 aufweist, und antimikrobiell wirksam ist, wobei die antimikrobielle Wirksamkeit wie in Beispiel 1 beschrieben bestimmt wird, wobei das Peptid mindestens 1 bis 4 Tryptophan-Reste und/oder 1 bis 3 Arginin-Reste und/oder 1 bis 5 Lysin-Reste umfasst.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 90% und zunehmend bevorzugt zu mindestens 91%, 92%, 93%, 94%, 95%, 96%, 97%, 98%, 99% oder 100% identisch ist, wobei das Peptid eine Länge von 10 bis 50 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +4 bis +6 aufweist, und antimikrobiell wirksam ist, wobei die antimikrobielle Wirksamkeit wie in Beispiel 1 beschrieben bestimmt wird, wobei das Peptid mindestens 1 bis 4 Tryptophan-Reste und/oder 1 bis 3 Arginin-Reste und/oder 1 bis 5 Lysin-Reste umfasst.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 90% und zunehmend bevorzugt zu mindestens 91%, 92%, 93%, 94%, 95%, 96%, 97%, 98%, 99% oder 100% identisch ist, wobei das Peptid eine Länge von 10 bis 30 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +2 bis +8 aufweist, und antimikrobiell wirksam ist, wobei die antimikrobielle Wirksamkeit wie in Beispiel 1 beschrieben bestimmt wird, wobei das Peptid mindestens 1 bis 4 Tryptophan-Reste und/oder 1 bis 3 Arginin-Reste und/oder 1 bis 5 Lysin-Reste umfasst.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 90% und zunehmend bevorzugt zu mindestens 91%, 92%, 93%, 94%, 95%, 96%, 97%, 98%, 99% oder 100% identisch ist, wobei das Peptid eine Länge von 10 bis 30 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +3 bis +7 aufweist, und antimikrobiell wirksam ist, wobei die antimikrobielle Wirksamkeit wie in Beispiel 1 beschrieben bestimmt wird, wobei das Peptid mindestens 1 bis 4 Tryptophan-Reste und/oder 1 bis 3 Arginin-Reste und/oder 1 bis 5 Lysin-Reste umfasst.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 90% und zunehmend bevorzugt zu mindestens 91%, 92%, 93%, 94%, 95%, 96%, 97%, 98%, 99% oder 100% identisch ist, wobei das Peptid eine Länge von 10 bis 30 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +4 bis +6 aufweist, und antimikrobiell wirksam ist, wobei die antimikrobielle Wirksamkeit wie in Beispiel 1 beschrieben bestimmt wird, wobei das Peptid mindestens 1 bis 4 Tryptophan-Reste und/oder 1 bis 3 Arginin-Reste und/oder 1 bis 5 Lysin-Reste umfasst.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 90% und zunehmend bevorzugt zu mindestens 91%, 92%, 93%, 94%, 95%, 96%, 97%, 98%, 99% oder 100% identisch ist, wobei das Peptid eine Länge von 12 bis 25 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +2 bis +8 aufweist, und antimikrobiell wirksam ist, wobei die antimikrobielle Wirksamkeit wie in Beispiel 1 beschrieben bestimmt wird, wobei das Peptid mindestens 1 bis 4 Tryptophan-Reste und/oder 1 bis 3 Arginin-Reste und/oder 1 bis 5 Lysin-Reste umfasst.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 90% und zunehmend bevorzugt zu mindestens 91%, 92%, 93%, 94%, 95%, 96%, 97%, 98%, 99% oder 100% identisch ist, wobei das Peptid eine Länge von 12 bis 25 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +3 bis +7 aufweist, und antimikrobiell wirksam ist, wobei die antimikrobielle Wirksamkeit wie in Beispiel 1 beschrieben bestimmt wird, wobei das Peptid mindestens 1 bis 4 Tryptophan-Reste und/oder 1 bis 3 Arginin-Reste und/oder 1 bis 5 Lysin-Reste umfasst.

In bevorzugten Ausführungsformen umfasst das Peptid eine Aminosäuresequenz, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 90% und zunehmend bevorzugt zu mindestens 91%, 92%, 93%, 94%, 95%, 96%, 97%, 98%, 99% oder 100% identisch ist, wobei das Peptid eine Länge von 12 bis 25 Aminosäuren aufweist, die aus der aus Alanin, Arginin, Glutaminsäure, Glycin, Isoleucin, Leucin, Lysin, Phenylalanin, Prolin, Serin, Threonin, Tryptophan, Tyrosin und Valin bestehenden Gruppe ausgewählt sind, eine Nettoladung von +4 bis +6 aufweist, und antimikrobiell wirksam ist, wobei die antimikrobielle Wirksamkeit wie in Beispiel 1 beschrieben bestimmt wird, wobei das Peptid mindestens 1 bis 4 Tryptophan-Reste und/oder 1 bis 3 Arginin-Reste und/oder 1 bis 5 Lysin-Reste umfasst.

In besonders bevorzugten Ausführungsformen umfasst das Peptid eine Aminosäuresequenz, die identisch zu einer der in SEQ ID NO:1, SEQ ID NO:2, SEQ ID NO:3, SEQ ID NO:4 oder SEQ ID NO:5 angegebenen Aminosäuresequenzen ist. In besonders bevorzugten Ausführungsformen umfasst das Peptid eine Aminosäuresequenz, die identisch zu einer der in SEQ ID NO:1, SEQ ID NO:2, SEQ ID NO:3, SEQ ID NO:4 oder SEQ ID NO:5 angegebenen Aminosäuresequenzen ist, wobei das Peptid antimikrobiell wirksam ist, wobei die antimikrobielle Wirksamkeit wie in Beispiel 1 beschrieben bestimmt wird.

In besonders bevorzugten Ausführungsformen umfasst das Peptid eine Aminosäuresequenz, die identisch zu einer der in SEQ ID NO:1 angegebenen Aminosäuresequenzen ist. In besonders bevorzugten Ausführungsformen umfasst das Peptid eine Aminosäuresequenz, die identisch zu einer der in SEQ ID NO:1 angegebenen Aminosäuresequenzen ist, wobei das Peptid antimikrobiell wirksam ist, wobei die antimikrobielle Wirksamkeit wie in Beispiel 1 beschrieben bestimmt wird.

In besonders bevorzugten Ausführungsformen umfasst das Peptid eine Aminosäuresequenz, die identisch zu einer der in SEQ ID NO:2 angegebenen Aminosäuresequenzen ist. In besonders bevorzugten Ausführungsformen umfasst das Peptid eine Aminosäuresequenz, die identisch zu einer der in SEQ ID NO:2 angegebenen Aminosäuresequenzen ist, wobei das Peptid antimikrobiell wirksam ist, wobei die antimikrobielle Wirksamkeit wie in Beispiel 1 beschrieben bestimmt wird.

In besonders bevorzugten Ausführungsformen umfasst das Peptid eine Aminosäuresequenz, die identisch zu einer der in SEQ ID NO:3 angegebenen Aminosäuresequenzen ist. In besonders bevorzugten Ausführungsformen umfasst das Peptid eine Aminosäuresequenz, die identisch zu einer der in SEQ ID NO:3 angegebenen Aminosäuresequenzen ist, wobei das Peptid antimikrobiell wirksam ist, wobei die antimikrobielle Wirksamkeit wie in Beispiel 1 beschrieben bestimmt wird.

In besonders bevorzugten Ausführungsformen umfasst das Peptid eine Aminosäuresequenz, die identisch zu einer der in SEQ ID NO:4 angegebenen Aminosäuresequenzen ist. In besonders bevorzugten Ausführungsformen umfasst das Peptid eine Aminosäuresequenz, die identisch zu einer der in SEQ ID NO:4 angegebenen Aminosäuresequenzen ist, wobei das Peptid antimikrobiell wirksam ist, wobei die antimikrobielle Wirksamkeit wie in Beispiel 1 beschrieben bestimmt wird.

In besonders bevorzugten Ausführungsformen umfasst das Peptid eine Aminosäuresequenz, die identisch zu einer der in SEQ ID NO:5 angegebenen Aminosäuresequenzen ist. In besonders bevorzugten Ausführungsformen umfasst das Peptid eine Aminosäuresequenz, die identisch zu einer der in SEQ ID NO:5 angegebenen Aminosäuresequenzen ist, wobei das Peptid antimikrobiell wirksam ist, wobei die antimikrobielle Wirksamkeit wie in Beispiel 1 beschrieben bestimmt wird.

Peptide und Proteine sind üblicherweise Ketten aus Aminosäuren, wobei die einzelnen Aminosäuren über Peptidbindungen miteinander verknüpft sind. Ketten aus zwei Aminosäuren nennt man Dipeptide, Ketten aus drei Aminosäuren Tripeptide, Ketten aus vier Aminosäuren Tetrapeptide, usw. Ketten aus wenigen bis zu ca. 100 Aminosäuren nennt man Oligopeptide (bis zu ca. 10 Aminosäuren) oder Polypeptide (ca. 10 bis 100 Aminosäuren) oder einfach Peptide. Bei Ketten von mehr als ca. 100 Aminosäuren spricht man üblicherweise von Proteinen.

Erfindungsgemäße Peptide weisen keine katalytische Aktivität auf, wie sie für Enzyme typisch ist. Die Definition erfindungsgemäßer Peptide umfasst daher keine Enzyme. Während Enzyme mitunter eine antimikrobielle Wirkung aufweisen, da sie 1 ,4-β-glycosidische Verbindungen in Zellwand und/oder Zellmembranbestandteilen von Mikroorganismen spalten können, basiert die antimikrobielle Wirkung der hierin beschriebenen Peptide nicht auf einem solchen enzymatisch-katalytischen Wirkmechanismus, sondern unter anderem auf einer nicht enzymatisch-katalytischen Schädigung der Zellwand- und/oder Zellmembranintegrität und/oder dem Blockieren lebenswichtiger Stoffwechselwege und/oder dem Blockieren von Rezeptoren. Antimikrobielle Peptide können in ihrer Zusammensetzung und Struktur sehr unterschiedlich ausfallen und wurden aus verschiedenen Organismen isoliert, z.B. Vertebraten, Invertebraten, Insekten, Pflanzen und Mikroorganismen. Antimikrobielle Peptide treten zunächst zumeist elektrostatisch mit der mikrobiellen Zellwand bzw. Lipidmembran-Komponenten in Wechselwirkung und durchdringen dann häufig die Membran durch Ausbildung spannungsabhängiger Kanäle. So bilden sich "Poren" aus und können Löcher in die Viren- oder Bakterien- oder Pilzhülle reißen (vgl. z.B. Bahar et al., Antimicrobial Peptides, Pharmaceuticals, 2013, 6, 1543-157).

Erfindungsgemäße Peptide weisen eine Länge von 10 bis 50, vorzugsweise 10 bis 30, bevorzugt 12 bis 25, Aminosäuren auf. Erfindungsgemäß sind die Aminosäuren aus der aus Alanin, Arginin, Asparagin, Asparaginsäure, Cystein, Glutamin, Glutaminsäure, Glycin, Histidin, Isoleucin, Leucin, Lysin, Methionin, Phenylalanin, Prolin, Serin, Threonin, Tryptophan, Tyrosin und Valin bestehenden Gruppe ausgewählt, vorzugsweise ausgewählt aus Alanin, Arginin, Glutaminsäure, Glycin, Isoleucin, Leucin, Lysin, Phenylalanin, Prolin, Serin, Threonin, Tryptophan, Tyrosin und Valin.

Erfindungsgemäße Peptide weisen eine Nettoladung von +2 bis +8, vorzugsweise +3 bis +7, bevorzugt +4 bis +6, auf. Die Nettoladung eines Peptids entspricht die Summer der Ladung der sauren (-1), basischen (+1) und neutralen Aminosäuren-Seitenkette (0) bei einem pH-Wert von 7,0.

Unter Reinigungsleistung wird im Rahmen der Erfindung das Vermögen eines Mittels, eine vorhandene Anschmutzung teilweise oder vollständig zu entfernen, insbesondere die Aufhellungsleistung an einer oder mehreren Anschmutzungen auf Textilien, verstanden. Im Rahmen der Erfindung weisen sowohl ein Mittel bzw. die durch dieses Mittel gebildete Wasch-/Reinigungsflotte eine jeweilige Reinigungsleistung auf. Die Reinigungsleistung wird bevorzugt ermittelt wie weiter unten angegeben.

Unter Wasch- bzw. Reinigungsflotte wird diejenige das Wasch- bzw. Reinigungsmittel enthaltende Gebrauchslösung verstanden, die auf die Textilien oder Gewebe bzw. Oberflächen, insbesondere Geschirr oder Haushaltsoberflächen, einwirkt und damit mit den auf den Textilien oder Geweben bzw. Oberflächen vorhandenen Anschmutzungen in Kontakt kommt. Üblicherweise entsteht die Wasch-/Reinigungsflotte, wenn der Wasch-/Reinigungsvorgang beginnt und das Mittel z.B. in einer Wasch- oder Geschirrspülmaschine oder in einem anderen geeigneten Behältnis mit Wasser verdünnt wird.

Die Reinigungsleistung an Textilien oder Geweben kann in einem Waschsystem bestimmt werden, das ein Waschmittel in einer Dosierung zwischen 2,0 und 8,0 Gramm pro Liter Waschflotte enthält.

Die Konzentration des erfindungsgemäßen Peptids in dem für dieses Waschsystem bestimmten Waschmittel beträgt 1 x 10⁻⁸ bis 5 Gew.-%, vorzugsweise 0,0001 bis 1 Gew.-%, bevorzugt 0,0005 bis 0,5 Gew.-%, besonders bevorzugt 0,001 bis 0,1 Gew.-%, bezogen auf aktives Protein und Gesamtgewicht des Mittels.

Die Proteinkonzentration kann mit Hilfe bekannter Methoden, z.B. dem BCA-Verfahren (Bicinchoninsäure; 2,2'-Bichinolyl-4,4'-dicarbonsäure) oder dem Biuret-Verfahren (Gornall et al., J. Biol. Chem., 1948, 177, 751-766) bestimmt werden. Die Bestimmung der Aktivproteinkonzentration kann diesbezüglich über eine Titration der aktiven Zentren unter Verwendung eines geeigneten irreversiblen Inhibitors und Bestimmung der Restaktivität (Bender et al., J. Am. Chem. Soc., 1966, 88, 24, 5890-5913) erfolgen.

Ein flüssiges Referenzwaschmittel für ein solches Waschsystem kann z.B. wie folgt zusammengesetzt sein (alle Angaben in Gew.-%): 4,4% Alkylbenzolsulfonsäure, 5,6% weitere anionische Tenside, 2,4% C₁₂₋₁₈ Na-Salze von Fettsäuren (Seifen), 4,4% nicht-ionische Tenside, 0,2% Phosphonate, 1,4% Zitronensäure, 0,95% NaOH, 0,01% Entschäumer, 2% Glycerin, 0,08% Konservierungsstoffe, 1% Ethanol, Rest demineralisiertes Wasser. Bevorzugt beträgt die Dosierung des flüssigen Waschmittels zwischen 3,0 und 6,0 Gramm pro Liter Waschflotte, z.B. 3,0, 3,2, 3,5, 3,7, 4,0, 4,5, 4,7, 4,9 oder 5,9 Gramm pro Liter Waschflotte. Bevorzugt wird gewaschen in einem pH-Wertebereich zwischen pH 7 und pH 10,5, bevorzugt zwischen pH 8 und pH 9.

Die Reinigungsleistung wird gegenüber einer Anschmutzung auf Textil durch Messung des Reinigungsgrades der gewaschenen Textilien bestimmt. Beispielsweise kann der Waschvorgang für 60 Minuten bei einer Temperatur von 40°C erfolgen und das Wasser eine Wasserhärte zwischen 15,5°dH und 16,5°dH (deutsche Härte) aufweisen.

Der Weißegrad, d.h. die Aufhellung der Anschmutzungen, als Maß für die Reinigungsleistung wird mit optischen Messverfahren bestimmt, bevorzugt photometrisch. Ein hierfür geeignetes Gerät ist z.B. das Spektrometer Minolta CM508d. Üblicherweise werden die für die Messung eingesetzten Geräte zuvor mit einem Weißstandard, bevorzugt einem mitgelieferten Weißstandard, kalibriert.

Die Reinigungsleistung an Oberflächen, insbesondere Haushaltsoberflächen, bevorzugt in Wasch- oder Geschirrspülmaschinen, Pumpensümpfen, Vorratsbehältern, Wasserreservoir, Waschtrommel, Spülkammern, Wasch- oder Spülbecken, Toilettenbecken, Spülkasten, WC-Rand, Siphons, Wasserrohren und/oder -leitungen, Wasserzu- und/oder -abläufen, kann wie in der deutschen Patentanmeldung 102023201692.3 beschrieben bestimmt werden.

Bevorzugte Ausführungsformen erfindungsgemäßer Verwendungen und Mittel erzielen solche vorteilhaften Reinigungsleistungen auch schon bei niedrigen Temperaturen, vorzugsweise in einem Temperaturbereich von etwa 10°C bis etwa 60°C, bevorzugt etwa 15°C bis etwa 40°C, besonders bevorzugt etwa 20°C bis etwa 30°C.

In bevorzugten Ausführungsform können erfindungsgemäße Peptide, wie hierin beschrieben, in Wasch- und Reinigungsmitteln eingesetzt werden. Zu solchen erfindungsgemäßen Wasch- und Reinigungsmitteln zählen alle denkbaren Wasch- oder Reinigungsmittelarten, sowohl Konzentrate als auch unverdünnt anzuwendende Mittel, zum Einsatz im kommerziellen Maßstab, in der Waschmaschine oder bei der Handwäsche bzw. -reinigung (hierin folgend sind die Begriffe "Wasch- und/oder "Reinigungsmittel" und "Mittel" gleichbedeutend und synonym verwendet, außer es wird ausdrücklich auf ein "Waschmittel" oder ein "Reinigungsmittel" Bezug genommen). Dazu gehören z.B. Waschmittel für Textilien, Teppiche, oder Naturfasern, für die die Bezeichnung "Waschmittel" verwendet wird. Dazu gehören z.B. auch Geschirrspülmittel für Geschirrspülmaschinen (maschinelle Geschirrspülmittel) oder manuelle Geschirrspülmittel oder Reiniger für harte Oberflächen wie Metall, Glas, Porzellan, Keramik, Kacheln, Stein, lackierte Oberflächen, Kunststoffe, Holz oder Leder, für die die Bezeichnung "Reinigungsmittel" verwendet wird, also neben manuellen und maschinellen Geschirrspülmitteln z.B. auch Scheuermittel, Glasreiniger, WC-Duftspüler, usw. Zu den Wasch- und Reinigungsmittel im Rahmen der Erfindung zählen ferner Waschhilfsmittel, die bei der manuellen oder maschinellen Textilwäsche zum eigentlichen Waschmittel hinzudosiert werden, um eine weitere Wirkung zu erzielen. Ferner zählen zu Wasch- und Reinigungsmittel im Rahmen der Erfindung auch Textilvor- und -nachbehandlungsmittel, also solche Mittel, mit denen das Wäschestück vor der eigentlichen Wäsche in Kontakt gebracht wird, z.B. zum Anlösen hartnäckiger Verschmutzungen, und auch solche Mittel, die in einem der eigentlichen Textilwäsche nachgeschalteten Schritt dem Waschgut weitere wünschenswerte Eigenschaften wie angenehmen Griff, Knitterfreiheit oder geringe statische Aufladung verleihen. Zu letztgenannten Mittel werden u.a. die Weichspüler gerechnet. Mit umfasst sind dabei auch Mittel zur Verwendung in (halb-)automatisierten Wasch- oder Reinigungssystemen wie z.B. Wischrobotor oder Nassstaubsauger.

Erfindungsgemäße Mittel, die als pulverförmige oder granulare Feststoffe, in verdichteter oder nachverdichteter Teilchenform, als homogene Lösungen oder Suspensionen vorliegen können, können neben den erfindungsgemäßen Peptiden alle bekannten und in derartigen Mitteln üblichen Inhaltsstoffe enthalten, wobei bevorzugt mindestens ein weiterer Inhaltsstoff in dem Mittel vorhanden ist. Erfindungsgemäße Mittel können insbesondere Tenside, Builder, Komplexbildner, Polymere, Glaskorrosionsinhibitoren, Korrosionsinhibitoren, Bleichmittel wie Persauerstoffverbindungen, Bleichaktivatoren oder Bleichkatalysatoren, wassermischbare organische Lösungsmittel, Enzyme, Enzymstabilisatoren, Sequestrierungsmittel, Elektrolyte, pH-Regulatoren und/oder weitere Hilfsstoffe wie optische Aufheller, Vergrauungsinhibitoren, Farbübertragungsinhibitoren, Schaumregulatoren sowie Farb- und Duftstoffe enthalten. Vorteilhafte Inhaltsstoffe erfindungsgemäßer Mittel sind offenbart in der internationalen Patentanmeldung WO 2009/121725, dort beginnend auf Seite 5, vorletzter Absatz, und endend auf Seite 13 nach dem zweiten Absatz. Auf diese Offenbarung wird ausdrücklich Bezug genommen und der dortige Offenbarungsgehalt in die vorliegende
Patentanmeldung einbezogen.

Erfindungsgemäße Mittel enthalten die erfindungsgemäßen Peptide jeweils vorteilhafterweise in einer Menge von 2 µg bis 20 mg, vorzugsweise von 5 µg bis 17,5 mg, besonders bevorzugt von 20 µg bis 15 mg und ganz besonders bevorzugt von 50 µg bis 10 mg pro g des Mittels.

Erfindungsgemäße Mittel enthalten die erfindungsgemäßen Peptide zunehmend bevorzugt in einer Menge von jeweils 1 x 10⁻⁸ bis 5 Gew.-%, von 0,0001 bis 1 Gew.-%, von 0,0005 bis 0,5 Gew.-%, von 0,001 bis 0,1 Gew.-%, bezogen auf aktives Protein und Gesamtgewicht des Mittels.

In bevorzugten Ausführungsformen enthalten erfindungsgemäße Mittel die erfindungsgemäßen Peptide in einer Menge von 1 x 10⁻⁸ bis 5 Gew.-%, vorzugsweise 0,0001 bis 1 Gew.-%, bevorzugt 0,0005 bis 0,5 Gew.-%, besonders bevorzugt 0,001 bis 0,1 Gew.-%, bezogen auf aktives Protein und Gesamtgewicht des Mittels.

Weitere Ausführungsformen umfassen alle festen, pulverförmigen, flüssigen, gelförmigen oder pastösen Darreichungsformen erfindungsgemäßer Mittel, die ggf. auch aus mehreren Phasen bestehen können sowie in komprimierter oder nicht komprimierter Form vorliegen können. Das Mittel kann als rieselfähiges Pulver vorliegen, insbesondere mit einem Schüttgewicht von 300 g/l bis 1200 g/l, insbesondere 500 g/l bis 900 g/l oder 600 g/l bis 850 g/l. Zu den festen Darreichungsformen des Mittels zählen ferner Extrudate, Granulate, Tabletten oder Pouches. Alternativ kann das Mittel auch flüssig, gelförmig oder pastös sein, z.B. in Form eines nicht-wässrigen Flüssigwaschmittels oder einer nicht-wässrigen Paste oder in Form eines wässrigen Flüssigwaschmittels oder einer wasserhaltigen Paste. Flüssige Mittel sind generell bevorzugt. Weiterhin kann das Mittel als Einkomponentensystem vorliegen. Solche Mittel bestehen aus einer Phase. Alternativ kann ein Mittel auch aus mehreren Phasen bestehen. Ein solches Mittel ist demnach in mehrere Komponenten aufgeteilt.

In einer bevorzugten Ausführungsform ist das erfindungsgemäße Mittel ein Textilwaschmittel.

In einer bevorzugten Ausführungsform ist das erfindungsgemäße Mittel ein flüssiges Textilwaschmittel.

In einer bevorzugten Ausführungsform ist das erfindungsgemäße Mittel ein vorportioniertes Waschmittel, insbesondere eine Waschmittelportionseinheit umfassend eine erfindungsgemäße Waschmittelzubereitung und einen wasserlöslichen Film, welcher die Waschmittelzubereitung vollständig umschließt.

Der wasserlösliche Film, in welche die Waschmittelzubereitung verpackt ist, kann ein oder mehrere strukturell verschiedene wasserlösliche(s) Polymer(e) umfassen. Als wasserlösliche(s) Polymer(e) eignen sich insbesondere Polymere aus der Gruppe (ggf. acetalisierter) Polyvinylalkohole (PVAL) sowie deren Copolymere. Wasserlösliche Filme basieren bevorzugt auf einem Polyvinylalkohol oder einem Polyvinylalkoholcopolymer, dessen Molekulargewicht im Bereich von 10.000 bis 1.000.000 g/mol, vorzugsweise von 20.000 bis 500.000 g/mol, besonders bevorzugt von 30.000 bis 100.000 g/mol und insbesondere von 40.000 bis 80.000 g/mol liegt. Geeignete wasserlösliche Filme zum Einsatz werden u.a. von der Firma MonoSol LLC z.B. unter der Bezeichnung M8630, M8720, M8310, C8400 oder M8900 vertrieben. Geeignet sind z.B. auch Filme mit der Bezeichnung Solublon^{®} PT, Solublon^{®} GA, Solublon^{®} KC oder Solublon^{®} KL von der Aicello Chemical Europe GmbH oder die Folien VF-HP von Kuraray.

Wenn erfindungsgemäße Mittel in flüssiger Form vorliegen, enthalten sie vorzugsweise bezogen auf ihr Gesamtgewicht mehr als 40 Gew.-%, vorzugsweise 50 bis 90 Gew.-% und besonders bevorzugt 60 bis 80 Gew.-% Wasser.

Erfindungsgemäße Mittel können ein oder mehrere Tenside enthalten, wobei insbesondere anionische Tenside, nichtionische Tenside und deren Gemische in Frage kommen, aber auch kationische, zwitterionische und/oder amphotere Tenside können enthalten sein. Die Mittel enthalten vorzugsweise 5 bis 70 Gew.-% Tensid, bevorzugt 35 bis 60 Gew.-% und weiter bevorzugt 40 bis 55 Gew.-%, Tensid, bezogen auf das Gesamtgewicht des Mittels. In bevorzugten Ausführungsformen enthalten die Mittel vorzugsweise 3 bis 35 Gew.-%, bevorzugt 5 bis 30 Gew.-%, Tensid, bezogen auf das Gesamtgewicht des Mittels.

Geeignete anionische Tenside sind insbesondere Seifen und solche, die Sulfat- oder SulfonatGruppen mit bevorzugt Alkaliionen als Kationen enthalten. Verwendbare Seifen sind bevorzugt die Alkalisalze der gesättigten oder ungesättigten C₁₂₋₁₈-Fettsäuren. Derartige Fettsäuren können auch in nicht vollständig neutralisierter Form eingesetzt werden. Zu den brauchbaren Tensiden des Sulfat-Typs gehören die Salze der Schwefelsäurehalbester von C₁₂₋₁₈-Fettalkoholen und die Sulfatierungsprodukte der genannten nichtionischen Tenside mit niedrigem Ethoxylierungsgrad. Zu den verwendbaren Tensiden vom Sulfonat-Typ gehören z.B. C₉₋₁₄-Alkylbenzolsulfonate, Alkansulfonate, die aus C₁₂₋₁₈-Alkanen z.B. durch Sulfochlorierung oder Sulfoxidation mit anschließender Hydrolyse bzw. Neutralisation gewonnen werden, C₁₂₋₁₈-Olefinsulfonate, die bei der Umsetzung entsprechender Monoolefine mit Schwefeltrioxid entstehen, Gemische aus Alken- und Hydroxyalkansulfonaten, Disulfonaten, wie man sie z.B. aus C₁₂₋₁₈-Monoolefinen mit end- oder innenständigen Doppelbindung durch Sulfonieren mit gasförmigem Schwefeltrioxid und anschließende alkalische oder saure Hydrolyse der Sulfonierungsprodukte erhält, sowie α-Sulfofettsäureester (Estersulfonate), die bei der Sulfonierung von Fettsäuremethyl- oder -ethylestern entstehen, z.B. α-sulfonierte Methylester der hydrierten Kokos-, Palmkern- oder Talgfettsäuren.

Vorzugsweise weist das Mittel, bezogen auf das Gesamtgewicht des Mittels, 2 bis 55 Gew.-%, vorzugsweise 3 bis 35 Gew.-%, Aniontensid auf. Ganz besonders bevorzugt weist das Mittel 3 bis 25 Gew.-% Alkylbenzolsulfonat auf. Darüber hinaus kann das Mittel vorzugsweise noch weitere anionische Tenside, insbesondere Alkylethersulfate, sowie nichtionische Tenside, insbesondere Fettalkoholalkoxylate, enthalten. Diese können dann den Rest der Tenside ausmachen.

Geeignete Alkylbenzolsulfonate sind vorzugsweise ausgewählt aus linearen oder verzweigten Alkylbenzolsulfonaten der Formel 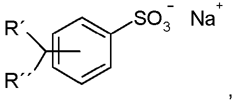 in der R' und R" unabhängig H oder Alkyl sind und zusammen 6 bis 19, vorzugsweise 7 bis 15 und insbesondere 9 bis 13 C-Atome enthalten. Ein ganz besonders bevorzugter Vertreter ist Natriumdodecylbenzylsulfonat.

Als Alk(en)ylsulfate werden die Alkali- und insbesondere die Natriumsalze der Schwefelsäurehalbester der C₁₂₋₁₈-Fettalkohole, z.B. aus Kokosfettalkohol, Talgfettalkohol, Lauryl-, Myristyl-, Cetyl- oder Stearylalkohol oder der C₁₀₋₂₀-Oxoalkohole und diejenigen Halbester sekundärer Alkohole dieser Kettenlängen bevorzugt. Weiterhin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten besitzen wie die adäquaten Verbindungen auf der Basis von fettchemischen Rohstoffen. Aus waschtechnischem Interesse sind die C₁₂₋₁₆-Alkylsulfate und C₁₂₋₁₅-Alkylsulfate sowie C₁₄₋₁₅-Alkylsulfate bevorzugt.

Auch die Schwefelsäuremonoester der mit 1 bis 6 Mol Ethylenoxid ethoxylierten geradkettigen oder verzweigten C₇₋₂₁-Alkohole, wie 2-Methyl-verzweigte C₉₋₁₁-Alkohole mit im Durchschnitt 3,5 Mol Ethylenoxid (EO) oder C₁₂₋₁₈-Fettalkohole mit 1 bis 4 EO, sind geeignet.

Geeignete Alkylethersulfate sind z.B. Verbindungen der Formel
R¹-O-(AO)ₙ-SO₃⁻ X⁺.

In dieser Formel steht R¹ für einen linearen oder verzweigten, substituierten oder unsubstituierten Alkylrest, vorzugsweise für einen linearen, unsubstituierten Alkylrest, besonders bevorzugt für einen Fettalkoholrest. Bevorzugte Reste R¹ sind ausgewählt aus Decyl-, Undecyl-, Dodecyl-, Tridecyl-, Tetradecyl-, Pentadecyl-, Hexadecyl-, Heptadecyl-, Octadecyl-, Nonadecyl-, Eicosylresten und deren Mischungen, wobei die Vertreter mit gerader Anzahl an C-Atomen bevorzugt sind. Besonders bevorzugte Reste R¹ sind abgeleitet von C₁₂₋₁₈-Fettalkoholen, z.B. von Kokosfettalkohol, Talgfettalkohol, Lauryl-, Myristyl-, Cetyl- oder Stearylalkohol oder von C₁₀₋₂₀-Oxoalkoholen. AO steht für eine Ethylenoxid-(EO) oder Propylenoxid-(PO)-Gruppierung, vorzugsweise für eine Ethylenoxidgruppierung. Der Index n steht für eine ganze Zahl von 1 bis 50, vorzugsweise von 1 bis 20 und insbesondere von 2 bis 10. Ganz besonders bevorzugt steht n für die Zahlen 2, 3, 4, 5, 6, 7 oder 8. X⁺ steht für ein einwertiges Kation oder den n-ten Teil eines n-wertigen Kations, bevorzugt sind dabei die Alkalimetallionen und darunter Na⁺ oder K⁺, wobei Na⁺ äußerst bevorzugt ist. Weitere Kationen X⁺ können ausgewählt sein aus NH₄⁺, ½ Zn²⁺, ½ Mg²⁺, ½ Ca²⁺, ½ Mn²⁺ und deren Mischungen.

In verschiedenen Ausführungsformen kann das Alkylethersulfat ausgewählt sein aus Fettalkoholethersulfaten der Formel

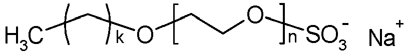

mit k = 11 bis 19, n = 2, 3, 4, 5, 6, 7 oder 8. Ganz besonders bevorzugte Vertreter sind Na-C₁₂₋₁₄-Fettalkoholethersulfate mit 2 EO (k = 11-13, n = 2). Der angegebenen Ethoxylierungsgrad stellt einen statistischen Mittelwert dar, der für ein spezielles Produkt eine ganze oder eine gebrochene Zahl sein kann. Die angegebenen Alkoxylierungsgrade stellen statistische Mittelwerte dar, die für ein spezielles Produkt eine ganze oder eine gebrochene Zahl sein können. Bevorzugte Alkoxylate/Ethoxylate weisen eine eingeengte Homologenverteilung auf (narrow range ethoxylates, NRE).

Es hat sich für die Kaltwaschleistung als vorteilhaft erwiesen, wenn die Mittel zusätzlich Seife(n) enthalten. Bevorzugte Mittel sind daher dadurch gekennzeichnet, dass sie Seife(n) enthalten. Geeignet sind gesättigte Fettsäureseifen, wie die Salze der Laurinsäure, Myristinsäure, Palmitinsäure, Stearinsäure, hydrierte Erucasäure und Behensäure sowie insbesondere aus natürlichen Fettsäuren, z.B. Kokos-, Palmkern- oder Talgfettsäuren, abgeleitete Seifengemische.

Geeignete nichtionische Tenside sind insbesondere Alkylglykoside und Ethoxylierungs- und/oder Propoxylierungsprodukte von Alkylglykosiden oder linearen oder verzweigten Alkoholen mit jeweils 8 bis etwa 18 C-Atomen im Alkylteil und 3 bis 20, vorzugsweise 4 bis 10 Alkylethergruppen. Weiterhin sind entsprechende Ethoxylierungs- und/oder Propoxylierungsprodukte von N-Alkylaminen, vicinalen Diolen, Fettsäureestern und Fettsäureamiden, die hinsichtlich des Alkylteils den genannten langkettigen Alkoholderivaten entsprechen, sowie von Alkylphenolen mit 5 bis 12 C-Atomen im Alkylrest brauchbar.

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, z.B. aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören z.B. C₁₂₋₁₄-Alkohole mit 3 EO oder 4 EO, C₉₋₁₁-Alkohol mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 5 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO.

Eine weitere Klasse bevorzugt eingesetzter nichtionischer Tenside, die entweder als alleiniges nichtionisches Tensid oder in Kombination mit anderen nichtionischen Tensiden eingesetzt werden, sind alkoxylierte, vorzugsweise ethoxylierte oder ethoxylierte und propoxylierte Fettsäurealkylester, vorzugsweise mit 1 bis 4 Kohlenstoffatomen in der Alkylkette, insbesondere Fettsäuremethylester.

Eine weitere Klasse von nichtionischen Tensiden, die vorteilhaft eingesetzt werden kann, sind die Alkylpolyglycoside (APG). Einsetzbare Alkylpolyglycoside genügen der allgemeinen Formel RO(G)_{z},

in der R für einen linearen oder verzweigten, insbesondere in 2-Stellung methylverzweigten, gesättigten oder ungesättigten, aliphatischen Rest mit 8 bis 22, vorzugsweise 12 bis 18 C-Atomen bedeutet und G das Symbol ist, das für eine Glykoseeinheit mit 5 oder 6 C-Atomen, vorzugsweise für Glucose, steht. Der Glycosidierungsgrad z liegt dabei zwischen 1 und 4, vorzugsweise zwischen 1 und 2 und insbesondere zwischen 1,1 und 1,4. Bevorzugt eingesetzt werden lineare Alkylpolyglycoside, also Alkylpolyglycoside, in denen der Polyglycosylrest ein Glucoserest und der Alkylrest ein n-Alkylrest ist.

Auch nichtionische Tenside vom Typ der Aminoxide, z.B. N-Kokosalkyl-N,N-dimethylaminoxid und N-Talgalkyl-N,N-dihydroxyethylaminoxid, und der Fettsäurealkanolamide können geeignet sein. Die Menge dieser nichtionischen Tenside beträgt vorzugsweise nicht mehr als die der ethoxylierten Fettalkohole, insbesondere nicht mehr als die Hälfte davon.

Geeignete Amphotenside sind z.B. Betaine der Formel
(Rⁱⁱⁱ)(R^{iv})(R^{v})N+CH₂COO⁻,

in der Rⁱⁱⁱ einen ggf. durch Heteroatome oder Heteroatomgruppen unterbrochenen Alkylrest mit 8 bis 25, vorzugsweise 10 bis 21 Kohlenstoffatomen und R^{iv} sowie R^{v} gleichartige oder verschiedene Alkylreste mit 1 bis 3 Kohlenstoffatomen bedeuten, insbesondere C₁₀₋₁₈-Alkyldimethylcarboxymethylbetain und C₁₁₋₁₇-Alkylamidopropyldimethylcarboxymethylbetain.

Geeignete kationische Tenside sind u.a. die quartären Ammoniumverbindungen der Formel
(R^{vi})(R^{vii})(R^{viii})(R^{ix})N⁺X⁻,

in der R^{vi} bis R^{ix} für vier gleich- oder verschiedenartige, insbesondere zwei lang- und zwei kurzkettige, Alkylreste und X- für ein Anion, insbesondere ein Halogenidion, stehen, z.B. Didecyldimethylammoniumchlorid, Alkylbenzyldidecylammoniumchlorid und deren Mischungen. Weitere geeignete kationische Tenside sind die quaternären oberflächenaktiven Verbindungen, insbesondere mit einer Sulfonium-, Phosphonium-, Jodonium- oder Arsoniumgruppe, die auch als antimikrobielle Wirkstoffe bekannt sind. Durch den Einsatz von quaternären oberflächenaktiven Verbindungen mit antimikrobieller Wirkung kann das Mittel mit einer antimikrobiellen Wirkung ausgestaltet werden bzw. dessen ggf. aufgrund anderer Inhaltsstoffe bereits vorhandene antimikrobielle Wirkung verbessert werden.

In bevorzugten Ausführungsformen umfasst das erfindungsgemäße Wasch- und Reinigungsmittel, jeweils bezogen auf das Gesamtgewicht des Mittels,
(i) 2 bis 20 Gew.-%, vorzugsweise 3 bis 17 Gew.-% anionische Tenside,
(ii) 1 bis 10 Gew.-%, vorzugsweise 3 bis 8 Gew.-%, nichtionische Tenside,
(iii) 0 bis 1 Gew.-%, vorzugsweise 0 bis 0,5 Gew.-%, Seife, und
(iv) 0 bis 5 Gew.-%, vorzugsweise 0 bis 3 Gew.-% Fettsäuren.

Ein weiterer bevorzugter Bestandteil erfindungsgemäßer Mittel sind Komplexbildner. Besonders bevorzugte Komplexbildner sind die Phosphonate, sofern ihr Einsatz regulatorisch zulässig ist. Die komplexbildenden Phosphonate umfassen neben der 1-Hydroxyethan-1,1-diphosphonsäure eine Reihe unterschiedlicher Verbindungen, wie z.B. Diethylentriaminpenta(methylenphosphonsäure) (DTPMP). In dieser Anmeldung bevorzugt sind insbesondere Hydroxyalkan- bzw. Aminoalkanphosphonate. Unter den Hydroxyalkanphosphonaten ist das 1-Hydroxyethan-1,1-diphosphonat (HEDP) von besonderer Bedeutung als Cobuilder. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, z.B. als Hexanatriumsalz der EDTMP bzw. als Hepta- und Octa-Natriumsalz der DTPMP, eingesetzt. Als Builder wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Mittel auch Bleiche enthalten, bevorzugt sein, Aminoalkanphosphonate, insbesondere DTPMP, einzusetzen, oder Mischungen aus den genannten Phosphonaten zu verwenden. Ein im Rahmen dieser Anmeldung bevorzugtes Mittel enthält ein oder mehrere Phosphonat(e) aus der Gruppe Aminotrimethylenphosphonsäure (ATMP) und/oder deren Salze; Ethylendiamintetra(methylenphosphonsäure) (EDTMP) und/oder deren Salze; Diethylentriaminpenta(methylenphosphonsäure) (DTPMP) und/oder deren Salze; 1-Hydroxyethan-1,1-diphosphonsäure (HEDP) und/oder deren Salze; 2-Phosphonobutan-1,2,4-tricarbonsäure (PBTC) und/oder deren Salze; Hexamethylendiamintetra(methylenphosphonsäure) (HDTMP) und/oder deren Salze; Nitrilotri(methylenphosphonsäure) (NTMP) und/oder deren Salze. Besonders bevorzugt sind Mittel, welche als Phosphonate 1-Hydroxyethan-1,1-diphosphonsäure (HEDP) oder Diethylentriaminpenta(methylenphosphonsäure) (DTPMP) enthalten. Selbstverständlich können erfindungsgemäße Mittel zwei oder mehr unterschiedliche Phosphonate enthalten.

Bevorzugte erfindungsgemäße Mittel sind dadurch gekennzeichnet, dass das Mittel mindestens einen Komplexbildner aus der Gruppe der Phosphonate, vorzugsweise 1-Hydroxyethan-1,1-diphosphonat, enthält, wobei der Gewichtsanteil des Phosphonat am Gesamtgewicht des Mittels vorzugsweise 0,1 und 8,0 Gew.-%, bevorzugt 0,2 und 5,0 Gew.-%, weiter bevorzugt 0,3 und 3,0 Gew.-% und besonders bevorzugt 0,5-2,0 Gew.-% beträgt.

In weiter bevorzugten Ausführungsformen sind erfindungsgemäße Mittel im Wesentlichen frei von phosphonathaltigen Verbindungen. "Im Wesentlichen frei von phosphonathaltigen Verbindungen" bedeutet in diesem Zusammenhang, dass die entsprechende Mittel oder Zusammensetzungen, bezogen auf das Gesamtgewicht des Mittels, weniger als 2 Gew.-%, vorzugsweise weniger als 1 Gew.-%, weiter bevorzugt weniger als 0,5 Gew.-% und besonders bevorzugt weniger als 0,1 Gew.-%, phosphonathaltige Verbindungen enthalten. In besonders bevorzugten Ausführungsformen sind diese Mittel/Zusammensetzungen frei von phosphonathaltigen Verbindungen.

Erfindungsgemäße Mittel enthalten weiterhin vorzugsweise Gerüststoff (Builder), vorzugsweise mindestens einen wasserlöslichen und/oder wasserunlöslichen, organischen und/oder anorganischen Builder. Zu den Gerüststoffe zählen dabei insbesondere die Silikate, Carbonate und organische Cobuilder.

Als organische Cobuilder sind insbesondere Polycarboxylate/Polycarbonsäuren, polymere Polycarboxylate, Asparaginsäure, Polyacetale, Dextrine, weitere organische Cobuilder sowie Phosphonate zu nennen. Diese Stoffklassen werden nachfolgend beschrieben. Organische Cobuildersubstanzen können gewünschtenfalls in Mengen bis zu 40 Gew.-%, insbesondere bis zu 25 Gew.-% und vorzugsweise von 1 bis 8 Gew.-% enthalten sein, bezogen auf das Gesamtgewicht des Mittels. Brauchbare organische Gerüstsubstanzen sind z.B. die in Form der freien Säure und/oder ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren und Carboxymethylinuline, monomere und polymere Aminopolycarbonsäuren, insbesondere Glycindiessigsäure, Methylglycindiessigsäure, Glutamindiessigsäure, Nitrilotriessigsäure (NTA), Iminodisuccinat wie Ethylendiamin-N,N'-dibernsteinsäure und Hydroxyiminodisuccinate, Ethylendiamintetraessigsäure sowie Polyasparaginsäure, Polyphosphonsäuren, insbesondere Aminotris(methylenphosphonsäure), Ethylendiamintetrakis(methylenphosphonsäure), Lysintetra(methylenphosphosäure) und 1-Hydroxyethan-1,1-diphosphonsäure, polymere Hydroxyverbindungen wie Dextrin sowie polymere (Poly)carbonsäuren, insbesondere durch Oxidation von Polysacchariden bzw. Dextrinen zugängliche Polycarboxylate, und/oder polymere Acrylsäuren, Methacrylsäuren, Maleinsäuren und Mischpolymere aus diesen, die auch geringe Anteile polymerisierbarer Substanzen ohne Carbonsäurefunktionalität einpolymerisiert enthalten können. Derartige organische Buildersubstanzen können gewünschtenfalls in Mengen bis zu 50 Gew.-%, insbesondere bis zu 25 Gew.-%, vorzugsweise von 10 bis 20 Gew.-% und besonders bevorzugt von 1 bis 5 Gew.-% enthalten sein, bezogen auf das Gesamtgewicht des Mittels. Die freien Säuren besitzen neben ihrer Builderwirkung typischerweise auch die Eigenschaft einer Säuerungskomponente und dienen somit auch zur Einstellung eines niedrigeren und milderen pH-Wertes von Mitteln. Insbesondere sind hierbei Citronensäure, Bernsteinsäure, Glutarsäure, Adipinsäure, Gluconsäure und beliebige Mischungen aus diesen zu nennen. Mit besonderem Vorzug wird als Gerüstsubstanz die Citronensäure oder Salze der Citronensäure eingesetzt. Weitere besonders bevorzugte Gerüstsubstanzen sind ausgewählt unter Methylglycindiessidsäure (MGDA), Glutaminsäurediacetat (GLDA), Asparaginsäurediacetat (ASDA), Hydroxyethyliminodiacetat (HEIDA), Iminodisuccinat (IDS) und Ethylendiamindisuccinat (EDDS), Carboxymethylinulin und Polyaspartat.

In bevorzugten Ausführungsformen wird als wasserlöslicher, organischer Builder Citronensäure und/oder Citrat eingesetzt. Besonders bevorzugt ist der Einsatz, bezogen auf das Gesamtgewicht des Mittels, von 0,5 bis 25 Gew.-%, vorzugsweise 0,75 bis 12,5 Gew.-%, weiter bevorzugt 1 bis 4 Gew.-% Citronensäure und/oder 0,5 bis 25 Gew.-%, vorzugsweise 0,75 bis 12,5 Gew.-%, weiter bevorzugt 1 bis 4 Gew.-% Citrat, vorzugsweise Alkalicitrat, noch bevorzugter Natriumcitrat. Citronensäure/Citrat können jeweils in Form ihrer Hydrate eingesetzt werden, so kann z.B. Citronensäure in Form des Monohydrats, Citrat in Form des Trinatriumcitratdihydrats eingesetzt werden.

In bevorzugteren Ausführungsformen sind die Builder-Substanzen ausgewählt aus MGDA und GLDA. Wie hier verwendet, umfasst der Begriff "MGDA" unter anderem Methylglycindiessigsäure, α-Alanindiessigsäure, N-(1-Carboxyethyl)-iminodiessigsäure und N,N-Bis(carboxymethyl)-DL-Alanin, wobei die freien Säureformen und die entsprechenden Salze, vorzugsweise Alkalisalze, insbesondere Trinatriumsalze, eingeschlossen sind. Wie hier verwendet, umfasst der Begriff "GLDA" unter anderem Glutaminsäurediessigsäure, L-Glutaminsäure-N,N-diessigsäure und N,N-Bis(carboxylatomethyl)-L-Glutamat, wobei freie Säureformen und entsprechende Salze, vorzugsweise Alkalisalze, insbesondere Tetranatriumsalze, eingeschlossen sind. Obwohl, bezogen auf das Gesamtgewicht des Mittels, höhere MGDA- oder GLDA-Konzentrationen möglich sind, insbesondere bis zu 25 Gew.-%, wird besonders bevorzugt 0,2 bis 5 Gew.-%, vorzugsweise 0,25 bis 3 Gew.-%, noch bevorzugter 0,5 bis 2 Gew.-% MGDA, vorzugsweise MGDA-Trinatriumsalz (MGDA-Nas) verwendet. Noch bevorzugter ist die Verwendung, bezogen auf das Gesamtgewicht des Mittels, von 0,2 bis 5 Gew.-%, vorzugsweise 0,25 bis 3 Gew.-%, noch bevorzugter von 0,5 bis 2 Gew.-% GLDA, vorzugsweise GLDA-Tetranatriumsalz (GLDA-Na₄).

Als Gerüststoffe sind weiter polymere Polycarboxylate geeignet, dies sind z.B. die Alkalimetallsalze der Polyacrylsäure oder der Polymethacrylsäure, z.B. solche mit einer relativen Molekülmasse von 500 bis 70.000 g/mol. Bei den für polymere Polycarboxylate angegebenen Molmassen handelt es sich im Sinne dieser Anmeldung um gewichtsmittlere Molmassen Mw der jeweiligen Säureform, die grundsätzlich mittels Gelpermeationschromatographie (GPC) bestimmt wurden, wobei ein UV-Detektor eingesetzt wurde. Die Messung erfolgte dabei gegen einen externen Polyacrylsäure-Standard, der aufgrund seiner strukturellen Verwandtschaft mit den untersuchten Polymeren realistische Molgewichtswerte liefert. Diese Angaben weichen deutlich von den Molgewichtsangaben ab, bei denen Polystyrolsulfonsäuren als Standard eingesetzt werden. Die gegen Polystyrolsulfonsäuren gemessenen Molmassen sind in der Regel deutlich höher als die in dieser Anmeldung angegebenen Molmassen. Geeignete Polymere sind insbesondere Polyacrylate, die bevorzugt eine Molekülmasse von 2.000 bis 20.000 g/mol aufweisen. Aufgrund ihrer überlegenen Löslichkeit können aus dieser Gruppe wiederum die kurzkettigen Polyacrylate, die Molmassen von 2.000 bis 10.000 g/mol, und besonders bevorzugt von 3.000 bis 5.000 g/mol, aufweisen, bevorzugt sein. Geeignet sind weiterhin copolymere Polycarboxylate, insbesondere solche der Acrylsäure mit Methacrylsäure und der Acrylsäure oder Methacrylsäure mit Maleinsäure. Als besonders geeignet haben sich Copolymere der Acrylsäure mit Maleinsäure erwiesen, die 50 bis 90 Gew.-% Acrylsäure und 50 bis 10 Gew.-% Maleinsäure enthalten. Ihre relative Molekülmasse, bezogen auf freie Säuren, beträgt im Allgemeinen 2.000 bis 70.000 g/mol, vorzugsweise 20.000 bis 50.000 g/mol und insbesondere 30.000 bis 40.000 g/mol.

Ein erfindungsgemäßes festes Mittel enthält vorzugsweise mindestens einen wasserlöslichen und/oder wasserunlöslichen, organischen und/oder anorganischen Builder. Zu den wasserlöslichen organischen Buildersubstanzen gehören die oben genannten organischen Gerüstsubstanzen.

Zusätzlich zu den vorstehend genannten wasserlöslichen organischen Buildern, können die Mittel der Erfindung weiterhin auch anorganische wasserlösliche Builder enthalten. Als wasserlösliche anorganische Buildermaterialien kommen insbesondere Alkalisilikate, Alkalicarbonate, Alkalihydrogenbarbonate, Alkaliphosphate und/oder Sesquicarbonate, die in Form ihrer alkalischen, neutralen oder sauren Natrium- oder Kaliumsalze vorliegen können, in Betracht. Ggf. können auch geringe Mengen an Calciumcarbonate in festen Textilwaschmitteln enthalten sein. Geeignet sind z.B. wasserlöslichen kristalline und/oder amorphe Alkalisilikate. Die in den erfindungsgemäßen Mitteln als Gerüststoffe brauchbaren Alkalisilikate weisen vorzugsweise ein molares Verhältnis von Alkalioxid zu SiO₂ unter 0,95, insbesondere von 1:1,1 bis 1:12 auf und können amorph oder kristallin vorliegen. Bevorzugte Alkalisilikate sind die Natriumsilikate, insbesondere die amorphen Natriumsilikate, mit einem molaren Verhältnis Na₂O:SiO₂ von 1:2 bis 1:2,8. Als kristalline Silikate, die allein oder im Gemisch mit amorphen Silikaten vorliegen können, werden vorzugsweise kristalline Schichtsilikate der allgemeinen Formel Na₂SiₓO₂ₓ₊₁ · y H₂O eingesetzt, in der x, das sogenannte Modul, eine Zahl von 1,9 bis 22, insbesondere 1,9 bis 4 und y eine Zahl von 0 bis 33 ist und bevorzugte Werte für x 2, 3 oder 4 sind. Bevorzugte kristalline Schichtsilikate sind solche, bei denen x in der genannten allgemeinen Formel die Werte 2 oder 3 annimmt. Insbesondere sind sowohl β- als auch δ-Natriumdisilikate (Na₂Si₂O · y H₂O) bevorzugt. Auch aus amorphen Alkalisilikaten hergestellte, praktisch wasserfreie kristalline Alkalisilikate der oben genannten allgemeinen Formel, in der x eine Zahl von 1,9 bis 2,1 bedeutet, können in erfindungsgemäßen Mitteln eingesetzt werden. In einer weiteren Ausführungsform erfindungsgemäßer Mittel wird ein kristallines Natriumschichtsilikat mit einem Modul von 2 bis 3 eingesetzt, wie es aus Sand und Soda hergestellt werden kann. Kristalline Natriumsilikate mit einem Modul im Bereich von 1,9 bis 3,5 werden in einer weiteren Ausführungsform erfindungsgemäßer Mittel eingesetzt. In Mitteln, die sowohl amorphe als auch kristalline Alkalisilikate enthalten, beträgt das Gewichtsverhältnis von amorphem Alkalisilikat zu kristallinem Alkalisilikat vorzugsweise 1:2 bis 2:1 und insbesondere 1:1 bis 2:1. Kristalline schichtförmige Silikate der oben angegebenen Formel werden von der Fa. Clariant GmbH unter dem Handelsnamen Na-SKS vertrieben, z.B. Na-SKS-1 (Na₂Si₂₂O₄₅ · x H₂O, Kenyait), Na-SKS-2 (Na₂Si₁₄O₂₉ · x H₂O, Magadiit), Na-SKS-3 (Na₂SisOi₇ · x H₂O) oder Na-SKS-4 (Na₂Si₄O₉ · x H₂O, Makatit). Von diesen eignen sich vor allem Na-SKS-5 (α-Na₂Si₂O₅), Na-SKS-7 (ß-Na₂Si₂O₅, Natrosilit), Na-SKS-9 (NaHSi₂O₅ · 3 H₂O), Na-SKS-10 (NaHSi₂O₅ · 3 H₂O, Kanemit), Na-SKS-11 (t-Na₂Si₂O₅) und Na-SKS-13 (NaHSi₂O₅), insbesondere aber Na-SKS-6 (δ-Na₂Si₂O₅). In einer Ausgestaltung erfindungsgemäßer Mittel setzt man ein granulares Compound aus kristallinem Schichtsilikat und Citrat, aus kristallinem Schichtsilikat und oben genannter (co-)polymerer Polycarbonsäure, oder aus Alkalisilikat und Alkalicarbonat ein, wie es z.B. unter dem Namen Nabion^{®} 15 im Handel erhältlich ist. Derartige wasserlösliche anorganischen Buildermaterialien sind in den erfindungsgemäßen Mitteln vorzugsweise in Mengen von 1 bis 20 Gew.-%, insbesondere 5 bis 15 Gew.-%, enthalten, bezogen auf das Gesamtgewicht des Mittels. Des Weiteren sind als wasserlösliche anorganische Buildersubstanzen noch die Carbonate (und Hyodrogencarbonate), insbesondere Natriumcarbonat, und die Phosphonsäuren/Phosphonate von Bedeutung.

Erfindungsgemäße Mittel sind vorzugsweise frei von Phosphat-Builder, d.h. enthalten, bezogen auf das Gesamtgewicht des Mittels, weniger als 1 Gew.-%, bevorzugt keinen bewusst zugegebenen Phosphat-Builder.

Erfindungsgemäße Mittel können auch wasserunlösliche Buildersubstanzen enthalten. Als wasserunlösliche anorganische Buildermaterialien werden insbesondere kristalline oder amorphe wasserdispergierbare Alkalialumosilikate, in Mengen von bis zu 50 Gew.-%, vorzugsweise nicht über 40 Gew.-%, insbesondere von 3 bis 20 Gew.-% und besonders bevorzugt von 1 bis 15 Gew.-%, bezogen auf das Gesamtgewicht des Mittels, eingesetzt. Unter diesen sind die kristallinen Natriumalumosilikate in Waschmittelqualität, insbesondere Zeolith A, Zeolith P, Zeolith MAP und ggf. Zeolith X, allein oder in Mischungen, z.B. in Form eines Co-Kristallisats aus den Zeolithen A und X (Vegobond^{®} AX, ein Handelsprodukt der Condea Augusta S.p.A.), bevorzugt. Mengen nahe der genannten Obergrenze werden vorzugsweise in festen, teilchenförmigen Mitteln eingesetzt. Geeignete Alumosilikate weisen insbesondere keine Teilchen mit einer Korngröße über 30 µm auf und bestehen vorzugsweise zu wenigstens 80 Gew.-% aus Teilchen mit einer Größe unter 10 µm. Ihr Calciumbindevermögen, das gemäß DE 2412837 A1 bestimmt werden kann, liegt in der Regel im Bereich von 100 bis 200 mg CaO pro Gramm.

In bevorzugten Ausführungsformen umfassen erfindungsgemäße Mittel ein Buildersystem, umfassend mindestens einen Builder, vorzugsweise in einer Menge von 0,5 bis 50 Gew.-%, bevorzugt 0,5 bis 20 Gew.-%, besonders bevorzugt 0,5 bis 10 Gew.-%, bezogen auf das Gesamtgewicht des Mittels, wobei der Builder aus der aus Polycarbonsäuren wie Zitronensäure, Adipinsäure, Bernsteinsäure, Glutarsäure, Äpfelsäure, Weinsäure, Maleinsäure Fumarsäure, Zuckersäuren und Carboxymethylinuline oder deren Salzen, monomeren und polymeren Aminopolycarbonsäuren wie Glycindiessigsäure, Methylglycindiessigsäure (MGDA), Glutaminsäurediessigsäure (GLDA), Nitriltriessigsäure, Iminodisuccinat wie Ethylendiamin-N,N'-Disuccinsäure und Hydroxyiminodisuccinate, Ethylendiamintetraessigsäure und Polyasparaginsäure oder deren Salze, Polyphosphonsäuren wie Aminotris(methylenphosphonsäure), Ethylendiamintetrakis(methylenphosphonsäure), Lysintetra(methylenphosphonsäure), Diethylentriaminpenta(methylenphosphonsäure) (DTPMP) und 1-Hydroxyethan-1,1-diphosphonsäure (HEDP) oder deren Salze, polymeren Hydroxyverbindungen wie Dextrin, und Mischungen davon bestehenden Gruppe ausgewählt ist.

In bevorzugten Ausführungsformen umfassen erfindungsgemäßes Mittel, jeweils bezogen auf das Gesamtgewicht des Mittels,
(i) 0 bis 10 Gew.-%, vorzugsweise 1 bis 4 Gew.-% Zitronensäure und/oder Citrat, vorzugsweise Alkalicitrat,
(ii) 0 bis 40 Gew.-%, vorzugsweise 0 bis 15 Gew.-%, stärker bevorzugt 1 bis 3 Gew.-%, Alkalicarbonat, vorzugsweise Natriumcarbonat,
(iii) 0 bis 20 Gew.-%, vorzugsweise 3 bis 10 Gew.-% Alkalisilikat,
(iv) 0 bis 10 Gew.-%, vorzugsweise 0,5 bis 2 Gew.-%, Phosphonsäure und/oder Alkaliphosphonat, besonders bevorzugt HEDP und/oder DTPMP, und/oder
(v) 0 bis 10 Gew.-%, vorzugsweise 0,5 bis 3 Gew.-%, Aminopolycarbonsäuren, vorzugsweise MGDA und/oder GLDA.

In Ergänzung zu den zuvor beschriebenen Gerüststoffen können in dem Mittel reinigungsaktive Polymere enthalten sein. Der Gewichtsanteil der reinigungsaktiven Polymere am Gesamtgewicht erfindungsgemäßer Mittel beträgt vorzugsweise 0,1 bis 20 Gew.-%, vorzugsweise 1,0 bis 15 Gew.-% und weiter bevorzugt 2,0 bis 12 Gew.-%.

Als für den Einsatz in erfindungsgemäßen Mitteln geeignete Persauerstoffverbindungen kommen insbesondere organische Persäuren bzw. persaure Salze organischer Säuren, wie Phthalimidopercapronsäure, Perbenzoesäure oder Salze der Diperdodecandisäure, Wasserstoffperoxid und unter den Waschbedingungen Wasserstoffperoxid abgebende anorganische Salze, zu denen Perborat, Percarbonat, Persilikat und/oder Persulfat wie Caroat gehören, sowie Wasserstoffperoxid-Einschlussverbindungen, wie HzOz-Harnstoffaddukte, in Betracht. Wasserstoffperoxid kann dabei auch mit Hilfe eines enzymatischen Systems, d.h. einer Oxidase und ihres Substrates, erzeugt werden. Sofern feste Persauerstoffverbindungen eingesetzt werden sollen, können diese in Form von Pulvern oder Granulaten verwendet werden, die auch in im Prinzip bekannter Weise umhüllt sein können. Die Persauerstoffverbindungen können als solche oder in Form diese enthaltender Mittel, die prinzipiell alle üblichen Wasch-, Reinigungs- oder Desinfektionsmittelbestandteile enthalten können, zu der Waschlauge zugegeben werden. Besonders bevorzugt wird Alkalipercarbonat oder Alkaliperborat-Monohydrat eingesetzt. Falls ein erfindungsgemäßes Mittel Persauerstoffverbindungen enthält, sind diese in Mengen von vorzugsweise bis zu 50 Gew.-%, insbesondere von 5 bis 30 Gew.-%, weiter bevorzugt von 0,1 bis 20 Gew.-% vorhanden, bezogen auf das Gesamtgewicht des Mittels.

In bevorzugten Ausführungsformen sind erfindungsgemäße Mittel im Wesentlichen frei von Persauerstoffverbindungen. "Im Wesentlichen frei von persauerstoffhaltigen Verbindungen" bedeutet in diesem Zusammenhang, dass die entsprechende Mittel oder Zusammensetzungen weniger als 2 Gew.-%, vorzugsweise weniger als 1 Gew.-%, weiter bevorzugt weniger als 0,5 Gew.-% und besonders bevorzugt weniger als 0,1 Gew.-%, persauerstoffhaltigen Verbindungen, bezogen auf das Gesamtgewicht des Mittels/der Zusammensetzung, enthalten. In besonders bevorzugten Ausführungsformen sind diese Mittel/Zusammensetzungen frei von persauerstoffhaltigen Verbindungen.

Als Bleichaktivatoren können in den Mitteln Verbindungen, die unter Perhydrolysebedingungen aliphatische Peroxocarbonsäuren mit vorzugsweise 1 bis 10 C-Atomen, insbesondere 2 bis 4 C-Atomen, und/oder ggf. substituierte Perbenzoesäure ergeben, eingesetzt werden. Geeignet sind Substanzen, die O- und/oder N-Acylgruppen der genannten C-Atomzahl und/oder ggf. substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate odercarboxylate bzw. die Sulfon- oder Carbonsäuren von diesen, insbesondere Nonanoyl- oder Isononanoyloxybenzolsulfonat oder Laroyloxybenzolsulfonat (NOBS bzw. iso-NOBS bzw. LOBS), 4-(2-Decanoyloxyethoxycarbonyloxy)-benzolsulfonat (DECOBS) oder Decanoyloxybenzoat (DOBA), Carbonsäureanhydride, insbesondere Phthalsäureanhydrid, acylierte mehrwertige Alkohole, insbesondere Triacetin, Ethylenglykoldiacetat, 2,5-Diacetoxy-2,5-dihydrofuran und Enolester sowie acetyliertes Sorbitol und Mannitol bzw. deren beschriebene Mischungen (SORMAN), acylierte Zuckerderivate, insbesondere Pentaacetylglukose (PAG), Pentaacetylfruktose, Tetraacetylxylose und Octaacetyllactose, acetyliertes, ggf. N-alkyliertes Glucamin und Gluconolacton, N-acylierte Lactame, z.B. N-Benzoylcaprolactam, Nitrile, aus denen sich Perimidsäuren bilden, insbesondere Aminoacetonitrilderivate mit quaterniertem Stickstoffatom, und/oder sauerstoffübertragende Sulfonimine und/oder Acylhydrazone. Die hydrophil substituierten Acylacetale und die Acyllactame werden ebenfalls bevorzugt eingesetzt. Auch Kombinationen konventioneller Bleichaktivatoren können eingesetzt werden. Derartige Bleichaktivatoren können, insbesondere bei Anwesenheit obengenannter Wasserstoffperoxid-liefernder Bleichmittel, im üblichen Mengenbereich, vorzugsweise in Mengen von 0,5 bis 10 Gew.-%, insbesondere 1 bis 8 Gew.-%, bezogen auf das Gesamtgewicht des Mittels, enthalten sein, fehlen bei Einsatz von Percarbonsäure als alleinigem Bleichmittel jedoch vorzugsweise ganz.

Zusätzlich zu den konventionellen Bleichaktivatoren oder an deren Stelle können in festen Mitteln auch Sulfonimine und/oder bleichverstärkende Übergangsmetallsalze bzw. Übergangsmetallkomplexe als sogenannte Bleichkatalysatoren enthalten sein.

In bevorzugten Ausführungsformen sind erfindungsgemäße Mittel im Wesentlichen frei von Bleichaktivatoren und/oder frei von Bleichkatalysatoren. "Im Wesentlichen frei" bedeutet in diesem Zusammenhang, dass die entsprechende Mittel oder Zusammensetzungen, bezogen auf das Gesamtgewicht des Mittels, weniger als 2 Gew.-%, vorzugsweise weniger als 1 Gew.-%, weiter bevorzugt weniger als 0,5 Gew.-% und besonders bevorzugt weniger als 0,1 Gew.-%, Bleichaktivatoren und/oder Bleichkatalysatoren enthalten. In besonders bevorzugten Ausführungsformen sind diese Mittel/Zusammensetzungen frei von Bleichaktivatoren und/oder Bleichkatalysatoren.

In bevorzugten Ausführungsformen sind erfindungsgemäße Mittel im Wesentlichen frei von Bleiche, d.h. im Wesentlichen frei von jeglichen bleichenden Substanzen, insbesondere frei von den zuvor stehend beschriebenen Persauerstoffverbindungen, Bleichaktivatoren und Bleichkatalysatoren. "Im Wesentlichen frei" bedeutet in diesem Zusammenhang, dass die entsprechende Mittel oder Zusammensetzungen, bezogen auf das Gesamtgewicht des Mittels, weniger als 2 Gew.-%, vorzugsweise weniger als 1 Gew.-%, weiter bevorzugt weniger als 0,5 Gew.-% und besonders bevorzugt weniger als 0,1 Gew.-%, Bleiche enthalten, d.h. jeglichen bleichenden Substanzen, insbesondere den zuvor stehend beschriebenen Persauerstoffverbindungen, Bleichaktivatoren und Bleichkatalysatoren. In besonders bevorzugten Ausführungsformen sind diese Mittel/Zusammensetzungen frei von Bleiche, d.h. frei von jeglichen bleichenden Substanzen, insbesondere frei von den zuvor stehend beschriebenen Persauerstoffverbindungen, Bleichaktivatoren und Bleichkatalysatoren.

Geeignete Vergrauungsinhibitoren bzw. soil-release-Wirkstoffe (soil release polymer) sind Celluloseether, wie Carboxymethylcellulose, Methylcellulose, Hydroxyalkylcellulosen und Cellulosemischether, wie Methylhydroxyethylcellulose, Methylhydroxypropylcellulose und Methylcarboxymethylcellulose. Vorzugsweise werden Natriumcarboxymethylcellulose, Hydroxypropylmethylcellulose und deren Gemische und ggf. deren Gemische mit Methylcellulose eingesetzt. Zu den üblicherweise eingesetzten Soil-release-Wirkstoffen gehören Copolyester, die Dicarbonsäureeinheiten, Alkylenglykoleinheiten und Polyalkylenglykoleinheiten enthalten. Der Anteil an Vergrauungsinhibitoren und/oder soil-release-Wirkstoffen in erfindungsgemäßen Mitteln liegt im Allgemeinen nicht über 2 Gew.-% und beträgt vorzugsweise 0,5 bis 1,5 Gew.-%, besonders bevorzugt 0,5 bis 2 Gew.-%, bezogen auf das Gesamtgewicht des Mittels.

Als optische Aufheller für insbesondere Textilien aus Cellulosefasern (z.B. Baumwolle) können z.B. Derivate der Diaminostilbendisulfonsäure bzw. deren Alkalimetallsalze enthalten sein. Geeignet sind z.B. Salze der 4,4'-Bis(2-anilino-4-morpholino-1,3,5-triazin-6-yl-amino)-stilben-2,2'-disulfonsäure oder gleichartig aufgebaute Verbindungen, die anstelle der Morpholinogruppe eine Diethanolaminogruppe, eine Methylaminogruppe oder eine 2-Methoxyethylaminogruppe tragen. Weiterhin können Aufheller vom Typ des substituierten 4,4'-Distyryl-diphenyl anwesend sein, z.B. 4,4'-Bis-(4-chlor-3-sulfostyryl)-diphenyl. Auch Gemische von Aufhellern können verwendet werden. Für Polyamidfasern eignen sich besonders gut Aufheller vom Typ der 1 ,3-Diaryl-2-pyrazoline, z.B. 1-(p-Sulfoamoylphenyl)-3-(p-chlorphenyl)-2-pyrazolin sowie gleichartig aufgebaute Verbindungen. Der Gehalt des Mittels an optischen Aufhellern bzw. Aufhellergemischen liegt im Allgemeinen nicht über 1 Gew.-%, vorzugsweise 0,05 bis 0,5 Gew.-%, bezogen auf das Gesamtgewicht des Mittels. In einer bevorzugten Ausführungsform der Erfindung ist das Mittel frei von derartigen Wirkstoffen.

Zu den in den erfindungsgemäßen Mitteln einsetzbaren üblichen Schaumregulatoren gehören z.B. Polysiloxan-Kieselsäure-Gemische, wobei die darin enthaltene feinteilige Kieselsäure vorzugsweise silaniert oder anderweitig hydrophobiert ist. Die Polysiloxane können sowohl aus linearen Verbindungen wie auch aus vernetzten Polysiloxan-Harzen sowie aus deren Gemischen bestehen. Weitere Entschäumer sind Paraffinkohlenwasserstoffe, insbesondere Mikroparaffine und Paraffinwachse, deren Schmelzpunkt oberhalb 40°C liegt, gesättigte Fettsäuren bzw. Seifen mit insbesondere 20 bis 22 C-Atomen, z.B. Natriumbehenat, und Alkalisalze von Phosphorsäuremono- und/oder -dialkylestern, in denen die Alkylketten jeweils 12 bis 22 C-Atome aufweisen. Unter diesen wird bevorzugt Natriummonoalkylphosphat und/oder-dialkylphosphat mit C₁₆₋₁₈-Alkylgruppen eingesetzt. Der Anteil der Schaumregulatoren kann vorzugsweise 0,2 bis 2 Gew.-%, besonders bevorzugt nicht mehr als 1 Gew.-%, bezogen auf das Gesamtgewicht des Mittels, betragen.

Zur Einstellung des gewünschten pH-Werts können erfindungsgemäße Mittel system- und umweltverträgliche Säuren, insbesondere Citronensäure, Essigsäure, Weinsäure, Äpfelsäure, Milchsäure, Glykolsäure, Bernsteinsäure, Glutarsäure und/oder Adipinsäure, aber auch Mineralsäuren, insbesondere Schwefelsäure oder Alkalihydrogensulfate, oder Basen, insbesondere Ammonium- oder Alkalihydroxide, vorzugsweise Natriumhydroxid, enthalten. Derartige pH-Regulatoren sind in den erfindungsgemäßen Mitteln vorzugsweise nicht über 10 Gew.-%, insbesondere von 0,5 bis 6 Gew.-%, besonders bevorzugt von 0,3 bis 2 Gew.-%, bezogen auf das Gesamtgewicht des Mittels, enthalten.

Als einen weiteren Bestandteil können erfindungsgemäße Mittel ein organisches Lösungsmittel enthalten. Der Zusatz organischer Lösungsmittel wirkt sich vorteilhaft auf die Enzymstabilität und die Reinigungsleistung dieser Mittel aus. Bevorzugte organische Lösungsmittel stammen aus der Gruppe ein- oder mehrwertigen Alkohole, Alkanolamine oder Glykolether. Vorzugsweise werden die Lösungsmittel ausgewählt aus Ethanol, n- oder i-Propanol, Butanol, Glykol, Propandiol, Butandiol, Glycerin, Diglykol, Propyldiglykol, Butyldiglykol, Hexylenglycol, Ethylenglykolmethylether, Ethylenglykolethylether, Ethylenglykolpropylether, Etheylenglykolmono-n-butylether, Diethylenglykolmethylether, Di-ethylenglykolethylether, Propylenglykolmethylether, Propylenglykolethylether, Propylenglykolpropylether, Dipropylenglykolmethylether, Dipropylenglykolethylether, Methoxytriglykol, Ethoxytriglykol, Butoxytriglykol, 1-Butoxyethoxy-2-propanol, 3-Methyl-3-methoxybutanol, Propylenglykol-t-butylether sowie Mischungen dieser Lösungsmittel. Der Gewichtsanteil dieser organischen Lösungsmittel am Gesamtgewicht erfindungsgemäßer Mittel beträgt vorzugsweise 0,1 bis 10 Gew.-%, bevorzugt 0,2 bis 8,0 Gew.-% und weiter bevorzugt 0,5 bis 5,0 Gew.-%. Ein besonders bevorzugtes und in Bezug auf die Stabilisierung der Mittel besonders wirksames organisches Lösungsmittel ist das Glycerin sowie das 1,2-Propylenglykol. Flüssige Mittel umfassen vorzugsweise mindestens ein Polyol, vorzugsweise aus der Gruppe Glycerin und 1 ,2-Propylenglycol, bezogen auf das Gesamtgewicht des Mittels, vorzugsweise zu 0,1 bis 10 Gew.-%, bevorzugt 0,2 bis 8,0 Gew.-% und weiter bevorzugt 0,5 bis 5,0 Gew.-%. Weitere bevorzugte organische Lösungsmittel sind die organischen Amine und Alkanolamine. Erfindungsgemäße Mittel enthalten diese Amine vorzugsweise in Mengen von 0,1 bis 10 Gew.-%, bevorzugt von 0,2 bis 8,0 Gew.-% und weiter bevorzugt von 0,5 bis 5,0 Gew.-%, bezogen auf das Gesamtgewicht des Mittels. Ein besonders bevorzugtes Alkanolamin ist das Ethanolamin.

Erfindungsgemäße Mittel können hydrolytische Enzyme oder andere Enzyme in einer für die Wirksamkeit des Mittels zweckmäßigen Konzentration enthalten. Eine weitere Ausführungsform der Erfindung stellen somit Mittel dar, die ferner eines oder mehrere Enzyme umfassen. Als Enzyme bevorzugt einsetzbar sind alle Enzyme, die in dem erfindungsgemäßen Mittel eine katalytische Aktivität entfalten können, insbesondere ausgewählt aus Proteasen, Lipasen, Amylasen, Cellulasen, Hemicellulasen, Mannanasen, Tannasen, Xylanasen, Xanthanasen, Xyloglucanasen, β-Glucosidasen, Pektinasen, Carrageenasen, Perhydrolasen, Oxidasen, Oxidoreduktasen, sowie deren Gemische. Enzyme sind in dem Mittel vorteilhafterweise jeweils in einer Menge von 1 × 10⁻⁸ bis 5 Gew.-%, bezogen auf aktives Protein und Gesamtgewicht des Mittels, enthalten. Zunehmend bevorzugt ist jedes Enzym in einer Menge von 1 × 10⁻⁷ bis 3 Gew.-%, von 0,00001 bis 1 Gew.-%, von 0,00005 bis 0,5 Gew.-%, von 0,0001 bis 0,1 Gew.-% und besonders bevorzugt von 0,0001 bis 0,05 Gew.-% in erfindungsgemäßen Mitteln enthalten, bezogen auf aktives Protein und Gesamtgewicht des Mittels. Besonders bevorzugt zeigen Enzyme synergistische Reinigungsleistungen gegenüber bestimmten Anschmutzungen oder Flecken, d.h. die in der Mittelzusammensetzung enthaltenen Enzyme unterstützen sich in ihrer Reinigungsleistung gegenseitig.

In bevorzugten Ausführungsformen enthält ein erfindungsgemäßes Mittel mindestens ein Enzym und zunehmend bevorzugt mindestens zwei, drei, vier oder fünf Enzyme, die vorzugsweise aus der aus Amylasen, Proteasen, Lipasen, Cellulasen, Mannanasen und Mischungen davon ausgewählt sind, insgesamt in einer Menge von 0,01 bis 10 Gew.-%, bevorzugt von 0,1 bis 8 Gew.-%, besonders bevorzugt von 0,2 bis 6 Gew.-%, bezogen auf aktives Protein und das Gesamtgewicht des Mittels.

Beispiele für Proteasen sind die Subtilisine BPN' aus *Bacillus amyloliquefaciens* und Carlsberg aus *Bacillus licheniformis,* die Protease PB92, die Subtilisine 147 und 309, die Protease aus *Bacillus lentus,* Subtilisin DY und die den Subtilasen, nicht mehr jedoch den Subtilisinen im engeren Sinne zuzuordnenden Enzyme Thermitase, Proteinase K und die Proteasen TW3 und TW7. Subtilisin Carlsberg ist in weiterentwickelter Form unter dem Handelsnamen Alcalase^{®} von dem Unternehmen Novozymes erhältlich. Die Subtilisine 147 und 309 werden unter den Handelsnamen Esperase^{®} bzw. Savinase^{®} von dem Unternehmen Novozymes vertrieben. Von der Protease aus *Bacillus lentus* DSM 5483 leiten sich Protease-Varianten ab, beschrieben in z.B. WO 95/23221, WO 92/21760, WO 2013/060621 und EP 3660151. Weitere brauchbare Proteasen sind z.B. die unter den Handelsnamen Durazym^{®}, Relase^{®}, Everlase^{®}, Nafizym^{®}, Natalase^{®}, Kannase^{®}, Progress Uno 101L^{®} und Ovozyme^{®} von dem Unternehmen Novozymes, die unter den Handelsnamen, Purafect^{®}, Purafect^{®} OxP, Purafect^{®} Prime, Excellase^{®}, Properase^{®}, Preferenz P100^{®} und Preferenz P300^{®} von dem Unternehmen Danisco/DuPont, das unter dem Handelsnamen Lavergy pro 104 LS^{®} von dem Unternehmen BASF, das unter dem Handelsnamen Protosol^{®} von dem Unternehmen Advanced Biochemicals Ltd., das unter dem Handelsnamen Wuxi^{®} von dem Unternehmen Wuxi Snyder Bioproducts Ltd., die unter den Handelsnamen Proleather^{®} und Protease P^{®} von dem Unternehmen Amano Pharmaceuticals Ltd., und das unter der Bezeichnung Proteinase K-16 von dem Unternehmen Kao Corp. erhältlichen Enzyme. Besonders bevorzugt eingesetzt werden auch die Proteasen aus *Bacillus gibsonii* und *Bacillus pumilus,* die offenbart sind in WO 2008/086916, WO 2007/131656, WO 2017/215925, WO 2021/175696 und WO 2021/175697.

Beispiele für Amylasen sind die α-Amylasen aus *Bacillus licheniformis, Bacillus amyloliquefaciens* oder *Bacillus stearothermophilus* sowie insbesondere auch deren für den Einsatz in Wasch- und Reinigungsmitteln verbesserte Weiterentwicklungen. Das Enzym aus *Bacillus licheniformis* ist von dem Unternehmen Novozymes unter dem Namen Termamyl^{®} und von dem Unternehmen Danisco/DuPont unter dem Namen Purastar^{®} ST erhältlich. Weiterentwicklungsprodukte dieser α-Amylase sind unter den Handelsnamen Duramyl^{®} und Termamyl^{®} ultra (beide von Novozymes), Purastar^{®} OxAm (Danisco/DuPont) und Keistase^{®} (Daiwa Seiko Inc.) erhältlich. Die α-Amylase von *Bacillus amyloliquefaciens* wird von dem Unternehmen Novozymes unter dem Namen BAN^{®} vertrieben, und abgeleitete Varianten von der α-Amylase aus *Bacillus stearothermophilus* unter den Namen BSG^{®} und Novamyl^{®}, ebenfalls von dem Unternehmen Novozymes. Des Weiteren sind für diesen Zweck die α-Amylase aus *Bacillus sp.* A 7-7 (DSM 12368) und die Cyclodextrin-Glucanotransferase (CGTase) aus *Bacillus agaradherens* (DSM 9948) hervorzuheben. Ebenso sind Fusionsprodukte aller genannten Moleküle einsetzbar. Darüber hinaus sind die unter den Handelsnamen Fungamyl^{®} von dem Unternehmen Novozymes erhältlichen Weiterentwicklungen der α-Amylase aus *Aspergillus niger* und *A*. *oryzae* geeignet. Weitere vorteilhaft einsetzbare Handelsprodukte sind z.B. die Amylase-LT^{®} und Stainzyme^{®} oder Stainzyme^{®} ultra bzw. Stainzyme^{®} plus sowie Amplity^{™} 12L oder Amplify Prime^{™} 100L oder Amplify Prime^{™} 120L, letztere ebenfalls von dem Unternehmen Novozymes, sowie die PREFERENZ S^{®} Serie von dem Unternehmen Danisco/DuPont, umfassend z.B. PREFERENZ S100^{®}, PREFERENZ S1000^{®} oder PREFERENZ S210^{®}. Auch durch Punktmutationen erhältliche Varianten dieser Enzyme können eingesetzt werden.

Geeignete Cellulasen umfassen solche bakterieller oder pilzlicher Herkunft. Chemisch modifizierte oder proteintechnisch veränderte Mutanten sind eingeschlossen. Geeignete Cellulasen sind Cellulasen aus den Gattungen *Bacillus, Pseudomonas, Humicola, Fusarium, Thielavia, Acremonium,* z. B. die Pilzcellulasen aus *Humicola insolens, Myceliophthora thermophila* und *Fusarium oxysporum,* die in US 4435307, US 5648263, US 5691178, US 5776757 und WO 89/09259 offenbart sind. Besonders geeignete Cellulasen sind die alkalischen oder neutralen Cellulasen mit farbpflegenden Eigenschaften. Beispiele für solche Cellulasen sind Cellulasen, die in EP 0495257, EP 0531372, WO 96/11262, WO 96/29397, WO 98/08940 beschrieben sind. Andere Beispiele sind Cellulase-Varianten, wie sie in WO 94/07998, EP 0531315, EP 3212777, EP 3502243, EP 3653705, EP 3653706, US 5457046, US 5686593, US 5763254, WO 95/24471, WO 98/12307 und WO 99/01544 und WO 2019/122520 beschrieben sind. Beispiele für Cellulasen mit Endo-1,4-Glucanase-Aktivität (EC 3.2.1.4) sind in WO 2002/099091 beschrieben, z.B. solche mit einer Sequenz von mindestens 97% Identität zu der Aminosäuresequenz der Positionen 1 bis 773 von SEQ ID NO:2 der WO 2002/099091. Ein weiteres Beispiel kann eine GH44-Xyloglucanase umfassen, z.B. ein Xyloglucanase-Enzym mit einer Sequenz von mindestens 60% Identität zu den Positionen 40 bis 559 der SEQ ID NO:2 der WO 2001/062903. Andere Beispiele für Cellulasen umfassen die in WO 96/29397 beschriebenen GH45-Cellulasen. Zu den kommerziell verfügbaren Cellulasen gehören Celluzyme^{™}, Carezyme^{™}, Carezyme Premium^{™}, Celluclean^{™} (z.B. Celluclean^{™} 5000L ans Cellulclean^{™} 4000T), Celluclean Classic^{™}, Cellusoft^{™}, Endolase^{®}, Renozyme^{®} und Whitezyme^{™} (Novozymes A/S), Clazinase^{™} und Puradax HA^{™} (Genencor International Inc.), KAC-500(B)^{™} (Kao Corporation), Revitalenz^{™} 1000, Revitalenz^{™} 2000 und Revitalenz^{™} 3000 (DuPont), sowie Ecostone^{®} und Biotouch^{®} (AB Enzymes).

Geeignete Lipasen sind z.B. aus *Thermomyces,* z.B. aus *T. lanuginosus* (früher *Humicola lanuginosa*), wie in EP 0258068 und EP 0305216 beschrieben, Lipase aus Stämmen von *Pseudomonas* (einige davon jetzt umbenannt in *Burkholderia*), z.B. *P. alcaligenes* oder *P. pseudoalcaligenes, P. cepacia, P. sp.* Stamm SD705, *P*. *wisconsinensis, Streptomyces-Lipasen* vom GDSL-Typ, Lipase aus *Thermobifida fusca,* Lipase aus *Geobacillus stearothermophilus,* Lipase aus *Bacillus subtilis* und Lipase aus *Streptomyces griseus* und *S. pristinaespiralis.* Zu bevorzugten Lipasen gehören z.B. die ursprünglich aus *Humicola lanuginosa* (*Thermomyces lanuginosus*) erhältlichen bzw. daraus weiterentwickelten Lipasen, insbesondere solche mit einem oder mehreren der folgenden Aminosäureaustausche ausgehend von der genannten Lipase in den Positionen D96L, T213R und/oder N233R, besonders bevorzugt T213R und N233R. Zu den bevorzugten kommerziellen Lipaseprodukten gehören Lipolase^{™}, Lipex^{™}, Lipolex^{™} und Lipoclean^{™} (Novozymes A/S), Lumafast (Genencor/DuPont) und Lipomax (Gist-Brocades).

Geeignete Mannanasen sind z.B. die *Bacillus subtilis* Endo-β-Mannanase, *Bacillus sp.* I633 Endo-β-Mannanase, *Bacillus sp.* AAI12 Endo-β-Mannanase, *Bacillus sp.* AA349 Endo-β-Mannanase, *Bacillus agaradhaerens* NCIMB 40482 Endo-β-Mannanase, *Bacillus halodurans* Endo-β-Mannanase, *Bacillus clausii* Endo-β-Mannanase, *Bacillus licheniformis* Endo-β-Mannanase, *Humicola insolens* Endo-β-Mannanase und *Caldocellulosiruptorsp.* Endo-β-Mannanase (z.B. US 6060299, WO 99/64573, US 6566114 und WO 99/64619).

Für Wasch- und Reinigungsmittel geeignete Pektatlyasen sind z.B. in WO 2003/095638 oder WO 2015/121133 beschrieben. Beispiele für geeignete pektinolytische Enzyme sind zudem die unter den Handelsbezeichnungen Gamanase^{®}, Pektinex AR^{®}, X-Pect^{®} oder Pectaway^{®} von dem Unternehmen Novozymes, unter den Handelsbezeichnungen Rohapect UF^{®}, Rohapect TPL^{®}, Rohapect PTE100^{®}, Rohapect MPE^{®}, Rohapect MA plus HC, Rohapect DA12L^{®}, Rohapect 10L^{®}, Rohapect B1L^{®} von dem Unternehmen AB Enzymes und unter der Handelsbezeichnung Pyrolase^{®} von dem Unternehmen Diversa Corp. erhältlichen Enzyme und Enzym-Zubereitungen.

Bei den hierin beschrieben Peptiden, Proteinen und Enzymen handelt es sich vorzugsweise um reife (mature) Peptide, Proteine oder Enzyme, d.h. um das katalytisch aktive Molekül ohne Signal- und/oder Propeptid(e). Soweit nicht anders angegeben beziehen sich auch die angegebenen Sequenzen auf jeweils reife (prozessierte) Peptide, Proteine oder Enzyme.

In verschiedenen Ausführungsformen der Erfindung ist das jeweilige Peptid, Protein oder Enzym ein frei vorliegendes Peptid, Protein oder Enzym. Dies bedeutet, dass das Peptid, Protein oder Enzym mit allen Komponenten des Mittels direkt agieren kann und, falls es sich bei dem Mittel um ein Flüssigmittel handelt, dass das Peptid, Protein oder Enzym direkt mit dem Lösungsmittel des Mittels (z.B. Wasser) in Kontakt steht. In anderen Ausführungsformen kann ein Mittel Peptide, Proteine oder Enzyme enthalten, die einen Interaktionskomplex mit anderen Molekülen bilden oder die eine "Umhüllung" enthalten. Hierbei kann ein einzelnes oder mehrere Peptid-, Protein- oder Enzymmolekül(e) durch eine sie umgebende Struktur von den anderen Bestandteilen des Mittels getrennt sein. Eine solche trennende Struktur kann entstehen durch, ist allerdings nicht beschränkt auf, Vesikel, wie etwa eine Micelle oder ein Liposom. Die umgebende Struktur kann aber auch ein Viruspartikel, eine bakterielle Zelle oder eine eukaryotische Zelle sein. In verschiedenen Ausführungsformen kann ein Mittel Zellen von *Bacillus pumilus* oder *Bacillus subtilus,* die Peptide, Proteine oder Enzyme exprimieren, oder Zellkulturüberstände solcher Zellen enthalten.

Im Zusammenhang mit der vorliegenden Erfindung bedeutet das Merkmal, dass ein Peptid, Protein oder Enzym die angegebene(n) Substitution(en) (oder Deletion oder Insertion) aufweist, dass sie an der jeweiligen Position eine (der angegebenen) Substitution(en) (oder Deletion oder Insertion) enthält, d.h. zumindest die angegebenen Positionen nicht anderweitig mutiert oder, z.B. durch Fragmentierung des Peptids, Proteins oder Enzyms, deletiert sind. In verschiedenen Ausführungsformen weisen die hierin beschriebenen Peptide, Proteine und/oder Enzyme mit Ausnahme der explizit erwähnten Substitutionen die Sequenz der jeweiligen Referenzsequenz auf, d.h. sind abgesehen von den substituierten Positionen 100% identisch zu der jeweiligen Referenzsequenz.

Die Bestimmung der Identität von Nukleinsäure- oder Aminosäuresequenzen erfolgt durch einen Sequenzvergleich. Dieser Sequenzvergleich basiert auf dem im Stand der Technik etablierten und üblicherweise genutzten BLAST-Algorithmus (Altschul et al., Basic local alignment search tool, J. Mol. Biol., 1990, 215, 403-410, und Altschul et al., Gapped BLAST and PSI-BLAST: a new generation of protein database search programs, Nucleic Acids Res., 1997, 25, 3389-3402) und geschieht prinzipiell dadurch, dass ähnliche Abfolgen von Nukleotiden oder Aminosäuren in den Nukleinsäure- oder Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus. Sequenzvergleiche (Alignments), insbesondere multiple Sequenzvergleiche, werden mit Computerprogrammen erstellt. Häufig genutzt werden z.B. die Clustal-Serie (Chenna et al., Multiple sequence alignment with the Clustal series of programs, Nucleic Acid Res., 2003, 31, 3497-3500), T-Coffee (Notredame et al., T-Coffee: A novel method for multiple sequence alignments, J. Mol. Biol., 2000, 302, 205-217) oder Programme, die auf diesen Programmen bzw. Algorithmen basieren. Ferner möglich sind Sequenzvergleiche (Alignments) mit dem Computer-Programm Vector NTI^{®} Suite 10.3 (Invitrogen Corporation, 1600 Faraday Avenue, Carlsbad, Kalifornien, USA) mit den vorgegebenen Standardparametern, dessen AlignX-Modul für die Sequenzvergleiche auf ClustalW basiert, oder Clone Manager 10 (Verwendung der Scoring Matrix BLOSUM 62 für Sequenz-Alignment auf Aminosäureebene). Soweit nicht anders angegeben, wird die hierin angegebene Sequenzidentität mit dem BLAST-Algorithmus bestimmt.

Solch ein Vergleich erlaubt auch eine Aussage über die Ähnlichkeit der verglichenen Sequenzen zueinander. Sie wird üblicherweise in Prozent Identität, das heißt dem Anteil der identischen Nukleotide oder Aminosäurereste an denselben oder in einem Alignment einander entsprechenden Positionen angegeben. Der weiter gefasste Begriff der Homologie bezieht bei Aminosäuresequenzen konservierte Aminosäure-Austausche in die Betrachtung mit ein, also Aminosäuren mit ähnlicher chemischer Aktivität, da diese innerhalb des Peptids, Proteins oder Enzyms meist ähnliche chemische Aktivitäten ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe oder identische Bereiche von verschiedenen Nukleinsäure- oder Aminosäuresequenzen sind daher durch Übereinstimmungen in den Sequenzen definiert. Solche Bereiche weisen oftmals identische Funktionen auf. Sie können klein sein und nur wenige Nukleotide oder Aminosäuren umfassen. Oftmals üben solche kleinen Bereiche für die Gesamtaktivität des Peptids, Proteins oder Enzyms essenzielle Funktionen aus. Es kann daher sinnvoll sein, Sequenzübereinstimmungen nur auf einzelne, ggf. kleine Bereiche zu beziehen. Soweit nicht anders angegeben beziehen sich Identitäts- oder Homologieangaben in der vorliegenden Anmeldung aber auf die Gesamtlänge der jeweils angegebenen Nukleinsäure- oder Aminosäuresäuresequenz.

Im Zusammenhang mit der vorliegenden Erfindung bedeutet die Angabe, dass eine Aminosäureposition einer numerisch bezeichneten Position in SEQ ID NO:1 entspricht daher, dass die entsprechende Position der numerisch bezeichneten Position in SEQ ID NO:1 in einem wie oben definierten Alignment zugeordnet ist.

Für die Beschreibung von Substitutionen, die genau eine Aminosäureposition betreffen (Aminosäureaustausche), wird hierin folgende Konvention angewendet: zunächst wird die natürlicherweise vorhandene Aminosäure in Form des international gebräuchlichen Einbuchstaben-Codes bezeichnet, dann folgt die zugehörige Sequenzposition und schließlich die eingefügte Aminosäure. Mehrere oder alternative Austausche innerhalb derselben Polypeptidkette werden durch Schrägstriche voneinander getrennt. "130D/V" bedeutet somit, dass die Position 130 zu D oder V mutiert ist. Bei Insertionen sind nach der Sequenzposition zusätzliche Aminosäuren benannt. Bei Deletionen ist die fehlende Aminosäure durch ein Symbol, z.B. einen Stern oder einen Strich, ersetzt oder vor der entsprechenden Position ein Δ angegeben. Beispielsweise beschreibt P9T die Substitution von Prolin an Position 9 durch Threonin, P9TH die Insertion von Histidin nach der Aminosäure Threonin an Position 9 und P9* oder ΔP9 die Deletion von Prolin an Position 9. Diese Nomenklatur ist dem Fachmann auf dem Gebiet der Enzymtechnologie bekannt.

Einem Fachmann ist es über heutzutage allgemein bekannte Methoden, wie z.B. die chemische Synthese oder die Polymerase-Kettenreaktion (PCR) in Verbindung mit molekularbiologischen und/oder proteinchemischen Standardmethoden möglich, anhand bekannter DNA- und/oder Aminosäuresequenzen die entsprechenden Nukleinsäuren bis hin zu vollständigen Genen herzustellen. Derartige Methoden sind z.B. aus Sambrook, J., Fritsch, E.F. and Maniatis, T. 2001. Molecular cloning: a laboratory manual, 3. Edition Cold Spring Laboratory Press. bekannt.

Ferner können die in dem Mittel enthaltenen Peptide, Proteine, Enzyme und/oder weitere Inhaltsstoffe des Mittels mit einer bei Raumtemperatur oder bei Abwesenheit von Wasser für das Peptid, Protein oder Enzym undurchlässigen Substanz umhüllt sein, welche unter Anwendungsbedingungen des Mittels durchlässig für das Peptid, Protein oder Enzym wird. Eine solche Ausführungsform der Erfindung ist somit dadurch gekennzeichnet, dass das Peptid, Protein oder Enzym mit einer bei Raumtemperatur oder bei Abwesenheit von Wasser für das Peptid, Protein oder Enzym undurchlässigen Substanz umhüllt ist. Weiterhin kann auch das Wasch- oder Reinigungsmittel selbst in einem Behältnis, vorzugsweise einem luftdurchlässigen Behältnis, verpackt sein, aus dem es kurz vor Gebrauch oder während des Waschvorgangs freigesetzt wird.

In den hierin beschriebenen Mitteln können die einzusetzenden Peptide, Proteine oder Enzyme ferner zusammen mit Begleitstoffen, etwa aus der Fermentation, konfektioniert sein. In flüssigen Formulierungen werden die Peptide, Protein oder Enzyme bevorzugt als Peptid-, Protein oder Enzymflüssigformulierung(en) eingesetzt.

Die Peptide, Protein oder Enzyme werden in der Regel nicht in Form des reinen Proteins, sondern vielmehr in Form stabilisierter, lager- und transportfähiger Zubereitungen bereitgestellt. Zu diesen vorkonfektionierten Zubereitungen zählen z.B. die durch Granulation, Extrusion oder Lyophilisierung erhaltenen festen Präparationen oder, insbesondere bei flüssigen oder gelförmigen Mitteln, Lösungen der Peptide, Proteine oder Enzyme, vorteilhafterweise möglichst konzentriert, wasserarm und/oder mit Stabilisatoren oder weiteren Hilfsmitteln versetzt.

Alternativ können die Peptide, Proteine oder Enzyme sowohl für die feste als auch für die flüssige Darreichungsform verkapselt werden, z.B. durch Sprühtrocknung oder Extrusion der Peptid-, Protein- oder Enzymlösung zusammen mit einem vorzugsweise natürlichen Polymer oder in Form von Kapseln, z.B. solchen, bei denen die Peptide, Proteine oder Enzyme wie in einem erstarrten Gel eingeschlossen sind oder in solchen vom Kern-Schale-Typ, bei dem ein enzymhaltiger Kern mit einer Wasser-, Luft- und/oder Chemikalien-undurchlässigen Schutzschicht überzogen ist. In aufgelagerten Schichten können zusätzlich weitere Wirkstoffe, z.B. Stabilisatoren, Emulgatoren, Pigmente, Bleich- oder Farbstoffe aufgebracht werden. Derartige Kapseln werden nach an sich bekannten Methoden, z.B. durch Schüttel- oder Rollgranulation oder in Fluid-bed-Prozessen aufgebracht. Vorteilhafterweise sind derartige Granulate, z.B. durch Aufbringen polymerer Filmbildner, staubarm und aufgrund der Beschichtung lagerstabil.

Weiterhin ist es möglich, zwei oder mehrere Peptide, Proteine oder Enzyme zusammen zu konfektionieren, so dass ein einzelnes Granulat mehrere Enzymaktivitäten aufweist.

Die Peptide, Proteine oder Enzyme können auch in wasserlösliche Filme, wie sie z.B. bei der Konfektionierung von Wasch- und Reinigungsmitteln in Einheitsdosisform verwendet werden, eingebracht werden. Ein derartiger Film ermöglicht die Freisetzung der Peptide, Proteine oder Enzyme nach Kontakt mit Wasser. Wie hierin verwendet, bezieht sich "wasserlöslich" auf eine Filmstruktur, die vorzugsweise vollständig wasserlöslich ist. Bevorzugt besteht ein solcher Film aus (vollständig oder teilweise hydrolysiertem) Polyvinylalkohol (PVA).

Erfindungsgemäße Mittel können einen oder mehrere reversible Enyzminhibitor(en)/- stabilisator(en) umfassen. Erfindungsgemäße Mittel können den/die reversiblen Enyzminhibitor(en)/- stabilisator(en) in einer Konzentration von 0,1 bis 2 Gew.-%, vorzugsweise 0,3 bis 1,5 Gew.-%, enthalten, bezogen auf das Gesamtgewicht des Mittels. Falls mehrere Inhibitoren/Stabilisatoren enthalten sind, beziehen sich diese Angaben auf die Gesamtkonzentration. Diese können insbesondere ausgewählt sein aus der aus Polyolen, wie Glycerin oder 1,2-Ethylenglycol, Benzamidinhydrochlorid, Borax, Borsäuren, Boronsäuren oder deren Salze oder Ester oder Derivate, insbesondere Phenylboronsäurederivate oder 4-Formylphenylboronsäure (4-FPBA), Antioxidantien, speziellen Peptidverbindungen und Kombinationen davon bestehenden Gruppe.

Im Rahmen der vorliegenden Erfindung wird unter "Phenylboronsäurederivat" eine Verbindung mit der folgenden Formel verstanden: 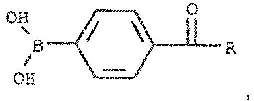 wobei R Wasserstoff, eine Hydroxyl-, eine C₁₋₆ Alkyl-, eine substituierte C₁₋₆ Alkyl-, eine C₁₋₆ Alkenyl oder eine substituierte C₁₋₆ Alkenyl-Gruppe ist. Bevorzugt ist der Rest R in dem Phenylboronsäurederivat eine C₁₋₆ Alkyl-Gruppe und hierunter weiter bevorzugt -CH₃, -CH₃CH₂ oder - CH₃CH₂CH₂ sein. Weiter bevorzugt ist der Rest R in dem Phenylboronsäurederivat Wasserstoff. Besonders bevorzugt ist das Phenylboronsäurederivat 4-Formylphenylboronsäure (4-FPBA).

Die eingesetzte Inhibitor/Stabilisatorverbindung kann Borsäure sein.

In bevorzugten Ausführungsformen ist das erfindungsgemäße Mittel im Wesentlichen frei von borhaltigen Verbindungen. "Im Wesentlichen frei von borhaltigen Verbindungen" bedeutet in diesem Zusammenhang, dass die erfindungsgemäßen Mittel weniger als 2 Gew.-%, vorzugsweise weniger als 1 Gew.-%, weiter bevorzugt weniger als 0,5 Gew.-% und besonders bevorzugt weniger als 0,1 Gew.-%, borhaltige Verbindungen, bezogen auf das Gesamtgewicht des Mittels, enthalten. In besonders bevorzugten Ausführungsformen sind die erfindungsgemäßen Mittel frei von borhaltigen Verbindungen, d.h. sie enthalten insbesondere keine Borsäure und/oder Phenylboronsäurederivate.

In verschiedenen Ausführungsformen können das Enzym und die Inhibitor/Stabilisatorverbindung in einer Enzymzusammensetzung vorformuliert vorliegen. Wie aus den vorherigen Ausführungen ersichtlich, bildet das Enzym-Protein nur einen Bruchteil des Gesamtgewichts üblicher Enzymzubereitungen. Bevorzugt eingesetzte Enzymzubereitungen enthalten zwischen 0,1 und 40 Gew.-%, bevorzugt zwischen 0,2 und 30 Gew.-%, besonders bevorzugt zwischen 0,4 und 20 Gew.-% und insbesondere zwischen 0,8 und 10 Gew.-% des Enzymproteins. In solchen Zusammensetzungen kann die Inhibitor/Stabilisatorverbindung in einer Menge von 0,05 bis 35 Gew.-%, vorzugsweise von 0,05 bis 10 Gew.-%, bezogen auf das Gesamtgewicht in der Enzymzusammensetzung, enthalten sein. Diese Enzymzusammensetzung kann in erfindungsgemäßen Mitteln eingesetzt werden, und zwar in Mengen, die zu den oben angegeben Endkonzentrationen im Mittel führen.

In bevorzugten Ausführungsformen betrifft die Erfindung ein Mittel zur Reinigung von Textilien und/oder harten Oberflächen, insbesondere Geschirr, dadurch gekennzeichnet, dass das Mittel ein Wasch- und Reinigungsmittel, vorzugsweise ein flüssiges Wasch- und Reinigungsmittel, bevorzugt ein flüssiges Textilwaschmittel ist, und umfasst
(A) mindestens ein Peptid, wobei das Peptid
   eine Länge von 10 bis 50, vorzugsweise 10 bis 30, bevorzugt 12 bis 25, Aminosäuren aufweist, die aus der aus Alanin, Arginin, Asparagin, Asparaginsäure, Cystein, Glutamin, Glutaminsäure, Glycin, Histidin, Isoleucin, Leucin, Lysin, Methionin, Phenylalanin, Prolin, Serin, Threonin, Tryptophan, Tyrosin und Valin bestehenden Gruppe ausgewählt sind, vorzugsweise ausgewählt aus Alanin, Arginin, Glutaminsäure, Glycin, Isoleucin, Leucin, Lysin, Phenylalanin, Prolin, Serin, Threonin, Tryptophan, Tyrosin und Valin,
   eine Nettoladung von +2 bis +8, vorzugsweise +3 bis +7, bevorzugt +4 bis +6, aufweist,
   antimikrobiell wirksam ist, wobei die antimikrobielle Wirksamkeit wie in Beispiel 1 beschrieben bestimmt wird,
   vorzugsweise in einer Menge von aktivem Peptid von 1 × 10⁻⁸ bis 5 Gew.-%, vorzugsweise 0,0001 bis 1 Gew.-%, bevorzugt 0,0005 bis 0,5 Gew.-%, besonders bevorzugt 0,001 bis 0,1 Gew.-%, bezogen auf das Gesamtgewicht des Mittels;
(B) mindestens ein Tensid, vorzugsweise in einer Menge von 3 bis 35 Gew.-%, bevorzugt 5 bis 30 Gew.-%, bezogen auf das Gesamtgewicht des Mittels, wobei das Tensid aus der aus anionischen Tensiden, nicht-ionischen Tensiden, kationischen Tensiden, zwitterionischen Tensiden, amphoteren Tensiden und Mischungen davon bestehenden Gruppe ausgewählt ist;
(C) ein Buildersystem, umfassend mindestens einen Builder, vorzugsweise in einer Menge von 0,5 bis 50 Gew.-%, bevorzugt 0,5 bis 20 Gew.-%, besonders bevorzugt 0,5 bis 10 Gew. %, bezogen auf das Gesamtgewicht des Mittels, wobei der Builder aus der aus Polycarbonsäuren wie Zitronensäure, Adipinsäure, Bernsteinsäure, Glutarsäure, Äpfelsäure, Weinsäure, Maleinsäure Fumarsäure, Zuckersäuren und Carboxymethylinuline oder deren Salzen, monomeren und polymeren Aminopolycarbonsäuren wie Glycindiessigsäure, Methylglycindiessigsäure (MGDA), Glutaminsäurediessigsäure (GLDA), Nitriltriessigsäure, Iminodisuccinat wie Ethylendiamin-N,N'-Disuccinsäure und Hydroxyiminodisuccinate, Ethylendiamintetraessigsäure und Polyasparaginsäure oder deren Salze, Polyphosphonsäuren wie Aminotris(methylenphosphonsäure), Ethylendiamintetrakis(methylenphosphonsäure), Lysintetra(methylenphosphonsäure), Diethylentriaminpenta(methylenphosphonsäure) (DTPMP) und 1-Hydroxyethan-1,1-diphosphonsäure (HEDP) oder deren Salze, polymeren Hydroxyverbindungen wie Dextrin, und Mischungen davon bestehenden Gruppe ausgewählt ist; und
(D) optional mindestens ein Enzym, vorzugsweise in einer Menge von 1 × 10⁻⁸ bis 5 Gew.-%, vorzugsweise 0,0001 bis 1 Gew.-%, bevorzugt 0,0005 bis 0,5 Gew.-%, besonders bevorzugt 0,001 bis 0,1 Gew.-%, bezogen auf aktives Protein und Gesamtgewicht des Mittels, wobei das Enzym aus der aus Proteasen, Amylasen, Lipasen, Mannanasen, Cellulasen oder Mischungen davon bestehenden Gruppe ausgewählt ist.

In einer bevorzugten Ausführungsform betrifft die Erfindung ein Mittel zur Reinigung von Oberflächen, insbesondere Haushaltsoberflächen, bevorzugt in Wasch- oder Geschirrspülmaschinen, Pumpensümpfen, Vorratsbehältern, Wasserreservoir, Waschtrommel, Spülkammern, Wasch- oder Spülbecken, Toilettenbecken, Spülkasten, WC-Rand, Siphons, Wasserrohren und/oder -leitungen, Wasserzu- und/oder -abläufen, und/oder Textilien, insbesondere zur Verhinderung und/oder Reduzierung von Schlechtgeruch und/oder zur Verbesserung der Hygiene, dadurch gekennzeichnet dass das Mittel ein Wasch- und Reinigungsmittel, vorzugsweise ein flüssiges Wasch- und Reinigungsmittel, bevorzugt flüssiges Textilwaschmittel ist, und umfasst
(A) mindestens ein Peptid, wobei das Peptid
   eine Länge von 10 bis 50, vorzugsweise 10 bis 30, bevorzugt 12 bis 25, Aminosäuren aufweist, die aus der aus Alanin, Arginin, Asparagin, Asparaginsäure, Cystein, Glutamin, Glutaminsäure, Glycin, Histidin, Isoleucin, Leucin, Lysin, Methionin, Phenylalanin, Prolin, Serin, Threonin, Tryptophan, Tyrosin und Valin bestehenden Gruppe ausgewählt sind, vorzugsweise ausgewählt aus Alanin, Arginin, Glutaminsäure, Glycin, Isoleucin, Leucin, Lysin, Phenylalanin, Prolin, Serin, Threonin, Tryptophan, Tyrosin und Valin,
   eine Nettoladung von +2 bis +8, vorzugsweise +3 bis +7, bevorzugt +4 bis +6, aufweist,
   antimikrobiell wirksam ist, wobei die antimikrobielle Wirksamkeit wie in Beispiel 1 beschrieben bestimmt wird,
   vorzugsweise in einer Menge von aktivem Peptid von 1 × 10⁻⁸ bis 5 Gew.-%, vorzugsweise 0,0001 bis 1 Gew.-%, bevorzugt 0,0005 bis 0,5 Gew.-%, besonders bevorzugt 0,001 bis 0,1 Gew.-%, besonders bevorzugt 0,001 bis 0,25 Gew.-%, bezogen auf das Gesamtgewicht des Mittels;
(B) mindestens ein Tensid, vorzugsweise in einer Menge von 3 bis 35 Gew.-%, bevorzugt 5 bis 30 Gew.-%, bezogen auf das Gesamtgewicht des Mittels, wobei das Tensid aus der aus anionischen Tensiden, nicht-ionischen Tensiden, kationischen Tensiden, zwitterionischen Tensiden, amphoteren Tensiden und Mischungen davon bestehenden Gruppe ausgewählt ist;
(C) ein Buildersystem, umfassend mindestens einen Builder, vorzugsweise in einer Menge von 0,5 bis 50 Gew.-%, bevorzugt 0,5 bis 20 Gew.-%, besonders bevorzugt 0,5 bis 10 Gew. %, bezogen auf das Gesamtgewicht des Mittels, wobei der Builder aus der aus Polycarbonsäuren wie Zitronensäure, Adipinsäure, Bernsteinsäure, Glutarsäure, Äpfelsäure, Weinsäure, Maleinsäure Fumarsäure, Zuckersäuren und Carboxymethylinuline oder deren Salzen, monomeren und polymeren Aminopolycarbonsäuren wie Glycindiessigsäure, Methylglycindiessigsäure (MGDA), Glutaminsäurediessigsäure (GLDA), Nitriltriessigsäure, Iminodisuccinat wie Ethylendiamin-N,N'-Disuccinsäure und Hydroxyiminodisuccinate, Ethylendiamintetraessigsäure und Polyasparaginsäure oder deren Salze, Polyphosphonsäuren wie Aminotris(methylenphosphonsäure), Ethylendiamintetrakis(methylenphosphonsäure), Lysintetra(methylenphosphonsäure), Diethylentriaminpenta(methylenphosphonsäure) (DTPMP) und 1-Hydroxyethan-1,1-diphosphonsäure (HEDP) oder deren Salze, polymeren Hydroxyverbindungen wie Dextrin, und Mischungen davon bestehenden Gruppe ausgewählt ist; und
(D) optional mindestens ein Enzym, vorzugsweise in einer Menge von 1 × 10⁻⁸ bis 5 Gew.-%, vorzugsweise 0,0001 bis 1 Gew.-%, bevorzugt 0,0005 bis 0,5 Gew.-%, besonders bevorzugt 0,001 bis 0,1 Gew.-%, bezogen auf aktives Protein und Gesamtgewicht des Mittels, wobei das Enzym aus der aus Proteasen, Amylasen, Lipasen, Mannanasen, Cellulasen oder Mischungen davon bestehenden Gruppe ausgewählt ist.

Mit umfasst sind dabei auch jeweils Mittel für (halb-)automatisierte Wasch- oder Reinigungssysteme wie z.B. Wischrobotor oder Nassstaubsauger.

Alle Sachverhalte, Gegenstände und Ausführungsformen, die für erfindungsgemäße Peptide und erfindungsgemäße Mittel beschrieben sind, sind auch auf diese Erfindungsgegenstände anwendbar. Daher wird an dieser Stelle ausdrücklich auf die Offenbarung an entsprechender Stelle verwiesen mit dem Hinweis, dass diese Offenbarung auch für die vorstehende erfindungsgemäße Verwendung gilt.

Ein weiterer Gegenstand der Erfindung betrifft auch kosmetische Mittel, wie z.B. Wasch- und Pflegeprodukten zur Haarpflege sowie industrielle und institutionelle Reiniger, wie z.B. Desinfektionsprodukte, insbesondere Desinfektionsmittel, wobei solche Mittel ein hierin beschriebenes antimikrobielles Peptid enthalten.

In weiteren bevorzugten Ausführungsformen betrifft die Erfindung
- Verfahren zur Reinigung von Textilien und/oder harten Oberflächen, insbesondere Geschirr, dadurch gekennzeichnet, dass in mindestens einem Verfahrensschritt ein hierin beschriebenes Wasch- und Reinigungsmittel angewendet wird, wobei das Verfahren vorzugsweise in einem Temperaturbereich von etwa 20°C bis etwa 60°C, bevorzugt etwa 40°C, besonders bevorzugt etwa 30°C, durchgeführt wird; und
- Verfahren zur Reinigung von Oberflächen, insbesondere Haushaltsoberflächen, bevorzugt in Wasch- oder Geschirrspülmaschinen, Pumpensümpfen, Vorratsbehältern, Wasserreservoir, Waschtrommel, Spülkammern, Wasch- oder Spülbecken, Toilettenbecken, Spülkasten, WC-Rand, Siphons, Wasserrohren und/oder -leitungen, Wasserzu- und/oder -abläufen, und/oder Textilien, insbesondere zur Verhinderung und/oder Reduzierung von Schlechtgeruch und/oder zur Verbesserung der Hygiene, dadurch gekennzeichnet, dass in mindestens einem Verfahrensschritt ein hierin beschriebenes Wasch- und Reinigungsmittel angewendet wird, wobei das Verfahren vorzugsweise in einem Temperaturbereich von etwa 20°C bis etwa 60°C, bevorzugt etwa 40°C, besonders bevorzugt etwa 30°C, durchgeführt wird.

Hierunter fallen sowohl manuelle als auch maschinelle Verfahren, wobei maschinelle Verfahren aufgrund ihrer präziseren Steuerbarkeit, was z.B. die eingesetzten Mengen und Einwirkzeiten angeht, bevorzugt sind. Verfahren zur Reinigung von Textilien zeichnen sich im Allgemeinen dadurch aus, dass in mehreren Verfahrensschritten verschiedene reinigungsaktive Substanzen auf das Reinigungsgut aufgebracht und nach der Einwirkzeit abgewaschen werden, oder dass das Reinigungsgut in sonstiger Weise mit einem Mittel oder einer Lösung oder Verdünnung dieses Mittels behandelt wird. Mit umfasst sind dabei auch Verfahren unter Verwendung (halb-)automatisierter Wasch- oder Reinigungssysteme wie z.B. Wischrobotor oder Nassstaubsauger.

Dieser Erfindungsgegenstand umfasst auch ein maschinelles Geschirrspülverfahren. Die Dosierung des erfindungsgemäßen Mittels in die Reinigungsflotte kann in einem solchen Verfahren z.B. mittels der Dosierkammer in der Tür oder mittels eines zusätzlichen Dosierbehälters im Innenraum der Geschirrspülmaschine erfolgen. Alternativ kann das Mittel auch direkt auf das verschmutzte Geschirr oder auf eine der Innenwände der Geschirrspülmaschine, z.B. die Innenseite der Tür aufgebracht werden. Die Durchführung des erfindungsgemäßen Verfahrens erfolgt im Innenraum einer handelsüblichen Geschirrspülmaschine. Das Reinigungsprogramm kann bei einer Geschirrspülmaschine in der Regel vor Durchführung des Geschirrspülverfahrens durch den Verbraucher gewählt und festgelegt werden. Das in dem erfindungsgemäßen Verfahren eingesetzte Reinigungsprogramm der Geschirrspülmaschine umfasst dabei mindestens einen Vorspülgang und einen Reinigungsgang. Erfindungsgemäß bevorzugt werden Reinigungsprogramme, die weitere Reinigungs- oder Spülgänge, z.B. einen Klarspülgang umfassen. Das erfindungsgemäße Verfahren ist mit besonderem Vorzug Bestandteil eines Reinigungsprogramms, umfassend einen Vorspülgang, einen Reinigungsgang sowie einen Klarspülgang. Das erfindungsgemäße Verfahren wird bevorzugt in Verbindung mit solchen Reinigungsprogrammen eingesetzt, bei denen die Waschflotte im Verlauf des Reinigungsgangs erwärmt wird. In einer bevorzugten Ausführungsform des erfindungsgemäßen Verfahrens ist der Reinigungsgang, in dessen Verlauf das erfindungsgemäße Mittel in den Innenraum der Geschirrspülmaschine eindosiert wird dadurch gekennzeichnet, dass in seinem Verlauf die Temperatur der Reinigungsflotte auf Werte oberhalb 30°C, vorzugsweise oberhalb 40°C und insbesondere oberhalb 50°C ansteigt.

Alternative Ausführungsformen dieses Erfindungsgegenstandes stellen auch Verfahren zur Behandlung von Textilrohstoffen oder zur Textilpflege dar, bei denen in wenigstens einem Verfahrensschritt ein erfindungsgemäßes Mittel aktiv wird. Hierunter sind Verfahren für Textilrohstoffe, Fasern oder Textilien mit natürlichen Bestandteilen bevorzugt, und ganz besonders für solche mit Wolle oder Seide.

Alle Sachverhalte, Gegenstände und Ausführungsformen, die für erfindungsgemäße Peptide und erfindungsgemäße Mittel beschrieben sind, sind auch auf diese Erfindungsgegenstände anwendbar. Daher wird an dieser Stelle ausdrücklich auf die Offenbarung an entsprechender Stelle verwiesen mit dem Hinweis, dass diese Offenbarung auch für die vorstehende erfindungsgemäße Verwendung gilt.

In weiteren bevorzugten Ausführungsformen betrifft die Erfindung
- Verwendung eines Peptids in einem Wasch- und Reinigungsmittels zur Reinigung von Textilien und/oder harten Oberflächen, insbesondere Geschirr, und/oder Haushaltsoberflächen, bevorzugt in Wasch- oder Geschirrspülmaschinen, Pumpensümpfen, Vorratsbehältern, Wasserreservoir, Waschtrommel, Spülkammern, Wasch- oder Spülbecken, Toilettenbecken, Spülkasten, WC-Rand, Siphons, Wasserrohren und/oder -leitungen, Wasserzu- und/oder -abläufen, vorzugsweise in einem Temperaturbereich von etwa 20°C bis etwa 60°C, bevorzugt etwa 40°C, besonders bevorzugt etwa 30°C, wobei das Peptid wie hierin definiert ist;
- Verwendung eines Peptids in einem Wasch- und Reinigungsmittels zur Reduzierung von Schlechtgeruch von Textilien und/oder harten Oberflächen, insbesondere Geschirr, und/oder Haushaltsoberflächen, bevorzugt in Wasch- oder Geschirrspülmaschinen, Pumpensümpfen, Vorratsbehältern, Wasserreservoir, Waschtrommel, Spülkammern, Wasch- oder Spülbecken, Toilettenbecken, Spülkasten, WC-Rand, Siphons, Wasserrohren und/oder -leitungen, Wasserzu- und/oder -abläufen, die mit dem Mittel behandelt wurden, vorzugsweise in einem Temperaturbereich von etwa 20°C bis etwa 60°C, bevorzugt etwa 40°C, besonders bevorzugt etwa 30°C, wobei das Peptid wie hierin definiert ist;
- Verwendung eines Peptids in einem Wasch- und Reinigungsmittels zur Desinfektion von Textilien und/oder harten Oberflächen, insbesondere Geschirr, und/oder Haushaltsoberflächen, bevorzugt in Wasch- oder Geschirrspülmaschinen, Pumpensümpfen, Vorratsbehältern, Wasserreservoir, Waschtrommel, Spülkammern, Wasch- oder Spülbecken, Toilettenbecken, Spülkasten, WC-Rand, Siphons, Wasserrohren und/oder -leitungen, Wasserzu- und/oder -abläufen, die mit dem Mittel behandelt wurden, vorzugsweise in einem Temperaturbereich von etwa 20°C bis etwa 60°C, bevorzugt etwa 40°C, besonders bevorzugt etwa 30°C, wobei das Peptid wie hierin definiert ist; und
- Verwendung eines Peptids in einem Wasch- und Reinigungsmittels zur Verhinderung und/oder Reduzierung mikrobiellen Wachstums auf Textilien und/oder harten Oberflächen, insbesondere Geschirr, und/oder Haushaltsoberflächen, bevorzugt in Wasch- oder Geschirrspülmaschinen, Pumpensümpfen, Vorratsbehältern, Wasserreservoir, Waschtrommel, Spülkammern, Wasch- oder Spülbecken, Toilettenbecken, Spülkasten, WC-Rand, Siphons, Wasserrohren und/oder -leitungen, Wasserzu- und/oder -abläufen, die mit dem Mittel behandelt wurden, vorzugsweise in einem Temperaturbereich von etwa 20°C bis etwa 60°C, bevorzugt etwa 40°C, besonders bevorzugt etwa 30°C, wobei das Peptid wie hierin definiert ist.

Alle Sachverhalte, Gegenstände und Ausführungsformen, die für erfindungsgemäße Peptide und erfindungsgemäße Mittel beschrieben sind, sind auch auf diese Erfindungsgegenstände anwendbar. Daher wird an dieser Stelle ausdrücklich auf die Offenbarung an entsprechender Stelle verwiesen mit dem Hinweis, dass diese Offenbarung auch für die vorstehende erfindungsgemäße Verwendung gilt.

### BEISPIELE

### Beispiel 1: Bestimmung der antimikrobiellen Wirksamkeit

**Tabelle 1: Verwendete Waschmittelmatrix**

| **Chemischer Name** | **Gew.-% Aktivsubstanz im Rohmaterial** | **Gew.-% Aktivsubstanz in der Formulierung** | |
|---|---|---|---|
| | | **A** | **B** |
| Wasser demin. | 100 | Rest | Rest |
| 1,2-Propandiol | 100 | 5,0 | 5,0 |
| Glycerin | 99,5 | 9,4 | 9,4 |
| Monoethanolamin | 100 | 6,0 | 6,0 |
| Alkylbenzolsulfonsäure | 96 | 21,7 | 21,7 |
| Palmkernöl | 100 | 7,0 | 7,0 |
| C₁₂₋₁₈ Fettalkoholethoxylat, 7EO | 100 | 22,4 | 22,4 |
| Ethoxyliertes Polyethylenimin | 80 | 4,5 | 4,5 |
| DTPMP-Na7 | 40 | 0,5 | 0,5 |
| Enzyme | t.q. | 0 | Minors |
| Misc. (SRP, DTI, Parfüm, opt. Aufheller, Bitterstoffe, Antifoam) | t.q. | minors | minors |
| Dosierung 3,17 g/L; pH 8,2-8,4 | | | |

**Tabelle 2: Verwendete Peptide**

| **Peptidnummer** | **Aminosäuresequenz** | **SEQ ID NO:** | **Quelle** |
|---|---|---|---|
| AMP 1 | ILRWPWWPWRRK | 1 | Sader et al.¹ |
| AMP 2 | FSTKTRNWFSEHFKKVKEKLKDTFA | 2 | Barksdale et al.² |
| AMP 3 | KTRNWFSEHFKKVKEKLKDTFA | 3 | Barksdale et al.² |
| AMP 4 | WRWAKWGLKLLKYKKIY | 4 | Fleeman et al.³ |
| AMP 5 | IWWAKWGLKLLRYRRWY | 5 | Fleeman et al.³ |

| | | | |
|---|---|---|---|
| ¹ Sader et al. (2004), Omiganan Pentahydrochloride (MBI 226), a Topical 12-Amino-Acid Cationic Peptide: Spectrum of Antimicrobial Activity and Measurements of BactericidalActivity, Antimicrobial Agents and Chemotherapy, 48 (8): 3112-3118; ² Barksdale et al. (2016) Peptides from American alligator plasma are antimicrobial against multi-drug resistant bacterial pathogens including Acinetobacter baumannii, BMC Microbiology, 16: 189-202; ³ Fleeman et al. (2020) Defining principles that influence antimicrobial peptide activity against capsulated Klebsiella pneumoniae, PNAS, 117 (44): 27620-27626 | | | |

Die antibakterielle Wirksamkeit der in Tabelle 2 gezeigten Peptide wurde in einer Konzentration von 5.000 mg/L in einem MPN-Suspensionstest (MPN = Most Probable Number) an verschiedenen Mikroorganismen (*Staphylococcus aureus* ATCC 6538/K3212, Ausgangskeimzahl 3,6E+0,6; *Klebsiella pneumoniae* ATCC 4352/K2510, Ausgangskeimzahl 6,1E+06) getestet. Die jeweiligen Peptidlösungen wurden in Wasser (Kontrolle), Waschmittel ohne Enzym (A) und Waschmittel mit Enzym (B) in einer Konzentration von 5.000 mg/L gelöst.

Der MPN-Suspensionstest erfolgte in Deep-Well-Mikrotiterplatten (1.000 µl Volumen pro Well), wobei jedes Well 800 µl Anwendungskonzentration (AWK, angesetzt in dest. Wasser) mit 1,25-fach konzentrierter Prüfsubstanz enthielt (Endkonzentration: 5.000 mg/L). Es wurden jeweils 100 µl der Keimsuspension hinzugegeben (Endkonzentration: 1,0E+08 KBE/ml). Zur Homogenisierung der Testansätze wurden die Mikrotiterplatten während des Tests auf einem Titramax platziert. Parallel zu den Testansätzen wurde eine Wasserkontrolle mitgeführt. Hierbei wurde anstelle der Prüfsubstanz dest. Wasser verwendet.

Nach einer Inkubation für 60 min bei 30°C erfolgte die Überimpfung eines Aliquots der Testansätze und der Wasserkontrolle in eine Inaktivierungslösung. Für den Inaktivierungsschritt (1:10 Verdünnung) wurden 100 µl Testgemisch mit 900 µl Nährbouillon (enthaltend Inaktivierungsmittel) vermischt (Endkonzentration: 1,0E+06 KBE /ml im Inaktivierungsansatz).

Nach mindestens 5 min und höchstens 30 min Inaktivierung erfolgte die Bestimmung der Anzahl lebender Testkeimzellen. In 96-Well-Mikrotiterplatten (200 µl Volumen pro Well) wurden dekadische Verdünnungsreihen angesetzt, in dem pro Well 180 µl Nährbouillon (enthaltend Inaktivierungsmittel) vorgelegt wurden. Die Durchführung der Verdünnungsreihen erfolgt in jeweils drei Parallelen, d.h. aus den Inaktivierungsansätzen (= Prüfgemisch plus Inaktivierungsmittel in Nährbouillon) wurden jeweils drei Wells beimpft und dekadisch verdünnt. Pro Well wurden 20 µl inaktivierter Ansatz + 180 µl Nährbouillon (enthaltend Inaktivierungsmitteln) in jeweils 5 dekadischen Verdünnungsschritten gemischt. Anschließend wurden die abgedeckten Mikrotiterplatten bei 37°C für 48 h inkubiert.

Die Ergebniskalkulation bzgl. der Abtötungsraten der eingesetzten Testkeime erfolgte in Abhängigkeit zur Wasserkontrolle. Zur Auswertung wurde das Wachstum in den Wells, angezeigt durch Trübung der Nährbouillon, visuell oder mit dem Tecan-Reader bestimmt.

Die mikrobizide Wirkung wurde mit folgender Formel berechnet:

RF = K / D oder log10 K - Iog10 D,

wobei RF = Reduktionsfaktor, K = Anzahl der KBE/ml ohne Einwirkung des Produktes (Wasserkontrolle) und D = Anzahl der KBE/ml nach Einwirkung des Produktes.

Die Ergebnisse sind in den Tabelle 3 dargestellt.

**Tabelle 3: Antimikrobielle Wirksamkeit gegen Staphylococcus aureus und Klebsiella pneumoniae**

| *S*. *aureus* (ATCC 6538/K3212) | | Log-Reduktion | | |
|---|---|---|---|---|
| Wirkstoff | Konzentration | Wasser | Waschmittel A | Waschmittel B |
| AMP 1 | 5000 mg/L | >4,65 | 2,40 | 1,48 |
| AMP 2 | 5000 mg/L | >4,65 | 1,48 | 2,40 |
| AMP 4 | 5000 mg/L | >4,65 | 2,40 | 1,48 |
| Benzalkoniumchlorid (Positivkontrolle) | 100 mg/L | >4,65 | keine Reduktion | keine Reduktion |
| | | | | |

| *K. pneumoniae* (ATCC 4352/K2510) | | Log-Reduktion | | |
|---|---|---|---|---|
| Wirkstoff | Konzentration | Wasser | Waschmittel A | Waschmittel B |
| AMP 1 | 5000 mg/L | >4,40 | >4,65 | >4,40 |
| AMP 2 | 5000 mg/L | >4,40 | >4,65 | >4,40 |
| AMP 4 | 5000 mg/L | >4,40 | >4,04 | >4,65 |
| Benzalkoniumchlorid (Positivkontrolle) | 100 mg/L | >4,40 | keine Reduktion | keine Reduktion |

Die Ergebnisse zeigen, dass die getesteten Peptide sowohl gegen *Staphylococcus aureus* (Gram-positives Bakterium) als auch *Klebsiella pneumoniae* (Gram-negatives Bakterium) eine antimikrobielle Wirkung aufweist und die Anzahl lebender Zellen signifikant reduziert ist, zum Teil sogar um bis zu 4 Log-Stufen.

### Beispiel 2: Beispielformulierungen

Die erfindungsgemäßen Peptide können in verschiedenen Wasch- und Reinigungsmitteln eingesetzt werden und einen antimikrobiellen Effekt verwirklichen.

**Tabelle 4: Flüssigwaschmittel**

| **Chemischer Name** | **Gew.-% Aktivsubstanz in der Formulierung** | | |
|---|---|---|---|
| | **A** | **B** | **C** |
| Borsäure | 0,6 | 0,6 | 0,6 |
| Zitronensäure | 0,3 | 0,3 | 0,3 |
| FAEOS | 3,9 | 3,9 | 3,9 |
| FAEO | 4,3 | 4,3 | 4,3 |
| LAS | 3,5 | 3,5 | 3,5 |
| Seife (Palmkernöl) | 0,6 | 0,6 | 0,6 |
| NaOH | 0,8 | 0,8 | 0,8 |
| 1,2-Propandiol | 0 | 0 | 0 |
| Glycerin | 0,6 | 0,6 | 0,6 |
| DTPMP | 0,3 | 0 | 0,13 |
| GLDA | 0 | 0,3 | 0,13 |
| Erfindungsgemäßes Peptid | 5000 mg/L | 5000 mg/L | 5000 mg/L |
| Wasser, demin. | ad 100 | ad 100 | ad 100 |
| DTI, SRP, weitere Enzyme, Entschäumer, u.a. | minors | minors | minors |
| Viskosität [mPas] = 250-550; Dichte [g/cm³] = 1,03-1,04; pH = 8,2-8,6 | | | |

**Tabelle 5: Flüssigwaschmittel**

| **Chemischer Name** | **Gew.-% Aktivsubstanz in der Formulierung** | | | | | |
|---|---|---|---|---|---|---|
| | **A** | **B** | **C** | **D** | **E** | **F** |
| Wasser demin. | Rest | Rest | Rest | Rest | Rest | Rest |
| LAS | 5,5 | 20 | 15,0 | 5,5 | 21,7 | 23,5 |
| FAEOS | 7,0 | | 5,0 | | | |
| Palmkernölsäure | 3,0 | 8,0 | | | 7,0 | 7,4 |
| FAEO | 5,5 | | 8,0 | | | |
| C_{13/15} Oxoalkohol, 8EO | | 25 | | | | |
| C₁₂₋₁₈ Fettalkoholethoxylat, 7EO | | | | | 22,4 | 23,4 |
| Alkylpolyglykosid | | | 4,0 | | | |
| Nichtionische Tenside | | | | 3,1 | | |
| Seife | | | 1,0 | 0,5 | | |
| HEDP | 0,5 | | | | | |
| DTPMPA 7Na | | 1,0 | 1,0 | 0,2 | 0,5 | 1,7 |
| Zitronensäure | 2,5 | | 3,0 | 0,23 | | |
| NaOH | 3,0 | | | 0,7 | | |
| Glycerin | 3,0 | 5,0 | | 0,5 | 9,4 | 10,2 |
| Ethanol | 1,5 | 3,0 | | | | 3,2 |
| 1,2-Propandiol | | 10,0 | 12,0 | | 5,0 | 5,6 |
| Monoethanolamin | | 6,0 | 7,0 | | 6,0 | 6,1 |
| Borsäure | 1,0 | | 1,0 | 0,5 | | |
| Polyalkoxyliertes Alkanolamin | | | | | 4,5 | |
| Ethoxyliertes Polyethylenimin | | | | | 4,5 | 3,0 |
| Erfindungsgemäße Peptide | 5000 mg/L | 5000 mg/L | 5000 mg/L | 5000 mg/L | 5000 mg/L | 5000 mg/L |
| DTI, SRP, weitere Enzyme, Entschäumer, u.a. | minors | minors | minors | minors | minors | minors |

**Tabelle 6: Feste Waschmittel**

| **Chemischer Name** | **Gew.-% Aktivsubstanz in der Formulierung** | | |
|---|---|---|---|
| | **A** | **B** | **C** |
| LAS | 12,2 | 12,0 | 10,1 |
| Na-Fettalkoholsulfat, C₁₂₋₁₈ | 4,2 | | |
| Fettalkohol, C₁₂₋₁₈, 7 EO | 4,1 | 2,3 | 1,5 |
| Seifen | 0,4 | | |
| Citrat | 2,0 | | |
| Natriumcarbonat | 2,4 | 17,9 | 25,1 |
| Builder | 23,0 | 7,0 | 7,6 |
| Phosphonat | 1,2 | 1,1 | 1,2 |
| Polyacrylat | 0,12 | 2,8 | 3,0 |
| Carboxymethylcellulose | 2,3 | 2,0 | 1,1 |
| 2Na2 Carbonat 3 H2O2 | 18,5 | 15,8 | |
| TAED | 10,9 | 3,5 | |
| Erfindungsgemäße Peptide | 5000 mg/L | 5000 mg/L | 5000 mg/L |
| Natriumsulfat, Schauminhibitor, optischer Aufheller, Duftstoffe, weitere Enzyme | Rest | Rest | Rest |

**Tabelle 7: Handgeschirrspülmittel**

| **Chemischer Name** | **Gew.-% Aktivsubstanz in der Formulierung** |
|---|---|
| Wasser demin. | Rest |
| FAEOS | 8,8 |
| Cocoamidopropylbetain | 1,2 |
| Calciumlactat | 1,0 |
| Amylase (Stainzyme 12L) | 0,8 |
| Parfüm | 0,2 |
| Farbstoff | 0,01 |
| Salze | 2,0 |
| Erfindungsgemäße Peptide | 5000 mg/L |
| pH | 8,0 |

**Tabelle 8: Zweiphasiges Geschirrspülmittel**

| **Pulverphase (Phase A)** | **A1** | **A2** |
|---|---|---|
| Aktivstoffgehalt in Gew.-% (soweit nichts anderes angegeben), bezogen auf das Gesamtgewicht der Pulverphase | | |
| Natriumpercarbonat | 13,0 | 15,0 |
| Nichtionisches Tenside | 4,0 | 4,0 |
| Sulfonsäuregruppenhaltiges Polymer | 4,0 | 4,0 |
| HEDP (Natriumsalz) | 6,0 | 6,0 |
| Natriumcarbonat (inkl. Natriumhydrogencarbonat) | 24,0 | 28,0 |
| MGDA (Trinatriumsalz) | 0 | 0 |
| Schichtsilikat (SKS 6 Pulver) | 4,0 | 4,0 |
| Natriumcitrat (als wasserfreies Natriumcitrat berechnet) | 21,0 | 21,0 |
| TAED | 2,1 | 2,1 |
| Zinkacetat | 0,15 | 0,15 |
| Amylase (Stainzyme^{®} Plus 24 Evity T; Angabe Gew.-% bezogen auf die Menge der eingesetzten Zubereitung, t.q.) | 1,5 | 1,5 |
| Protease (Unity 1000; Gesamtaktivprotein) | 40 mg/Job | 40 mg/Job |
| Erfindungsgemäße Peptide | 5000 mg/L | 5000 mg/L |
| Silberschutz (Cystein) | 0,15 | 0,15 |
| Misc (Parfüm, Farbstoffe, Konservierungsmittel, Füllstoffe z.B. Natriumsulfat, Bleichkatalysator (MnTACN)) | Add 100 | Add 100 |
| | | |

| **Gelphase (Phase B)** | **B1** | **B2** |
|---|---|---|
| Aktivstoffgehalt in Gew.-% (soweit nichts anderes angegeben), bezogen auf das Gesamtgewicht der Gelphase | | |
| Polymer umfassend Acrylsäure- und Amidopropylsulfonsäurehaltige Monomere | 10,0 | 11,0 |
| Glycerin | 27,0 | 25,0 |
| 1,3-Propandiol | 30,0 | 30,0 |
| PEG 400 | 15,0 | 17,0 |
| PVOH (POVAL 4-88) | 15,0 | 14,0 |
| Misc (u.a Prozesshilfsmittel, pH-Stellmittel, Parfüm, Farbstoff) | Add 100 | Add 100 |
| Gelierzeit / min | weniger als1 | weniger als1 |
| | | |
| Die Phasen A1 bzw. A2 und die Phasen B1 bzw. B2 können beliebig miteinander kombiniert werden. Gesamtgewicht beider Phasen in einer Einmalportion von 18,5 g. | | |

## Patentansprüche

1. Mittel, vorzugsweise Wasch- und/oder Reinigungsmittel, bevorzugt flüssiges Wasch- und/oder Reinigungsmittel, besonders bevorzugt flüssiges Textilwaschmittel, umfassend
(A) mindestens ein Peptid, wobei das Peptid
eine Länge von 10 bis 50, vorzugsweise 10 bis 30, bevorzugt 12 bis 25, Aminosäuren aufweist, die aus der aus Alanin, Arginin, Asparagin, Asparaginsäure, Cystein, Glutamin, Glutaminsäure, Glycin, Histidin, Isoleucin, Leucin, Lysin, Methionin, Phenylalanin, Prolin, Serin, Threonin, Tryptophan, Tyrosin und Valin bestehenden Gruppe ausgewählt sind, vorzugsweise ausgewählt aus Alanin, Arginin, Glutaminsäure, Glycin, Isoleucin, Leucin, Lysin, Phenylalanin, Prolin, Serin, Threonin, Tryptophan, Tyrosin und Valin,
eine Nettoladung von +2 bis +8, vorzugsweise +3 bis +7, bevorzugt +4 bis +6, aufweist,
antimikrobiell wirksam ist, wobei die antimikrobielle Wirksamkeit wie in Beispiel 1 beschrieben bestimmt wird,
(B) mindestens ein Tensid, wobei das Tensid aus der aus anionischen Tensiden, nicht-ionischen Tensiden, kationischen Tensiden, zwitterionischen Tensiden, amphoteren Tensiden und Mischungen davon bestehenden Gruppe ausgewählt ist;
(C) ein Buildersystem, umfassend mindestens einen Builder, wobei der Builder aus der aus Polycarbonsäuren wie Zitronensäure, Adipinsäure, Bernsteinsäure, Glutarsäure, Äpfelsäure, Weinsäure, Maleinsäure Fumarsäure, Zuckersäuren und Carboxymethylinuline oder deren Salzen, monomeren und polymeren Aminopolycarbonsäuren wie Glycindiessigsäure, Methylglycindiessigsäure (MGDA), Glutaminsäurediessigsäure (GLDA), Nitriltriessigsäure, Iminodisuccinat wie Ethylendiamin-N,N'-Disuccinsäure und Hydroxyiminodisuccinate, Ethylendiamintetraessigsäure und Polyasparaginsäure oder deren Salze, Polyphosphonsäuren wie Aminotris(methylenphosphonsäure), Ethylendiamintetrakis(methylenphosphonsäure), Lysintetra(methylenphosphonsäure), Diethylentriaminpenta(methylenphosphonsäure) (DTPMP) und 1-Hydroxyethan-1,1-diphosphonsäure (HEDP) oder deren Salze, polymeren Hydroxyverbindungen wie Dextrin, und Mischungen davon bestehenden Gruppe ausgewählt ist; und
(D) optional mindestens ein Enzym, wobei das Enzym aus der aus Proteasen, Amylasen, Lipasen, Mannanasen, Cellulasen oder Mischungen davon bestehenden Gruppe ausgewählt ist.

2. Mittel nach Anspruch 1, wobei das Peptid mindestens 1 bis 5 Tryptophan-Reste und/oder 1 bis 5 Arginin-Reste und/oder 1 bis 5 Lysin-Reste, vorzugsweise mindestens 1 bis 4 Tryptophan-Reste und/oder 1 bis 3 Arginin-Reste und/oder 1 bis 5 Lysin-Reste, umfasst.

3. Mittel nach Anspruch 1 oder 2, wobei das Peptid eine Aminosäuresequenz umfasst, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% oder 100% identisch ist.

4. Mittel, wobei das Mittel umfasst, jeweils bezogen auf das Gesamtgewicht des Mittels,
das mindestens eine Peptid in einer Menge von aktivem Peptid von 1 × 10⁻⁸ bis 5 Gew.-%, vorzugsweise 0,0001 bis 1 Gew.-%, bevorzugt 0,0005 bis 0,5 Gew.-%, besonders bevorzugt 0,001 bis 0,1 Gew.-%,
das mindestens eine Tensid in einer Menge von 3 bis 35 Gew.-%, bevorzugt 5 bis 30 Gew.-%, bezogen auf das Gesamtgewicht des Mittels,
den mindestens einen Builder in einer Menge von 0,5 bis 50 Gew.-%, bevorzugt 0,5 bis 20 Gew.-%, besonders bevorzugt 0,5 bis 10 Gew. %, und
das mindestens eine Enzym in einer Menge von aktivem Protein von 1 × 10⁻⁸ bis 5 Gew.-%, vorzugsweise 0,0001 bis 1 Gew.-%, bevorzugt 0,0005 bis 0,5 Gew.-%, besonders bevorzugt 0,001 bis 0,1 Gew.-%.

5. Wasch- und/oder Reinigungsmittel nach einem der vorhergehenden Ansprüche, wobei das Mittel umfasst, jeweils bezogen auf das Gesamtgewicht des Mittels,
(i) 2 bis 20 Gew.-%, vorzugsweise 3 bis 17 Gew.-% anionische Tenside,
(ii) 1 bis 10 Gew.-%, vorzugsweise 3 bis 8 Gew.-%, nichtionische Tenside,
(iii) 0 bis 1 Gew.-%, vorzugsweise 0 bis 0,5 Gew.-%, Seife, und
(iv) 0 bis 5 Gew.-%, vorzugsweise 0 bis 3 Gew.-% Fettsäuren.

6. Wasch- und/oder Reinigungsmittel nach einem der vorhergehenden Ansprüche, wobei das Buildersystem umfasst, jeweils bezogen auf das Gesamtgewicht des Mittels,
(i) 0 bis 10 Gew.-%, vorzugsweise 1 bis 4 Gew.-% Zitronensäure und/oder Citrat, vorzugsweise Alkalicitrat,
(ii) 0 bis 40 Gew.-%, vorzugsweise 0 bis 15 Gew.-%, stärker bevorzugt 1 bis 3 Gew.-%, Alkalicarbonat, vorzugsweise Natriumcarbonat,
(iii) 0 bis 20 Gew.-%, vorzugsweise 3 bis 10 Gew.-% Alkalisilikat,
(iv) 0 bis 10 Gew.-%, vorzugsweise 0,5 bis 2 Gew.-%, Phosphonsäure und/oder Alkaliphosphonat, besonders bevorzugt HEDP und/oder DTPMP, und/oder
(v) 0 bis 10 Gew.-%, vorzugsweise 0,5 bis 3 Gew.-%, Aminopolycarbonsäuren, vorzugsweise MGDA und/oder GLDA.

7. Wasch- und/oder Reinigungsmittel nach einem der vorhergehenden Ansprüche, wobei
das Mittel im Wesentlichen frei von borhaltigen Verbindungen ist, vorzugsweise frei von borhaltigen Verbindungen ist; und/oder
das Mittel im Wesentlichen frei von Phosphonaten ist, vorzugsweise frei von Phosphonaten, insbesondere frei von HEDP und/oder DTPMP; und/oder
das Mittel in 1 Gew.-%iger Lösung in entionisiertem Wasser bei 20°C einen pH-Wert in einem Bereich von etwa 6 bis etwa 11, insbesondere von etwa 6,5 bis etwa 10,5, weiter bevorzugt von etwa 7 bis etwa 10, besonders bevorzugt von etwa 8 bis etwa 9 aufweist; und/oder
das Mittel, bezogen auf das Gesamtgewicht des Mittels, weniger als 10 Gew.-% LAS, vorzugsweise weniger als 6 Gew.-%, noch bevorzugter weniger als 3 Gew.-% enthält, noch bevorzugter im Wesentlichen frei von LAS ist, besonders bevorzugt die Zusammensetzung frei von LAS ist; und/oder
das Mittel im Wesentlichen frei von Bleiche ist, vorzugsweise frei von Bleiche ist.

8. Verfahren zur Reinigung von Textilien und/oder harten Oberflächen, insbesondere Geschirr, **dadurch gekennzeichnet, dass** in mindestens einem Verfahrensschritt ein Wasch- und/oder Reinigungsmittel nach einem der Ansprüche 1 bis 7 angewendet wird, wobei das Verfahren vorzugsweise in einem Temperaturbereich von etwa 20°C bis etwa 60°C, bevorzugt etwa 40°C, besonders bevorzugt etwa 30°C, durchgeführt wird.

9. Verfahren zur Reinigung von Oberflächen, insbesondere Haushaltsoberflächen, bevorzugt in Wasch- oder Geschirrspülmaschinen, Pumpensümpfen, Vorratsbehältern, Wasserreservoir, Waschtrommel, Spülkammern, Wasch- oder Spülbecken, Toilettenbecken, Spülkasten, WC-Rand, Siphons, Wasserrohren und/oder -leitungen, Wasserzu- und/oder -abläufen, und/oder Textilien, insbesondere zur Verhinderung und/oder Reduzierung von Schlechtgeruch und/oder zur Verbesserung der Hygiene, **dadurch gekennzeichnet, dass** in mindestens einem Verfahrensschritt ein Wasch- und/oder Reinigungsmittel nach einem der Ansprüche 1 bis 7 angewendet wird, wobei das Verfahren vorzugsweise in einem Temperaturbereich von etwa 20°C bis etwa 60°C, bevorzugt etwa 40°C, besonders bevorzugt etwa 30°C, durchgeführt wird.

10. Verwendung eines Peptids in einem Wasch- und/oder Reinigungsmittels zur Reinigung von Textilien und/oder harten Oberflächen, insbesondere Geschirr, und/oder Haushaltsoberflächen, bevorzugt in Wasch- oder Geschirrspülmaschinen, Pumpensümpfen, Vorratsbehältern, Wasserreservoir, Waschtrommel, Spülkammern, Wasch- oder Spülbecken, Toilettenbecken, Spülkasten, WC-Rand, Siphons, Wasserrohren und/oder -leitungen, Wasserzu- und/oder -abläufen, vorzugsweise in einem Temperaturbereich von etwa 20°C bis etwa 60°C, bevorzugt etwa 40°C, besonders bevorzugt etwa 30°C,
wobei das Peptid
eine Länge von 10 bis 50, vorzugsweise 10 bis 30, bevorzugt 12 bis 25, Aminosäuren aufweist, die aus der aus Alanin, Arginin, Asparagin, Asparaginsäure, Cystein, Glutamin, Glutaminsäure, Glycin, Histidin, Isoleucin, Leucin, Lysin, Methionin, Phenylalanin, Prolin, Serin, Threonin, Tryptophan, Tyrosin und Valin bestehenden Gruppe ausgewählt sind, vorzugsweise ausgewählt aus Alanin, Arginin, Glutaminsäure, Glycin, Isoleucin, Leucin, Lysin, Phenylalanin, Prolin, Serin, Threonin, Tryptophan, Tyrosin und Valin,
eine Nettoladung von +2 bis +8, vorzugsweise +3 bis +7, bevorzugt +4 bis +6, aufweist, und
antimikrobiell wirksam ist, wobei die antimikrobielle Wirksamkeit wie in Beispiel 1 beschrieben bestimmt wird.

11. Verwendung eines Peptids in einem Wasch- und/oder Reinigungsmittels zur Reduzierung von Schlechtgeruch von Textilien und/oder harten Oberflächen, insbesondere Geschirr, und/oder Haushaltsoberflächen, bevorzugt in Wasch- oder Geschirrspülmaschinen, Pumpensümpfen, Vorratsbehältern, Wasserreservoir, Waschtrommel, Spülkammern, Wasch- oder Spülbecken, Toilettenbecken, Spülkasten, WC-Rand, Siphons, Wasserrohren und/oder -leitungen, Wasserzu- und/oder -abläufen, die mit dem Mittel behandelt wurden, vorzugsweise in einem Temperaturbereich von etwa 20°C bis etwa 60°C, bevorzugt etwa 40°C, besonders bevorzugt etwa 30°C,
wobei das Peptid
eine Länge von 10 bis 50, vorzugsweise 10 bis 30, bevorzugt 12 bis 25, Aminosäuren aufweist, die aus der aus Alanin, Arginin, Asparagin, Asparaginsäure, Cystein, Glutamin, Glutaminsäure, Glycin, Histidin, Isoleucin, Leucin, Lysin, Methionin, Phenylalanin, Prolin, Serin, Threonin, Tryptophan, Tyrosin und Valin bestehenden Gruppe ausgewählt sind, vorzugsweise ausgewählt aus Alanin, Arginin, Glutaminsäure, Glycin, Isoleucin, Leucin, Lysin, Phenylalanin, Prolin, Serin, Threonin, Tryptophan, Tyrosin und Valin,
eine Nettoladung von +2 bis +8, vorzugsweise +3 bis +7, bevorzugt +4 bis +6, aufweist, und antimikrobiell wirksam ist, wobei die antimikrobielle Wirksamkeit wie in Beispiel 1 beschrieben bestimmt wird.

12. Verwendung eines Peptids in einem Wasch- und/oder Reinigungsmittels zur Desinfektion von Textilien und/oder harten Oberflächen, insbesondere Geschirr, und/oder Haushaltsoberflächen, bevorzugt in Wasch- oder Geschirrspülmaschinen, Pumpensümpfen, Vorratsbehältern, Wasserreservoir, Waschtrommel, Spülkammern, Wasch- oder Spülbecken, Toilettenbecken, Spülkasten, WC-Rand, Siphons, Wasserrohren und/oder -leitungen, Wasserzu- und/oder -abläufen, die mit dem Mittel behandelt wurden, vorzugsweise in einem Temperaturbereich von etwa 20°C bis etwa 60°C, bevorzugt etwa 40°C, besonders bevorzugt etwa 30°C,
wobei das Peptid
eine Länge von 10 bis 50, vorzugsweise 10 bis 30, bevorzugt 12 bis 25, Aminosäuren aufweist, die aus der aus Alanin, Arginin, Asparagin, Asparaginsäure, Cystein, Glutamin, Glutaminsäure, Glycin, Histidin, Isoleucin, Leucin, Lysin, Methionin, Phenylalanin, Prolin, Serin, Threonin, Tryptophan, Tyrosin und Valin bestehenden Gruppe ausgewählt sind, vorzugsweise ausgewählt aus Alanin, Arginin, Glutaminsäure, Glycin, Isoleucin, Leucin, Lysin, Phenylalanin, Prolin, Serin, Threonin, Tryptophan, Tyrosin und Valin,
eine Nettoladung von +2 bis +8, vorzugsweise +3 bis +7, bevorzugt +4 bis +6, aufweist, und antimikrobiell wirksam ist, wobei die antimikrobielle Wirksamkeit wie in Beispiel 1 beschrieben bestimmt wird.

13. Verwendung eines Peptids in einem Wasch- und/oder Reinigungsmittels zur Unterdrückung und/oder Reduzierung mikrobiellen Wachstums auf Textilien und/oder harten Oberflächen, insbesondere Geschirr, und/oder Haushaltsoberflächen, bevorzugt in Wasch- oder Geschirrspülmaschinen, Pumpensümpfen, Vorratsbehältern, Wasserreservoir, Waschtrommel, Spülkammern, Wasch- oder Spülbecken, Toilettenbecken, Spülkasten, WC-Rand, Siphons, Wasserrohren und/oder -leitungen, Wasserzu- und/oder -abläufen, die mit dem Mittel behandelt wurden, vorzugsweise in einem Temperaturbereich von etwa 20°C bis etwa 60°C, bevorzugt etwa 40°C, besonders bevorzugt etwa 30°C,
wobei das Peptid
eine Länge von 10 bis 50, vorzugsweise 10 bis 30, bevorzugt 12 bis 25, Aminosäuren aufweist, die aus der aus Alanin, Arginin, Asparagin, Asparaginsäure, Cystein, Glutamin, Glutaminsäure, Glycin, Histidin, Isoleucin, Leucin, Lysin, Methionin, Phenylalanin, Prolin, Serin, Threonin, Tryptophan, Tyrosin und Valin bestehenden Gruppe ausgewählt sind, vorzugsweise ausgewählt aus Alanin, Arginin, Glutaminsäure, Glycin, Isoleucin, Leucin, Lysin, Phenylalanin, Prolin, Serin, Threonin, Tryptophan, Tyrosin und Valin,
eine Nettoladung von +2 bis +8, vorzugsweise +3 bis +7, bevorzugt +4 bis +6, aufweist, und antimikrobiell wirksam ist, wobei die antimikrobielle Wirksamkeit wie in Beispiel 1 beschrieben bestimmt wird.

14. Verwendung nach einem der Ansprüche 10 bis 13, wobei das Peptid mindestens 1 bis 5 Tryptophan-Reste und/oder 1 bis 5 Arginin-Reste und/oder 1 bis 5 Lysin-Reste, vorzugsweise mindestens 1 bis 4 Tryptophan-Reste und/oder 1 bis 3 Arginin-Reste und/oder 1 bis 5 Lysin-Reste, umfasst; und/oder das Peptid eine Aminosäuresequenz umfasst, die zu einer der in SEQ ID NO:1, SEQ ID NO:2, SEQ ID NO:3, SEQ ID NO:4 oder SEQ ID NO:5 angegebenen Aminosäuresequenzen über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% oder 100% identisch ist.

15. Verwendung eines Wasch- und/oder Reinigungsmittels nach einem der Ansprüche 1 bis 7
zur Reinigung von Textilien und/oder harten Oberflächen, insbesondere Geschirr, und/oder Haushaltsoberflächen, bevorzugt in Wasch- oder Geschirrspülmaschinen, Pumpensümpfen, Vorratsbehältern, Wasserreservoir, Waschtrommel, Spülkammern, Wasch- oder Spülbecken, Toilettenbecken, Spülkasten, WC-Rand, Siphons, Wasserrohren und/oder -leitungen, Wasserzu- und/oder-abläufen, und/oder
zur Reduzierung von Schlechtgeruch von Textilien und/oder harten Oberflächen, insbesondere Geschirr, und/oder Haushaltsoberflächen, bevorzugt in Wasch- oder Geschirrspülmaschinen, Pumpensümpfen, Vorratsbehältern, Wasserreservoir, Waschtrommel, Spülkammern, Wasch- oder Spülbecken, Toilettenbecken, Spülkasten, WC-Rand, Siphons, Wasserrohren und/oder -leitungen, Wasserzu- und/oder -abläufen, die mit dem Mittel behandelt wurden, und/oder
zur Desinfektion von Textilien und/oder harten Oberflächen, insbesondere Geschirr,
und/oder Haushaltsoberflächen, bevorzugt in Wasch- oder Geschirrspülmaschinen, Pumpensümpfen, Vorratsbehältern, Wasserreservoir, Waschtrommel, Spülkammern, Wasch- oder Spülbecken, Toilettenbecken, Spülkasten, WC-Rand, Siphons, Wasserrohren und/oder -leitungen, Wasserzu- und/oder -abläufen, die mit dem Mittel behandelt wurden, und/oder
zur Unterdrückung und/oder Reduzierung mikrobiellen Wachstums auf Textilien und/oder harten Oberflächen, insbesondere Geschirr, und/oder Haushaltsoberflächen, bevorzugt in Wasch- oder Geschirrspülmaschinen, Pumpensümpfen, Vorratsbehältern, Wasserreservoir, Waschtrommel, Spülkammern, Wasch- oder Spülbecken, Toilettenbecken, Spülkasten, WC-Rand, Siphons, Wasserrohren und/oder -leitungen, Wasserzu- und/oder -abläufen, die mit dem Mittel behandelt wurden,
vorzugsweise in einem Temperaturbereich von etwa 20°C bis etwa 60°C, bevorzugt etwa 40°C, besonders bevorzugt etwa 30°C.
